(19) Europäisches Patentamt
European Patent Office
Office européen des brevets

(11) EP 3 337 467 B1

(12) # EUROPEAN PATENT SPECIFICATION

(45) Date of publication and mention of the grant of the patent:
**09.12.2020 Bulletin 2020/50**

(21) Application number: **16757498.7**

(22) Date of filing: **19.08.2016**

(51) Int Cl.:
*A61K 31/04* (2006.01) *A61K 31/166* (2006.01)
*A61K 31/416* (2006.01) *A61K 31/4184* (2006.01)
*A61K 31/436* (2006.01) *A61K 31/4745* (2006.01)
*A61K 31/475* (2006.01) *A61K 31/502* (2006.01)
*A61K 31/55* (2006.01) *A61K 9/127* (2006.01)
*A61K 47/06* (2006.01) *A61P 35/00* (2006.01)

(86) International application number:
**PCT/US2016/047814**

(87) International publication number:
**WO 2017/031442 (23.02.2017 Gazette 2017/08)**

(54) **COMBINATION THERAPY USING LIPOSOMAL IRINOTECAN AND A PARP INHIBITOR FOR CANCER TREATMENT**

KOMBINATIONSTHERAPIE UNTER VERWENDUNG VON LIPOSOMALEM IRINOTECAN UND EINEM PARP-HEMMER ZUR BEHANDLUNG VON KREBS

POLYTHÉRAPIE UTILISANT L'IRINOTÉCAN LIPOSOMAL ET UN INHIBITEUR DE PARP POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**20.08.2015 US 201562207709 P**
**20.08.2015 US 201562207760 P**
**18.12.2015 US 201562269756 P**
**18.12.2015 US 201562269511 P**
**16.03.2016 US 201662308924 P**
**15.04.2016 US 201662323422 P**

(43) Date of publication of application:
**27.06.2018 Bulletin 2018/26**

(73) Proprietor: **Ipsen Biopharm Ltd.**
**Wrexham Industrial Estate**
**Wrexham**
**LL13 9UF (GB)**

(72) Inventors:
- **BLANCHETTE, Sarah, F.**
  **Lynnfield, MA 01940 (US)**
- **DRUMMOND, Daryl, C.**
  **Lincoln, MA 01773 (US)**
- **FITZGERALD, Jonathan, Basil**
  **Arlington, MA 02474 (US)**
- **MOYO, Victor**
  **Ringoes, NJ 08551 (US)**

(74) Representative: **Carpmaels & Ransford LLP**
**One Southampton Row**
**London WC1B 5HA (GB)**

(56) References cited:
- **MESSERER CORRIE LYNN ET AL: "Liposomal irinotecan: formulation development and therapeutic assessment in murine xenograft models of colorectal cancer", CLINICAL CANCER RESEARCH : AN OFFICIAL JOURNAL OF THE AMERICAN ASSOCIATION FOR CANCER RESEARCH, vol. 10, no. 19, 1 October 2004 (2004-10-01), pages 6638-6649, XP002763354, ISSN: 1078-0432**
- **SYBIL M GENTHER WILLIAMS ET AL: "Treatment with the PARP inhibitor, niraparib, sensitizes colorectal cancer cell lines to irinotecan regardless of MSI/MSS status", CANCER CELL INTERNATIONAL, vol. 15, no. 1, 4 February 2015 (2015-02-04), page 14, XP021213062, BIOMED CENTRAL, LONDON, GB ISSN: 1475-2867, DOI: 10.1186/S12935-015-0162-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DAVIDSON DAVID ET AL: "The PARP inhibitor ABT-888 synergizes irinotecan treatment of colon cancer cell lines", INVESTIGATIONAL NEW DRUGS, vol. 31, no. 2, April 2013 (2013-04), pages 461-468, XP002763355, ISSN: 1573-0646
- DOUILLARD J ET AL: "Irinotecan combined with fluorouracil compared with fluorouracil alone as first-line treatment for metastatic colorectal cancer: a multicentre randomised trial", THE LANCET, vol. 355, no. 9209, 25 March 2000 (2000-03-25), pages 1041-1047, XP004814762, THE LANCET PUBLISHING GROUP, GB ISSN: 0140-6736, DOI: 10.1016/S0140-6736(00)02034-1
- HARE JENNIFER I ET AL: "Treatment of colorectal cancer using a combination of liposomal irinotecan (Irinophore C(TM)) and 5-fluorouracil", PLOS ONE, vol. 8, no. 4, E623491, 23 April 2013 (2013-04-23), pages 1-12, XP002763356, ISSN: 1932-6203, DOI: 10.1371/journal.pone.0062349

## Description

### Field of Invention

This invention is defined in the appended claims and relates to liposomal irinotecan for use in an antineoplastic therapy for the treatment of a solid tumor in a patient, wherein the liposomal irinotecan is administered in combination with a Poly(ADP-ribose) polymerase (PARP) inhibitor.

### Background of the Invention

Topoisomerase I (Top1) inhibitors have proven valuable as anticancer agents, with regulatory approvals for topotecan in cervical, ovarian, and small cell lung cancer, and for irinotecan in colorectal cancer. Type 1 topoisomerases are enzymes that cut one strand of DNA to allow relaxation during DNA replication and repair. The inhibitors create a stable complex of DNA and Top1 that induces DNA damage. Preclinical strategies to improve the activity of Top1 inhibitors are aimed at increasing the level of DNA damage to promote cell death.

Nanoliposomal irinotecan (nal-IRI) is a highly stabilized liposomal formulation of irinotecan that provides for sustained exposure of irinotecan, and the active metabolite SN-38, in the tumor to a higher proportion of cells during the more sensitive S-phase of the cell cycle. Nal-IRI has shown promising preclinical and clinical activity in a range of cancer types, and was recently approved in the United States in combination with 5-FU/LV for patients with metastatic adenocarcinoma of the pancreas after disease progression following gemcitabine-based therapy.

Poly(ADP-ribose) polymerase (PARP) inhibitors are a new class of chemotherapeutic agents currently in development for the treatment of various cancer types. PARPs are a family of enzymes involved in DNA repair. Inhibition of the repair pathway results in cell death.

Combinations of PARP and Top1 inhibitors have shown to be synergistic in *in vitro* assays. However, the clinical development of PARP inhibitor and Top1 inhibitor combinations has been limited due to increased toxicities and resultant dose reductions, thereby limiting the potential clinical utility of the combination. For example, significant myelosuppression was seen in a dose-escalation study of veliparib and topotecan, wherein the maximum tolerated dose was exceeded at the first planned dose level. Most PARP inhibitors are being developed to date solely as monotherapies.

As a result, there is a need for methods to safely and effectively combine a PARP inhibitor with a Top1 inhibitor to treat cancer. The present disclosure addresses this need and provides additional benefits. Messerer et al. (Clin Cancer Res. 2004 Oct 1;10(19):6638-49) discusses "Liposomal irinotecan: formulation development and therapeutic assessment in murine xenograft models of colorectal cancer". Genther Williams et al. (Cancer Cell International 2015 15:14) states that "Treatment with the PARP inhibitor, niraparib, sensitizes colorectal cancer cell lines to irinotecan regardless of MSI/MSS status". Davidson et al. (Invest New Drugs, 2013 Apr;31(2):461-8. doi: 10.1007/s10637-012-9886-7. Epub 2012 Oct 9.) states that "The PARP inhibitor ABT-888 synergizes irinotecan treatment of colon cancer cell lines".

### Summary

The invention relates to liposomal irinotecan for use in an antineoplastic therapy for the treatment of a solid tumor in a patient, wherein the liposomal irinotecan is administered in combination with a Poly(ADP-ribose) Polymerase (PARP) inhibitor , wherein the liposomal irinotecan is repeatedly administered, and wherein the treatment comprises:

i) administering to the patient liposomal irinotecan once every two weeks; and

ii) administering the PARP inhibitor daily for 3 to 10 days between consecutive administrations of the liposomal irinotecan, wherein the PARP inhibitor is administered at least 2, 3, 4 or 5 days after the administration of the liposomal irinotecan and at least 2, 3, 4 or 5 days prior to the next administration of the liposomal irinotecan. The invention also relates to liposomal irinotecan for use in an antineoplastic therapy for the treatment of a solid tumor in a patient, wherein the liposomal irinotecan is administered in combination with a Poly(ADP-ribose) Polymerase (PARP) inhibitor, the treatment comprising a 28-day antineoplastic therapy treatment cycle consisting of:

  i) administering the liposomal irinotecan on days 1 and 15 of the treatment cycle, and

  ii) administering the PARP inhibitor on one or more days starting at least 3 days after the liposomal irinotecan and ending at least 1 day prior to administration of additional liposomal irinotecan. Preferred embodiments of the invention are defined in the dependent claims.

The present disclosure provides for methods of administering with reduced peripheral toxicity a combination of a Top1 inhibitor and a PARP inhibitor to a tumor. In one aspect, a method of treating a cancer, e.g., a malignant tumor, is provided, the method comprising a treatment regimen comprising one or more instances of co-administration of the

Top1 inhibitor and the PARP inhibitor, each instance of co-administration comprising: (a) administering to a patient in need thereof an effective amount of an irinotecan liposomal formulation; and (b) after completion of the administration of the Top1 inhibitor, administering to the patient an effective amount of a PARP inhibitor, wherein the PARP inhibitor is administered to the patient following an interval that allows for a reduction in peripheral toxicity as compared to simultaneous administration of the Top1 inhibitor and the PARP inhibitor.

The present disclosure provides methods of treating cancer by administering a topoisomerase inhibitor and a PARP inhibitor with reduced peripheral toxicity. This can be accomplished by administering the topoisomerase inhibitor in a form (e.g., liposomal irinotecan) that prolongs accumulation of the topoisomerase inhibitor in a tumor relative to sites outside the tumor, and then subsequently administering the PARP inhibitor(s) to the patient after an interval between the administration of the topoisomerase inhibitor and the PARP inhibitor. The interval can be selected to provide enough time for the topoisomerase inhibitor (e.g., irinotecan and/or its metabolite SN-38) to clear plasma or tissue outside of the tumor to a greater extent than inside the tumor. Preferably, the interval is an effective topoisomerase-1 inhibitor plasma clearing interval. As used herein, the term "effective topoisomerase-1 inhibitor plasma clearing interval" (e.g., irinotecan plasma clearing interval) is that interval between concluding the administration of a topoisomerase-1 inhibitor formulation (e.g., liposomal irinotecan) and initiating the administration of one or more PARP inhibitors, where the time interval is selected to allow sufficient clearance of the topoisomerase-1 inhibitor (e.g., irinotecan or its active metabolite SN-38) from the blood plasma (or peripheral tissue) but allows an effective quantity of the topoisomerase-1 inhibitor (e.g., irinotecan and/or SN38) to remain in one or more tumors within the patient during the subsequent administration of the PARP inhibitor in an amount effective to provide a desired effect on the tumor (e.g., heightened combined toxicity localized within the tumor). Preferably, the PARP inhibitor is administered after an irinotecan plasma clearing interval of 3-5 days (e.g., 3, 4 or 5 days) after completing the administration of liposomal irinotecan on days 1 and 15 during each of one or more 28-day treatment cycles.

Methods of treating cancer disclosed herein include the treatment of solid tumors. In certain examples, the cancer treated can be selected from the group consisting of cervical cancer, ovarian cancer, triple negative breast cancer, non-small cell lung cancer, small cell lung cancer, gastrointestinal stromal tumors gastric cancer, pancreatic cancer, colorectal cancer, and a neuroendocrine cancer. Preferably, the cancer is cervical cancer.

The topoisomerase inhibitor can be provided as a liposome formulation. The topoisomerase inhibitor used in the invention is liposomal irinotecan. The liposomal irinotecan can provide an irinotecan terminal elimination half-life of 26.8 hours and a maximal irinotecan plasma concentration of 38.0 micrograms/ml. In some examples, the liposomal irinotecan can include irinotecan sucrose octasulfate encapsulated within phospholipid vesicles having a size of about 110 nm. For example, the liposomal irinotecan can be the product ONIVYDE® (irinotecan liposome injection) (Merrimack Pharmaceuticals, Inc., Cambridge, MA), previously designated "MM-398." The PARP inhibitor can include one or more compounds selected from the group consisting of niraparib, olaparib, veliparib, and rucaparib, preferably veliparib or olaparib.

The topoisomerase-1 inhibitor is preferably a liposomal irinotecan (e.g., MM-398), which can be administered at dose of 80 mg/m$^2$ (salt) irinotecan once every 2 weeks in combination with a PARP inhibitor (e.g., veliparib, olaparib, niraparib or rucaparib) administered daily during each two week cycle starting 3-5 days after administration of liposomal irinotecan without administering the PARP inhibitor on days when the liposomal irinotecan is administered (e.g., without administering the PARP inhibitor 1, 2 or 3 days before the next liposomal irinotecan administration). Preferably, the PARP inhibitor is not administered within 3 days of (i.e., neither 3 days after nor 3 days before) the administration of liposomal irinotecan.

Specific methods of treating a cancer provided herein include administering an antineoplastic therapy consisting of the administration of liposomal irinotecan every 2 weeks (e.g., on days 1 and 15 of a 28-day treatment cycle), and the administration of a PARP inhibitor one or more times per day (e.g., twice per day) for one or more days (e.g., 7-9 days) starting at least 3 days (e.g., 3, 4 or 5 days) after each administration of the liposomal irinotecan, without administering other antineoplastic agents during the antineoplastic therapy. For example, one antineoplastic therapy is a 28-day treatment cycle consisting of: administering 70 mg/m$^2$ ONIVYDE/MM-398 liposomal irinotecan (free base) on days 1 and 15, and administering a therapeutically effective amount of the PARP inhibitor (e.g., 50-400 mg twice per day for veliparib) on each of days 5-12 and days 19-25 of the treatment cycle, where no other antineoplastic agent is administered during the treatment cycle. Another antineoplastic therapy is a 28-day treatment cycle consisting of: administering 70 mg/m$^2$ ONIVYDE/MM-398 liposomal irinotecan (free base) on days 1 and 15, and administering a therapeutically effective amount of the PARP inhibitor (e.g., 50-400 mg twice per day for veliparib) on each of days 3-12 and days 17-25 of the treatment cycle, where no other antineoplastic agent is administered during the treatment cycle.

Liposomal irinotecan and a PARP inhibitor can be combined in an antineoplastic therapy for the treatment of a solid tumor, comprising a 28-day antineoplastic therapy treatment cycle consisting of: administering the liposomal irinotecan on days 1 and 15 of the treatment cycle, and administering the PARP inhibitor on one or more days starting at least 3 days after the liposomal irinotecan and ending at least 1 day prior to administration of additional liposomal irinotecan. In some embodiments, the PARP inhibitor is not administered for at least 3 days after the administration of

liposomal irinotecan. For example, the PARP inhibitor can be administered on one or more of days 5-12 of the antineoplastic therapy treatment cycle, and administered on one or more of days 19-25 of the antineoplastic therapy treatment cycle. In some embodiments, the PARP inhibitor is administered on one or more of days 3-12 of the antineoplastic therapy treatment cycle, and administered on one or more of days 17-25 of the antineoplastic therapy treatment cycle. In some embodiments, the PARP inhibitor is not administered within 3 days before or after the administration of the liposomal irinotecan. In addition, therapeutically effective doses of the topoisomerase inhibitor and PARP inhibitor compounds are provided herein. In some embodiments, each administration of liposomal irinotecan is administered at a dose of 80 mg/m$^2$ (salt) of ONIVYDE/MM-398. In some embodiments, each administration of the PARP inhibitor is administered at a dose of from about 20 mg/day to about 800 mg/day. Each administration of the PARP inhibitor can be administered once or twice daily at a dose of from about 20 mg/day to about 400 mg/day.

Without wishing to be bound by any particular theory of operation, it is believed that such an interval allows time for sufficient clearance of the Top1 inhibitor (e.g., either or both of irinotecan and SN-38) from the blood plasma to avoid peripheral toxicity due to the synergistic toxic effects of the combination of Top1 inhibitor and PARP inhibitor, while allowing an effective quantity of Top1 inhibitor to remain in one or more tumors within the patient for the subsequent administration of the PARP inhibitor to have a desired synergistic therapeutic effect.

This treatment regimen provides one or more attributes, which may include increased efficacy of the combination as compared to single agent treatment; reduced side effects, dosing the drugs at a higher dose compared with administration of the combination of a PARP inhibitor and a non-liposomal Top1 inhibitor.

Further aspects include providing an existing standard of care therapy to the patients, which may or may not include treatment with appropriate single agents. In some instances, the standard of care may include administration of a PARP inhibitor compound.

Thus, in one aspect, the present disclosure provides a method of treating a patient with cancer and having a tumor, the method comprising:

i. parenterally (e.g., intravenously) administering to the patient an effective amount of an irinotecan liposomal formulation; and
ii. administering to the patient an effective amount of a PARP inhibitor wherein the PARP inhibitor is administered after an effective irinotecan plasma clearing interval.

As disclosed herein, the administration of a PARP inhibitor can be delayed after administration of liposomal irinotecan for a period of time after administering liposomal irinotecan to allow for the treatment of cancerous tumors.

**Brief Description of the Drawings**

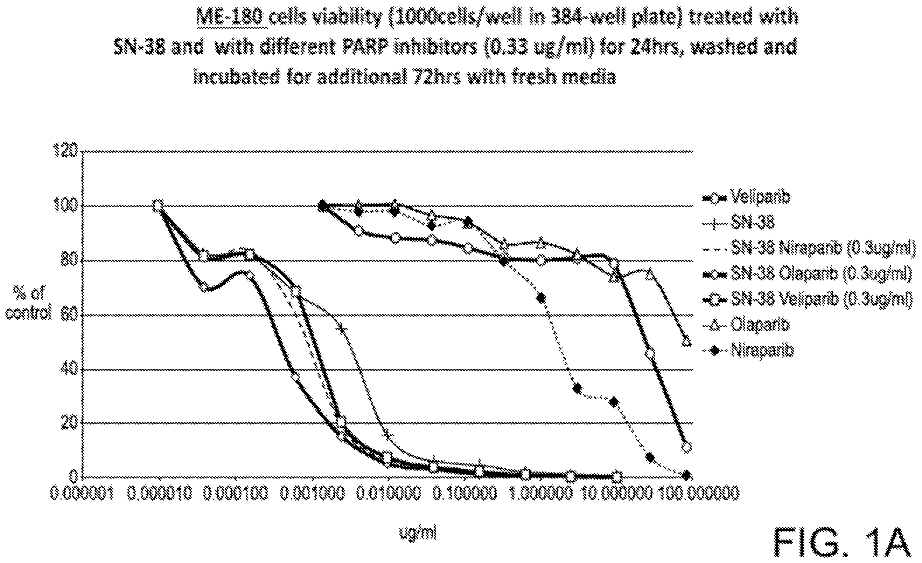

Figure 1A is a graph showing the results of a cell viability *in vitro* measurement of ME-180 human cervical cancer cells treated with the topoisomerase 1 inhibitor SN-38 and various PARP inhibitors.

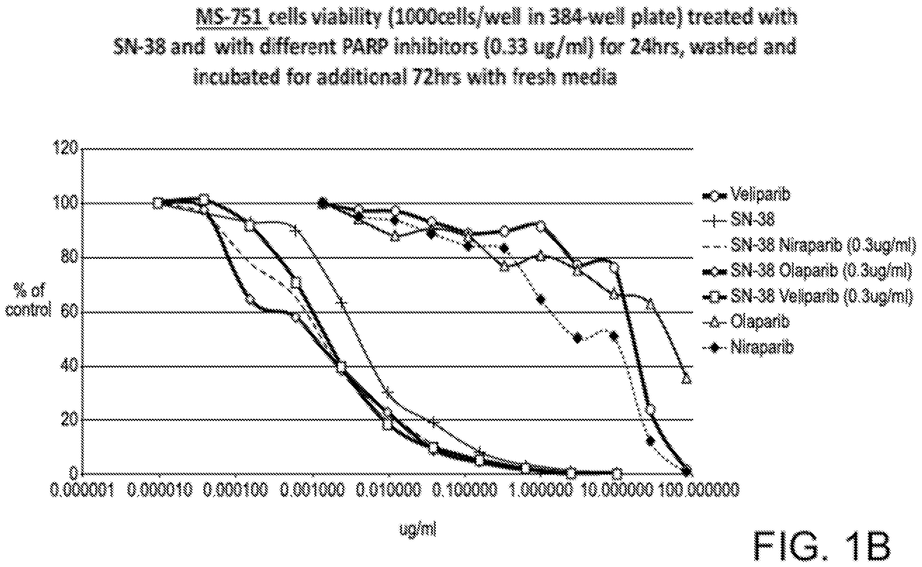

Figure 1B is a graph showing the results of a cell viability *in vitro* measurement of MS-751 human cervical cancer cells treated with the topoisomerase 1 inhibitor SN-38 and various PARP inhibitors.

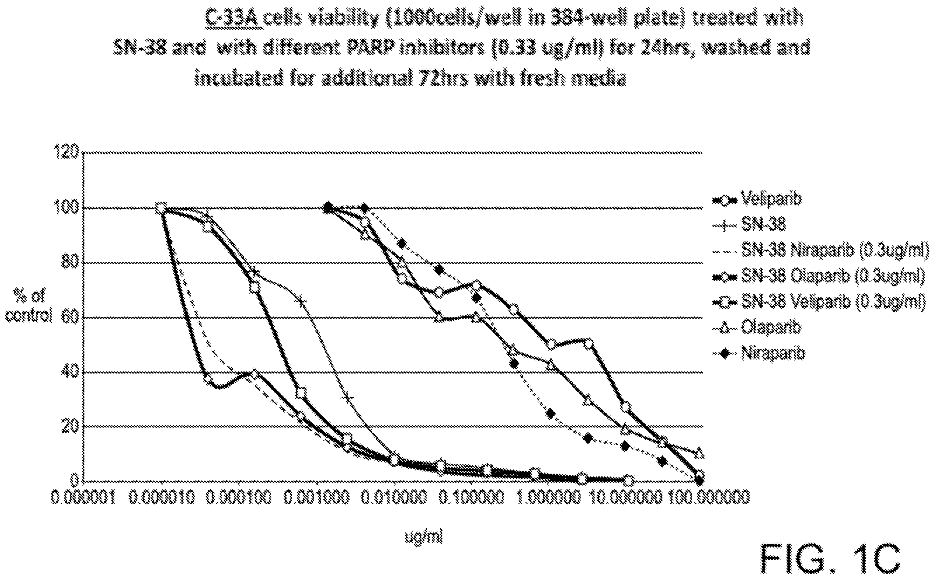

Figure 1C is a graph showing the results of a cell viability *in vitro* measurement of C-33A human cervical cancer cells treated with the topoisomerase 1 inhibitor SN-38 and various PARP inhibitors.

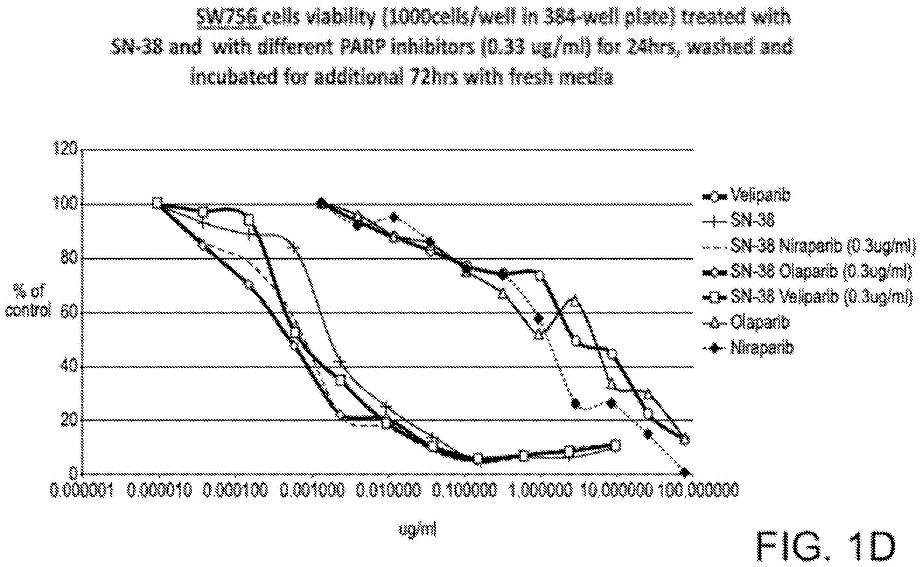

Figure 1D is a graph showing the results of a cell viability *in vitro* measurement of SW756 human cervical cancer cells treated with the topoisomerase 1 inhibitor SN-38 and various PARP inhibitors.

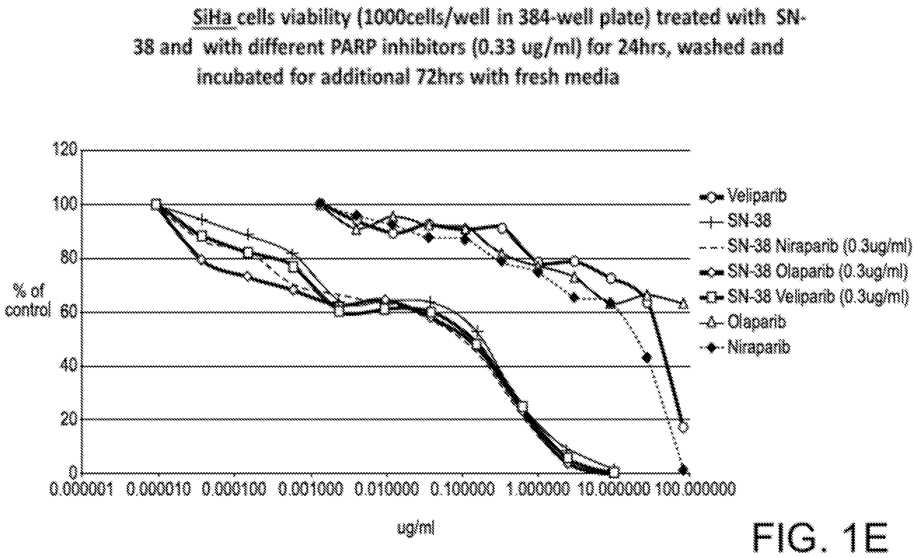

Figure 1E is a graph showing the results of a cell viability *in vitro* measurement of SiHa human cervical cancer cells treated with the topoisomerase 1 inhibitor SN-38 and various PARP inhibitors.

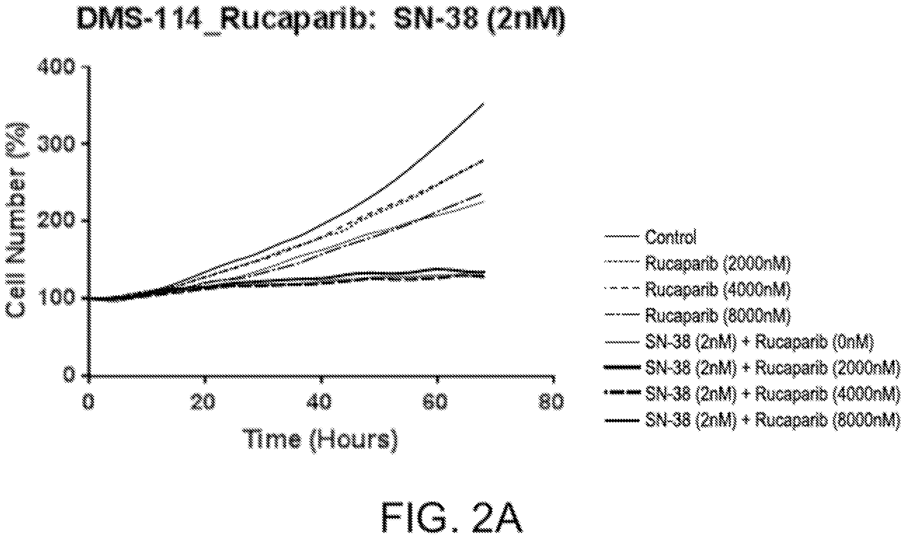

Figure 2A is a graph showing the results of *in vitro* measurement of % cell number over time for DMS-114 small cell lung cancer cells treated with the topoisomerase inhibitor SN-38 and the PARP inhibitor rucaparib.

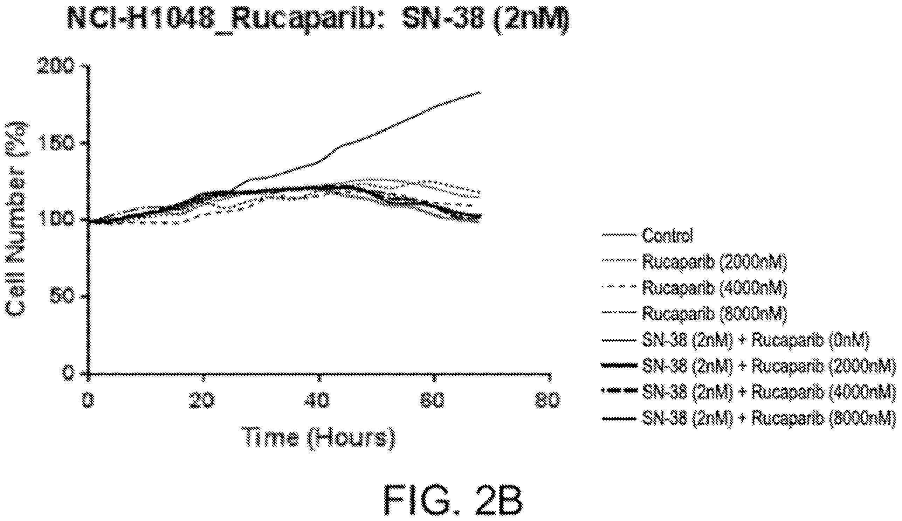

Figure 2B is a graph showing the results of *in vitro* measurement of % cell number over time for NCI-H1048 small cell lung cancer cells treated with the topoisomerase inhibitor SN-38 and the PARP inhibitor rucaparib.

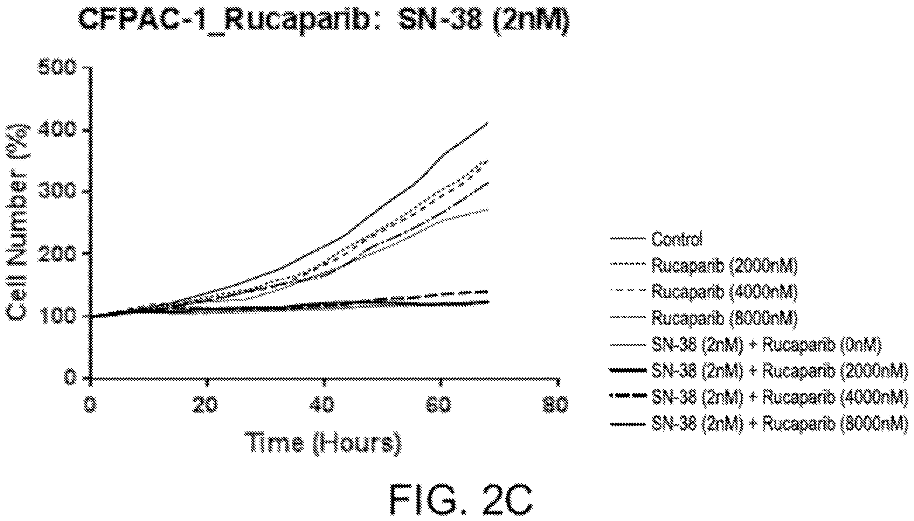

Figure 2C is a graph showing the results of *in vitro* measurement of % cell number over time for CFPAC-1 pancreatic cancer cells treated with the topoisomerase inhibitor SN-38 and the PARP inhibitor rucaparib.

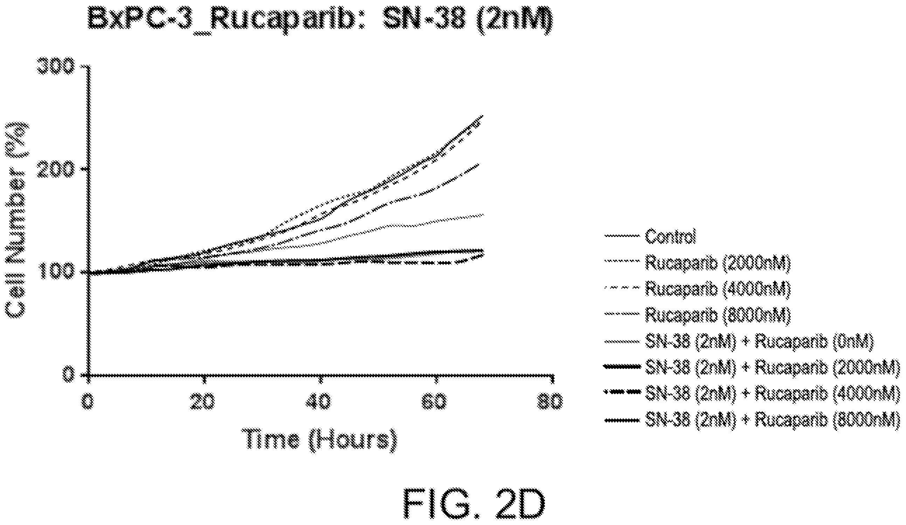

Figure 2D is a graph showing the results of *in vitro* measurement of % cell number over time for BxPC-3 pancreatic cancer cells treated with the topoisomerase inhibitor SN-38 and the PARP inhibitor rucaparib.

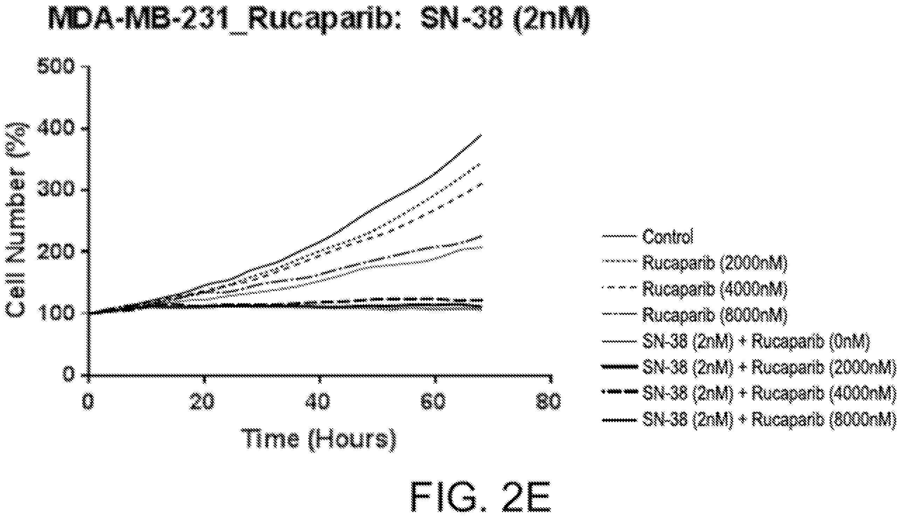

Figure 2E is a graph showing the results of *in vitro* measurement of % cell number over time for MDA-MB-231 triple negative breast cancer (TNBC) cancer cells treated with the topoisomerase inhibitor SN-38 and the PARP inhibitor rucaparib.

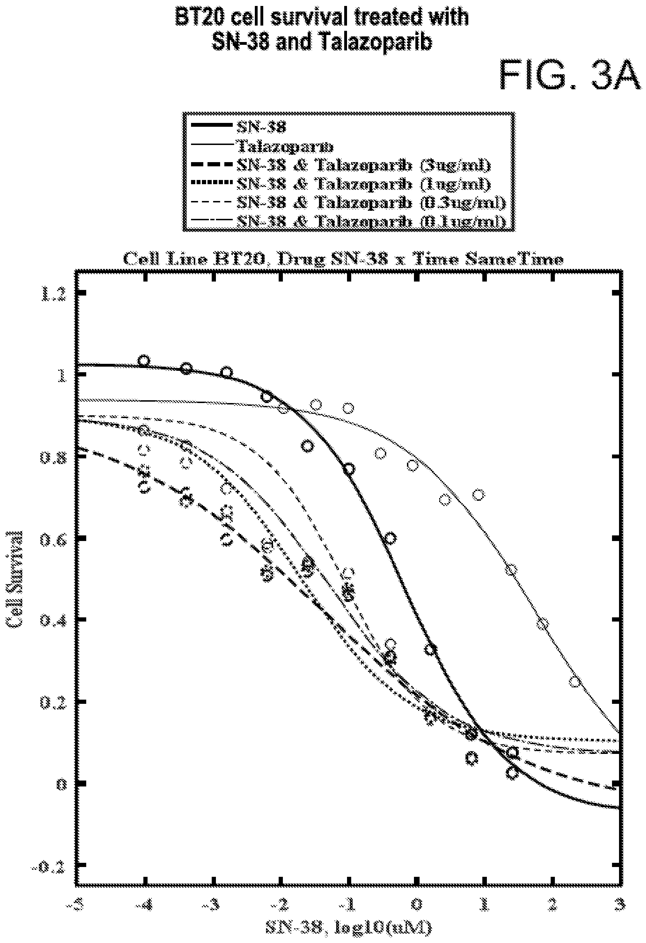

Figure 3A is a graph showing the results of *in vitro* measurement of cell survival for BT-20 triple negative breast

cancer (TNBC) cancer cells treated with the topoisomerase inhibitor SN-38 and the PARP inhibitor talazoparib.

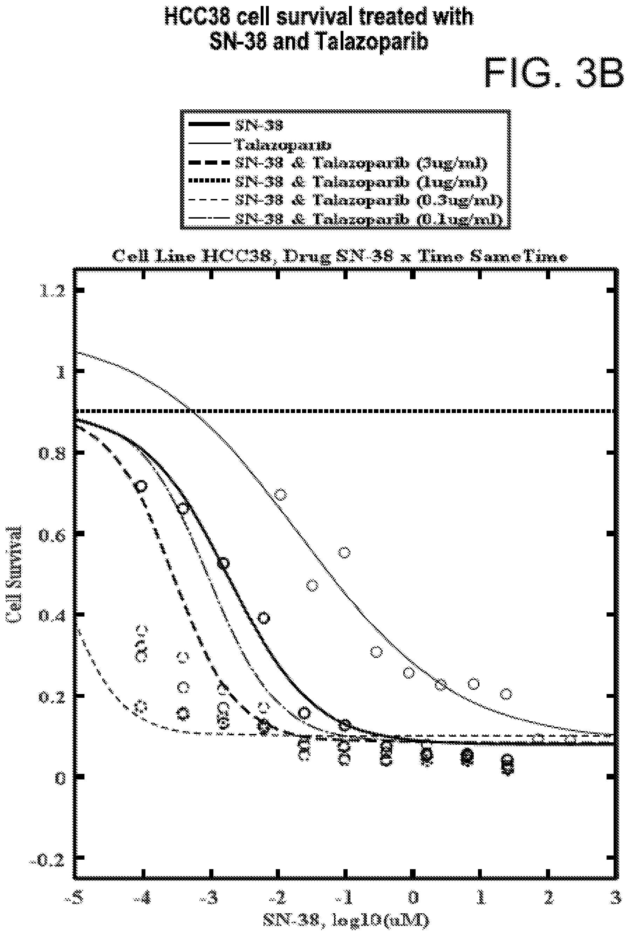

Figure 3B is a graph showing the results of *in vitro* measurement of cell survival for HCC38 triple negative breast cancer (TNBC) cancer cells treated with the topoisomerase inhibitor SN-38 and the PARP inhibitor talazoparib.

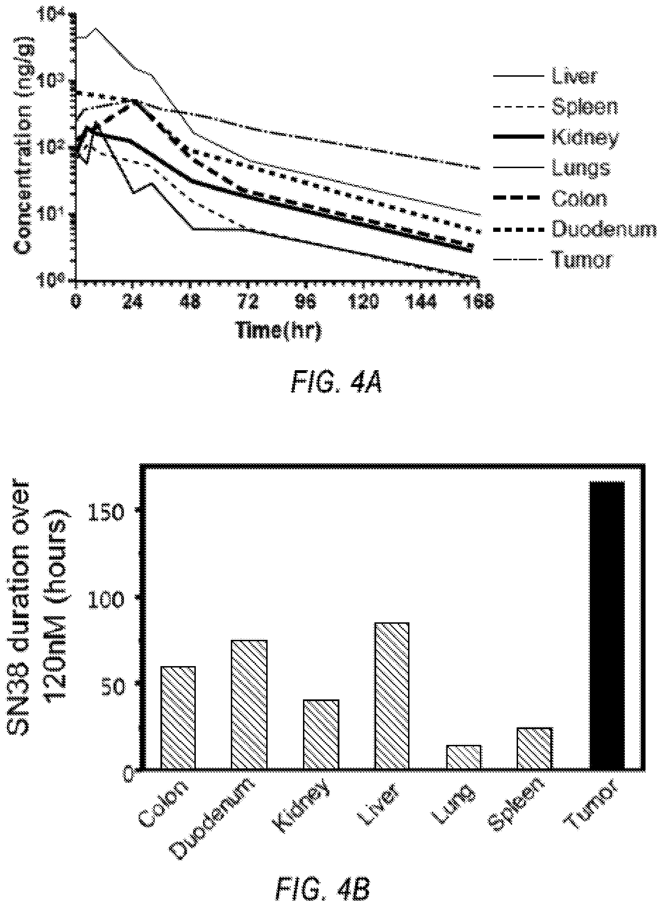

Figures 4A and 4B are graphs depicting the prolonged accumulation of SN-38 seen in tumor after MM-398 administration compared to other organs. (A) HT-29 colorectal cancer (CRC) tumor xenograft-bearing mice were injected intravenously (IV) with MM-398 at a dose of 20 mg/kg and following a single injection, tissue samples were collected at various time points (1, 4, 8, 24, 48, 72, 168 hours). HPLC analysis was used to measure the levels of SN-38 in these samples. (B) Time of SN-38 duration over a threshold of 120 nmol/L was computed from the pharmacokinetic profiles of SN-38 in tumor and normal tissues following 20 mg/kg of MM-398.

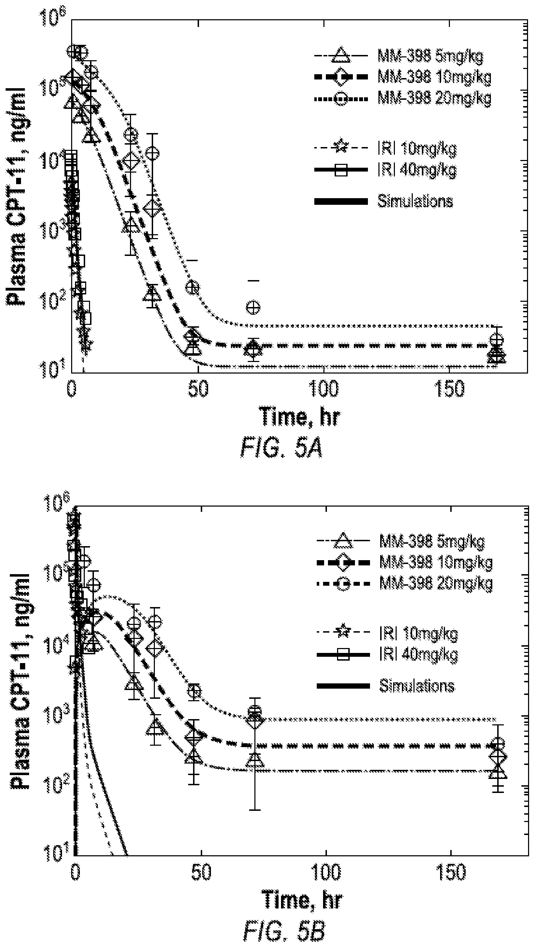

Figures. 5A-5D are graphs showing the MM-398 PK parameters in a murine HT-29 colorectal cancer (CRC) xenograft study. Plasma CPT-11 levels (A) or SN-38 levels (B) following IV injections of various doses of MM-398 or free irinotecan. Tumor CPT-11 levels (C) or SN-38 levels (D) were calculated at various time points following dosing with equivalent doses of either free irinotecan (red) or MM-398 (blue). HPLC analysis was used to measure the levels of the CPT-11 and its metabolite SN-38 in these samples.

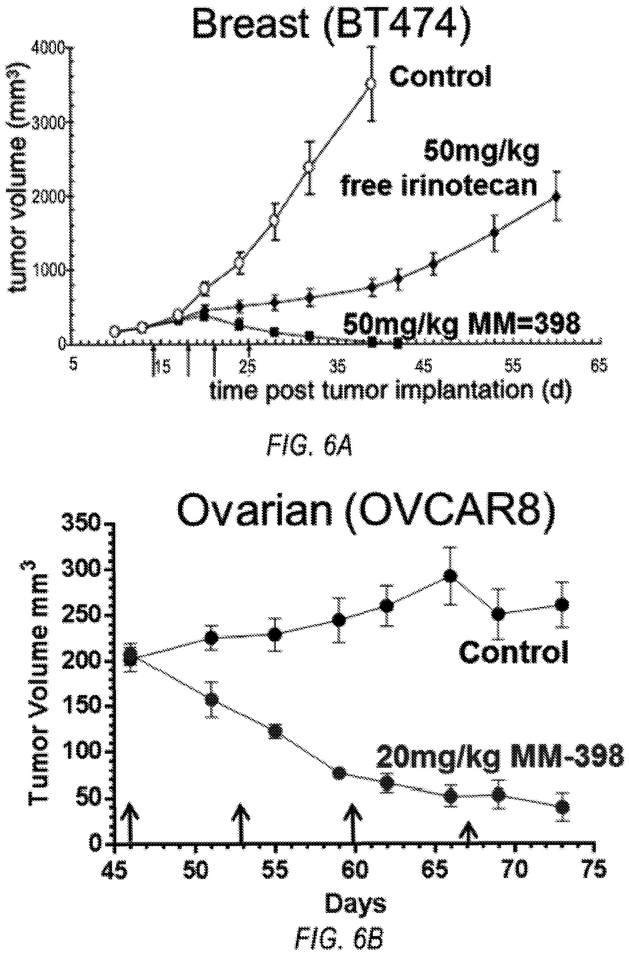

Figures. 6A-6D are graphs showing the efficacy of MM-398 in various cancer models. Cancer cells were implanted subcutaneously in mice; when tumors were well established and had reached mean volumes of 200 $mm^3$, IV treatment with free irinotecan, MM-398 or control was initiated. The doses of free and nanoliposomal irinotecan used in each study are indicated above, with dose time points indicated by arrows. (A) BT474 breast cancer model treated with control (o), drug- and liposome-free vehicle only; free CPT-11 (●); or nanoliposomal CPT-11 (■). (B) OVCAR8 ovarian cancer model treated with control (black) or MM-398 (blue). (C) HT-29 CRC model treated with control (black), free irinotecan (red) or MM-398 (blue). (D) An orthotopic pancreatic tumor xenograft model dosed with control (PBS, black), free irinotecan (red), or MM-398 (blue).

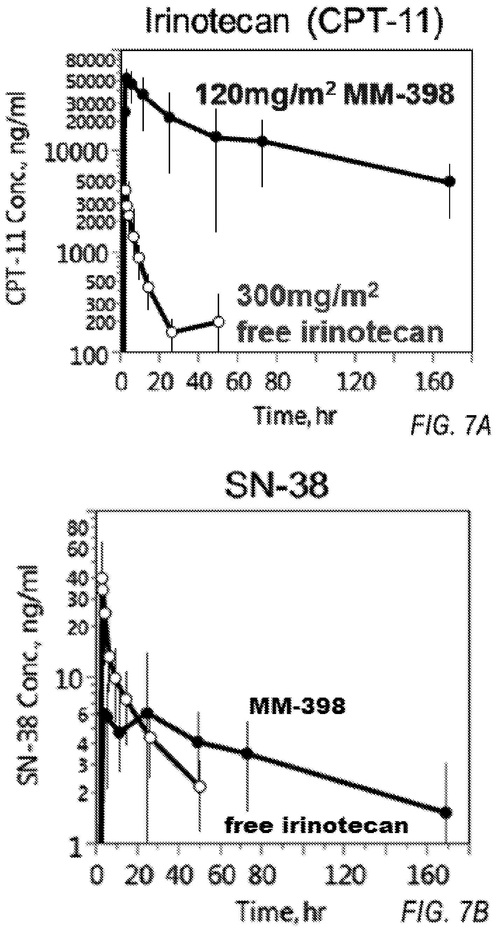

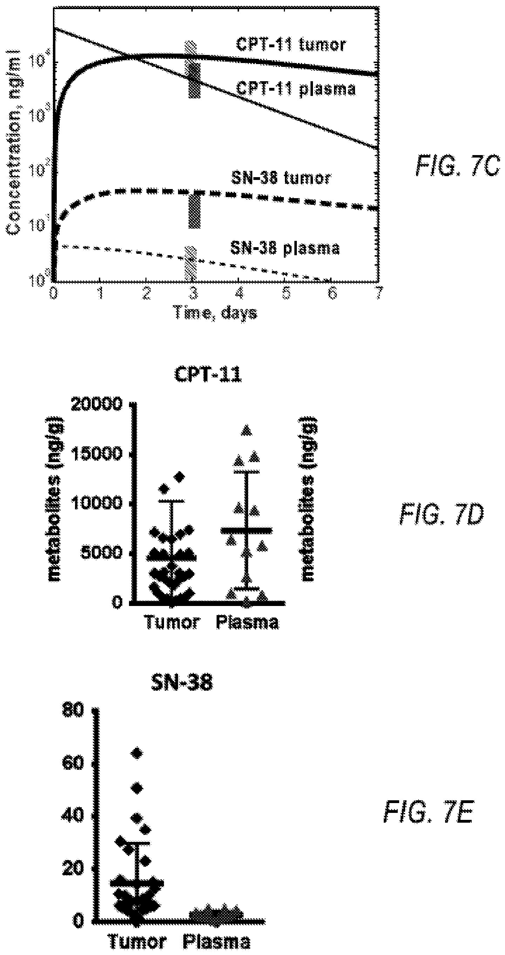

Figures 7A and 7B are graphs depicting PK analysis from a Phase II clinical study. Gastric cancer patients received either MM-398 at a dose of 120 $mg/m^2$ (dark grey line) or free irinotecan at a dose of 300 $mg/m^2$ (lighter grey line) every 3 weeks. CPT-11 (A) and its active metabolite, SN-38 (B) were measured during Cycle 1. Figures 7C-7E depicts clinical evidence for local activation and accumulation of SN-38 in tumor tissue. (C) The mechanistic tumor PK model of nal-IRI predicted higher SN-38 levels in tumor compared to plasma. The range of actual data, collected from a Phase I study of patients (n=12) with advanced solid tumors, is indicated by the black (tumor) or grey (plasma) vertical bars. (D) CPT-11 levels and (E) SN-38 levels, as measured from patient tumor (black) and plasma (grey) samples collected 72h post-MM-398 infusion.

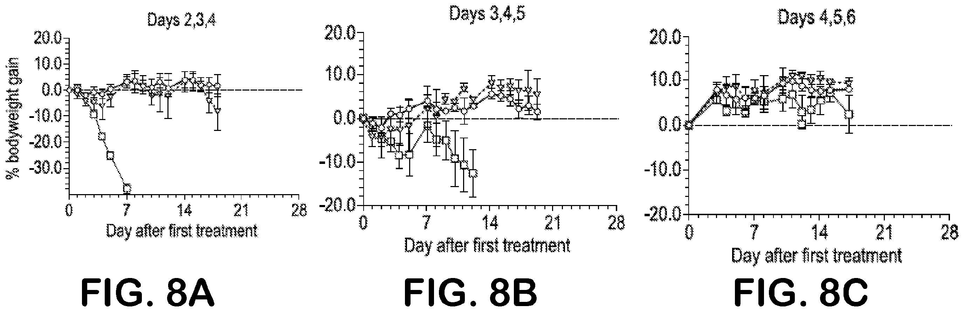

Figure 8 depicts a dose tolerability study of MM-398 + veliparib combination in mice. All mice were dosed chronically once weekly on day 1, with veliparib subsequently dosed for 3 consecutive days either on days 2-4 (A), days 3-5 (B), or days 4-6 (C). Mice were weighed daily and % bodyweight gain is indicated on the Y-axis. Weight loss is indicative of intolerability of the combination. In more detail Figure 8A is a graph showing the results of a murine tolerability study measuring % change in bodyweight after administration of liposomal irinotecan (15 mg/kg, 28 mg/kg, or 50- mg/kg of MM398 (salt) once weekly) followed by administration of 50 mg/kg veliparib daily on days 2, 3 and 4 after MM-398 administration.

Figure 8B is a graph showing the results of a murine tolerability study measuring % change in bodyweight after administration of different doses of liposomal irinotecan (15 mg/kg, 28 mg/kg, or 50 mg/kg of MM398 (salt) once weekly) followed by administration of 50 mg/kg veliparib daily on days 3, 4 and 5 after MM-398 administration.

Figure 8C is a graph showing the results of a murine tolerability study measuring % change in bodyweight after administration of different doses of liposomal irinotecan (15 mg/kg, 28 mg/kg, or 50- mg/kg of MM398 (salt) once weekly) followed by administration of 50 mg/kg veliparib daily on days 4, 5 and 6 after MM-398 administration.

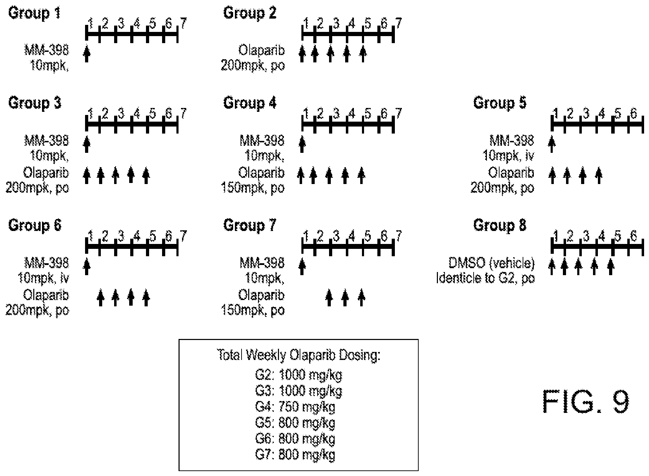

Figure 9 depicts a graphical representation of a murine tolerability study design comparing MM-398 and olaparib as a monotherapy or in combination using a fixed dose of MM-398 and varying doses of olaparib, with various dosing schedules for different groups.

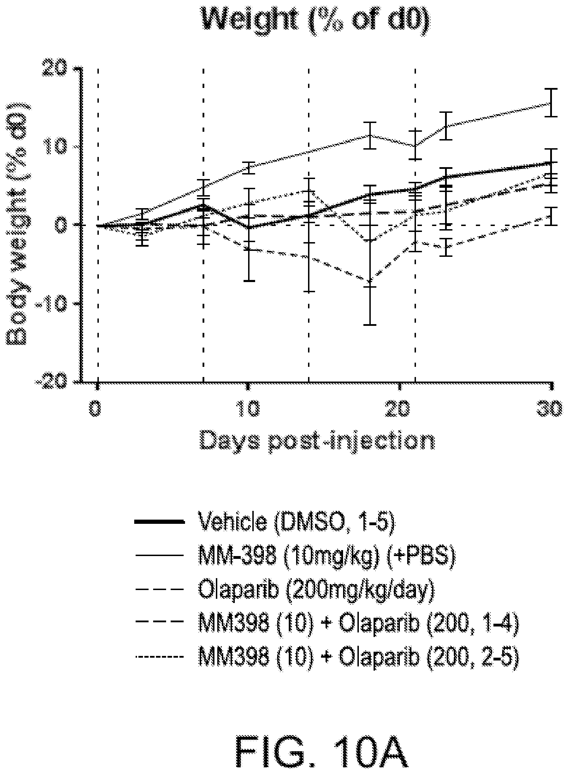

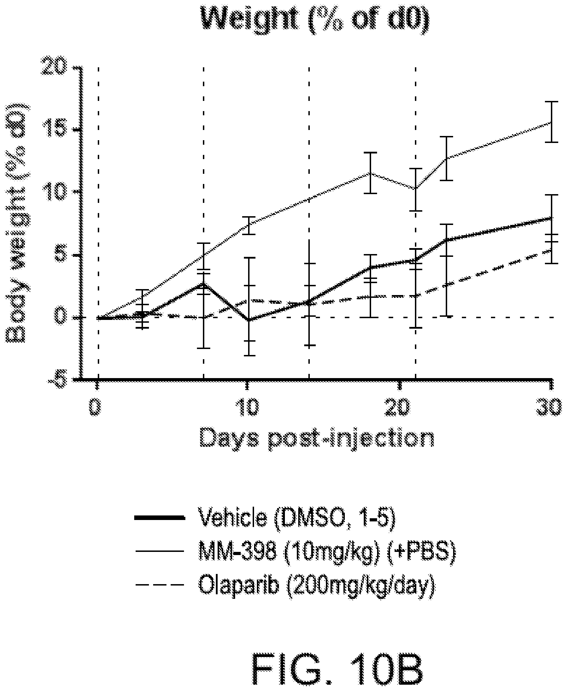

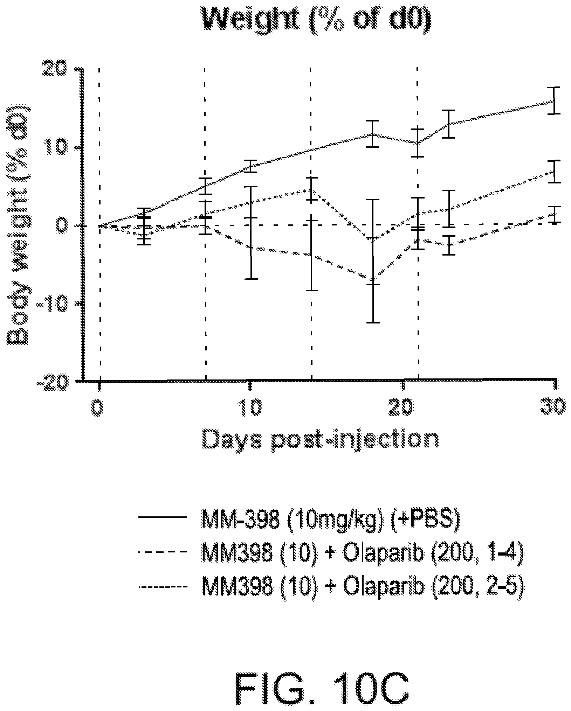

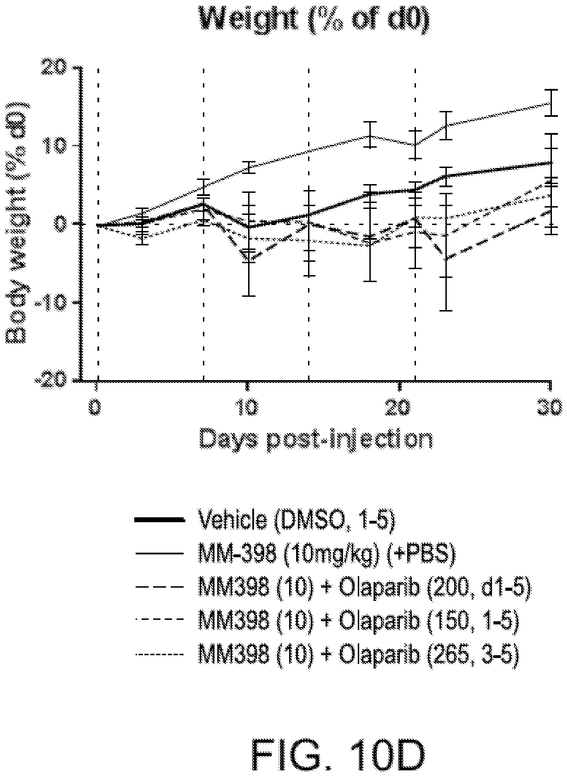

Figure 10A-D is a series of graphs graph showing the results of a murine tolerability study measuring % change in bodyweight after administration of liposomal irinotecan (10mg/kg), olaparib alone, and combinations of MM-398 with different dosing schedules of olaparib.

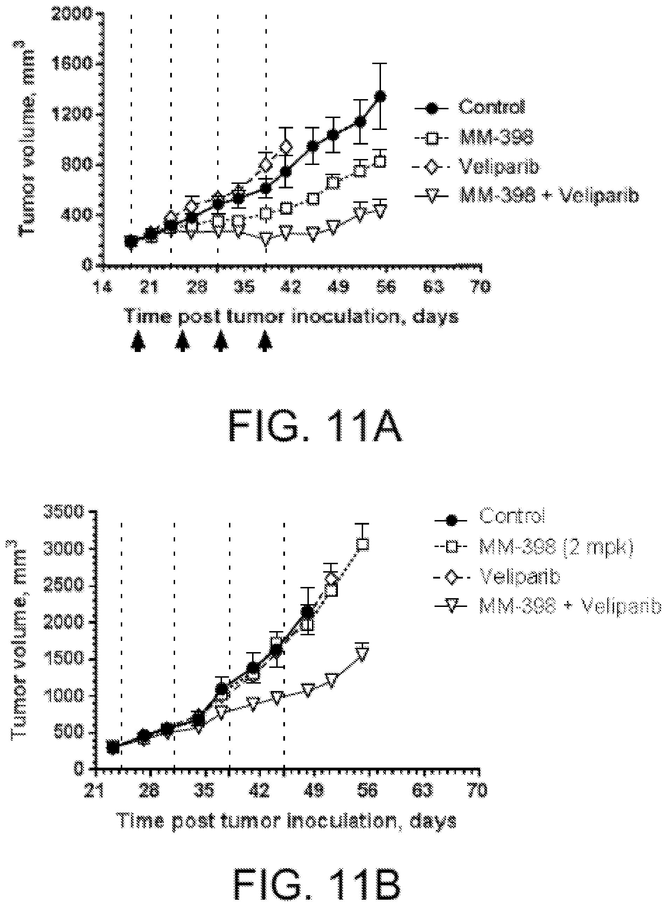

Figure 11 shows that the combination of MM-398 + veliparib is synergistic. Two different cervical cancer xenograft models were utilized to study the efficacy of MM-398 dosed once weekly on Day 1 (blue arrows), veliparib dosed at 50 mg/kg orally once daily for 3 consecutive days on Days 4-6 of each week, or the combination dosed on the same schedule as the single agent treatments combined. (A) MS751 cervical cancer xenograft model using MM-398 dosed at 5 mg/kg and (B) C33A cervical cancer xenograft model using MM-398 dosed at 2 mg/kg.

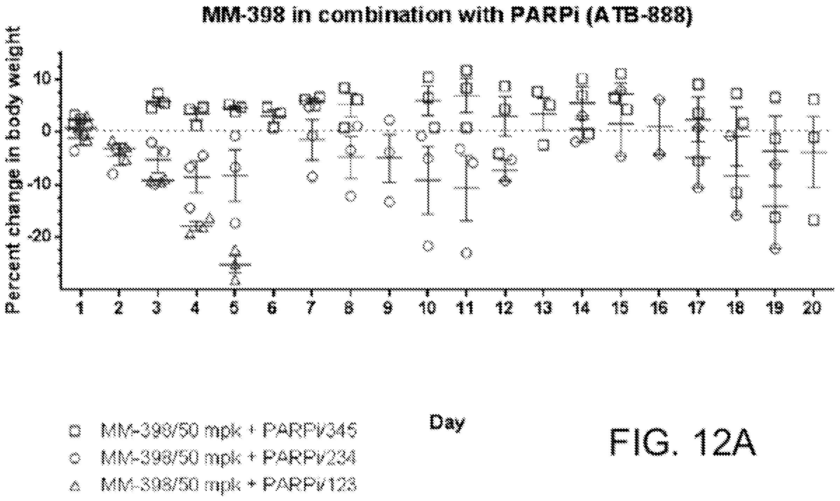

Figure 12A depicts the *in vivo* tolerability of MM-398 in combination with veliparib results, with an adjusted lower limit, bar is SEM. In particular it depicts the *in vivo* tolerability of 50 mg/kg dose of MM-398 in combination with 50 mg/kg veliparib given on days 1, 2 3 or 2, 3, 4 or 3, 4, 5 after administration of the MM-398.

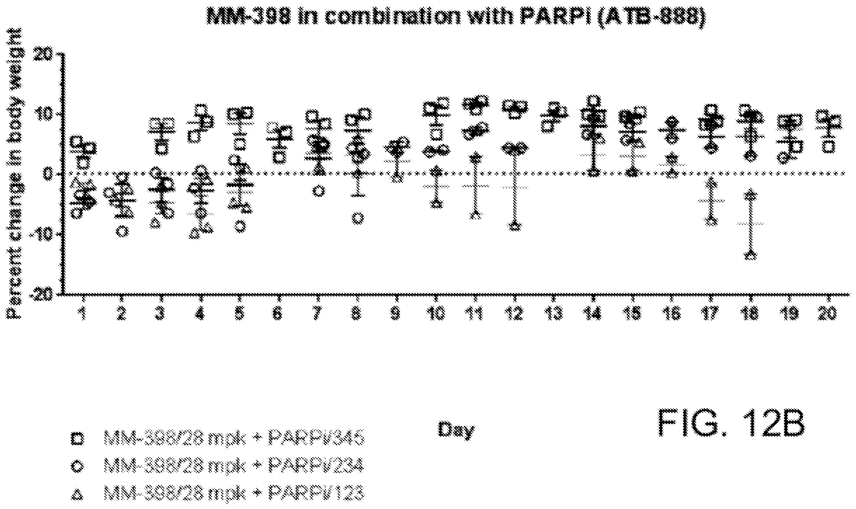

Figure 12B depicts the *in vivo* tolerability of MM-398 in combination with veliparib results. In particular it depicts the

*in vivo* tolerability of 28 mg/kg dose of MM-398 in combination with 50 mg/kg veliparib given on days 1, 2 3 or 2, 3, 4 or 3, 4, 5 after administration of the MM-398.

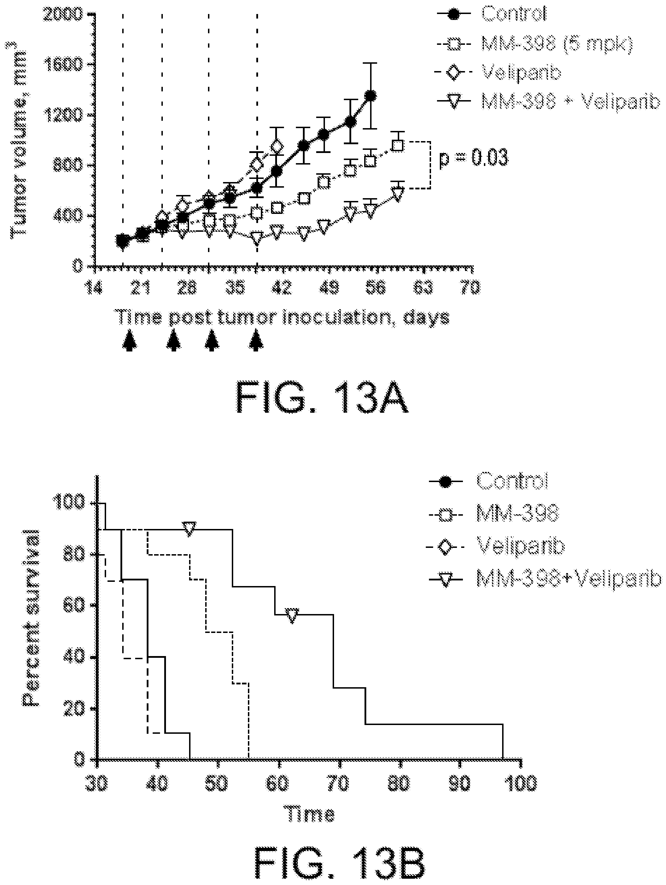

Figure 13A shows antitumor efficacy of MM-398 in combinations with veliparib in MS751 xenograft model where veliparib was dosed 72 h following MM-398 (nal-IRI) administration. In particular, the graph shows data from a mouse xenograft study using MS751 cervical cancer cells in a murine model treated with liposomal irinotecan (5 mg/kg MM398) and/or the PARP inhibitor veliparib (50 mpk) on days 3-5 starting after administration of MM398.

Figure 13B depicts the effect of MM-398 in combinations with veliparib in MS751 xenograft model on the animal's survival. In particular, the graph shows survival data from a mouse xenograft study using MS751 cervical cancer cells in a murine model treated with liposomal irinotecan (5 mg/kg MM398) and/or the PARP inhibitor veliparib (50 mpk) on days 3-5 starting after administration of MM398.

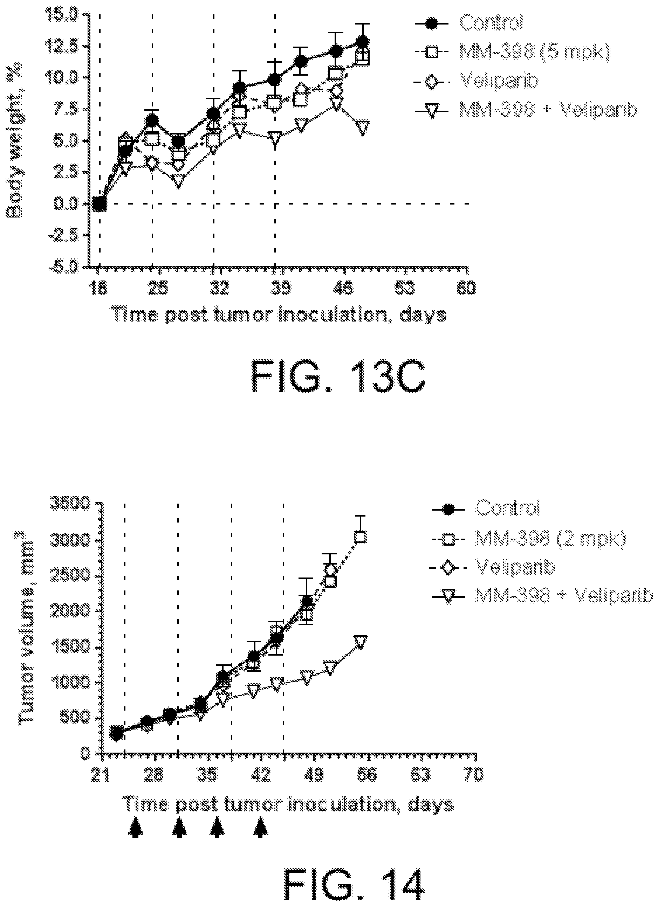

Figure 13C depicts the effect of MM-398 in combinations with veliparib in MS751 xenograft model and on body weight, where veliparib was dosed 72 h following MM-398 (nal-IRI) administration. In particular, the graph depicts the effect of MM-398 in combinations with veliparib in MS751 xenograft murine model treated with liposomal irinotecan (5 mg/kg MM398) and/or the PARP inhibitor veliparib (50 mpk) on days 3-5 starting after administration of MM398.

Figure 14 depicts the antitumor efficacy of MM-398 in combinations with veliparib in C33A xenograft model, where veliparib was dosed 72 h following MM-398 (nal-IRI) administration. The mouse xenograft study using C33 cervical cancer cells is in a murine model treated with liposomal irinotecan (2 mg/kg MM398) and/or the PARP inhibitor veliparib (50 mpk) on days 3-5 starting after administration of MM398.

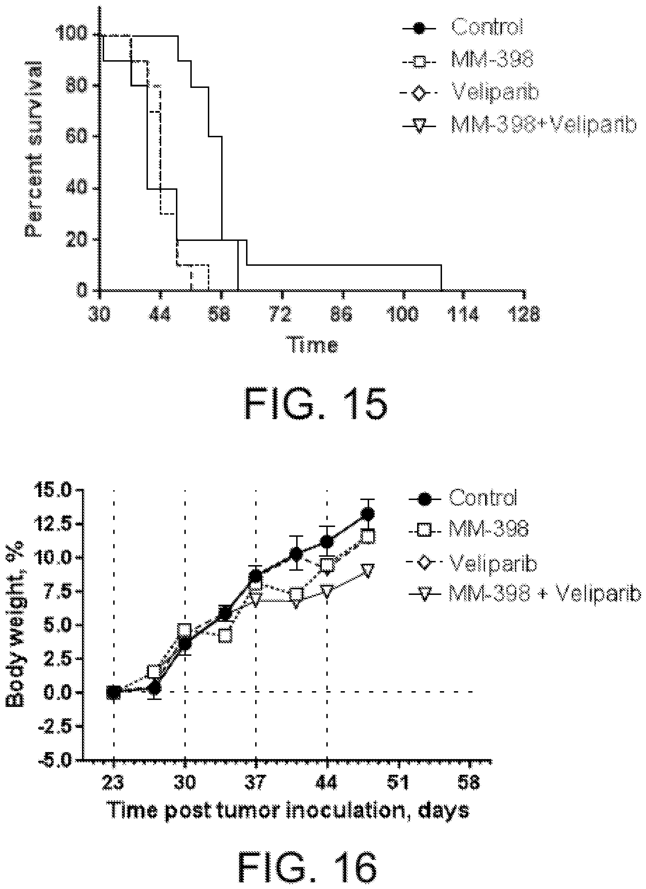

Figure 15 depicts the effect of MM-398 in combinations with veliparib in C33A xenograft model an animal's survival. In the mouse xenograft study using C33 cervical cancer cells the murine model is treated with liposomal irinotecan (5 mg/kg MM398) and/or the PARP inhibitor veliparib (50 mpk) on days 3-5 starting after administration of MM398.

Figure 16 depicts the effect of MM-398 in combination with veliparib in C33A xenograft model and body weight, where veliparib was dosed 72 h following MM-398 (nal-IRI) administration. (5 mg/kg MM398) and/or the PARP inhibitor veliparib (50 mpk) on days 3-5 starting after administration of MM398.

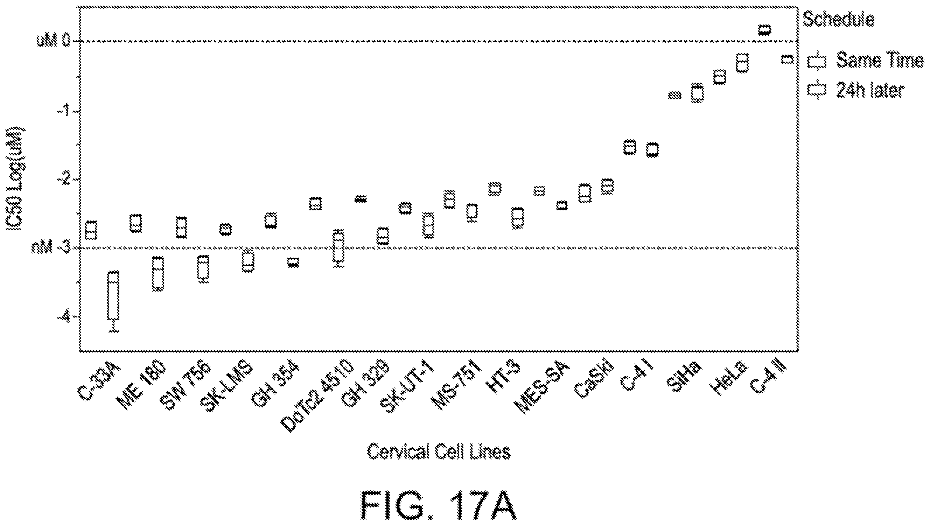

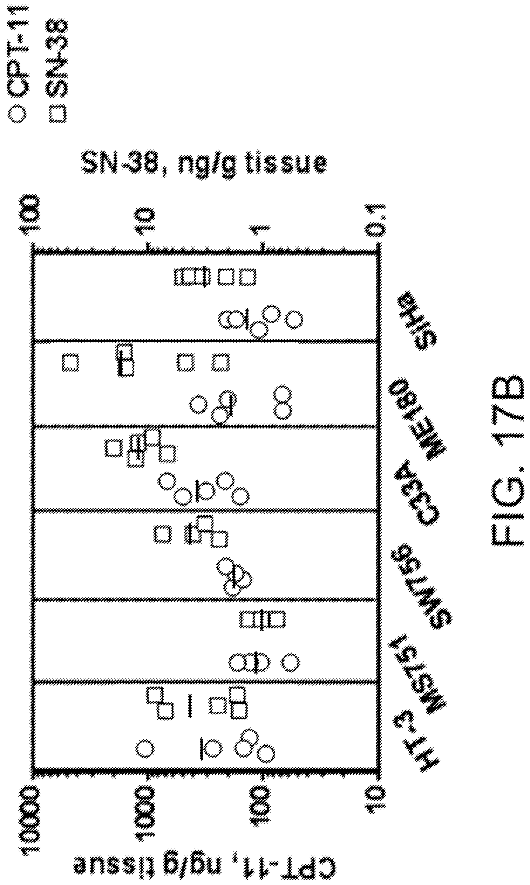

Figures 17A and 17B depict the *in vitro* activity (A) and tumor content (B) of SN-38 in cervical models. (A) Cervical cells lines were treated with veliparib and SN-38 at either the same time or with scheduling with veliparib being added 24 h after SN-38, and cell viability was measured using CTG assay. The *in vitro* activity (IC50) is measured for multiple cervical cancer cell lines. (B) Nude mice bearing cervical tumors were injected with a single dose of nal-IRI at 10 mg/kg and tumor content of CPT-11 and SN-38 were measured by LC-MS.

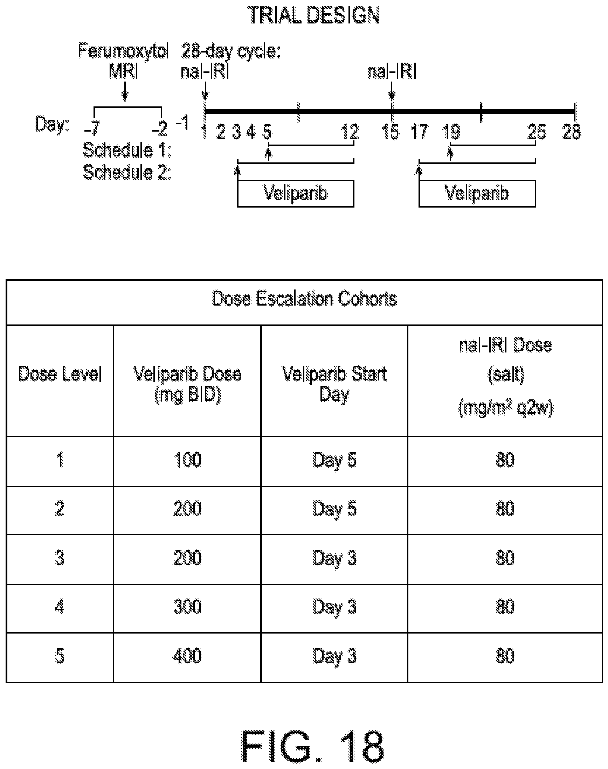

Figure 18 is a graphical representation of a phase I study design employing the combinations of MM-398 (nal-IRI) and veliparib. The primary endpoint readout is to identify MTD / RP2D, and the secondary endpoint readouts is AE profile, PK parameters, and biomarker analysis that includes pre-treatment MRI to measure nanoparticle tumor delivery and efficacy.

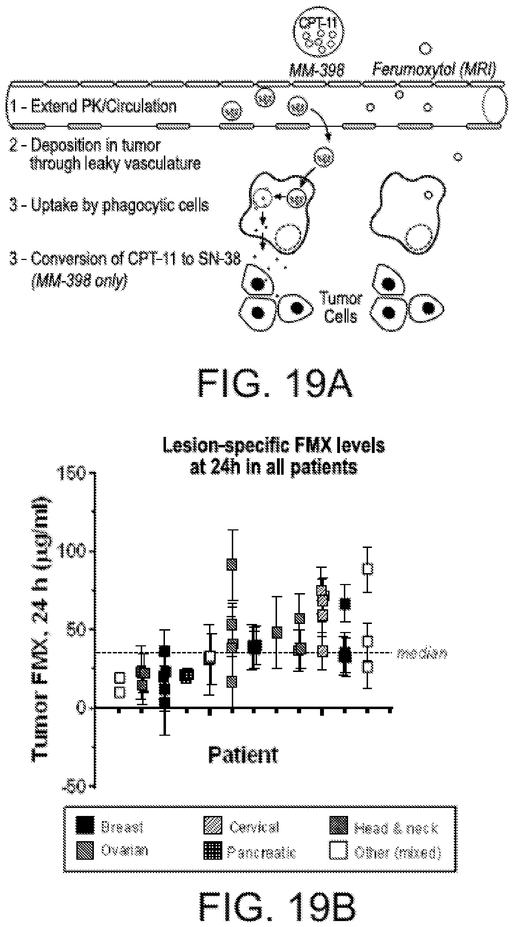

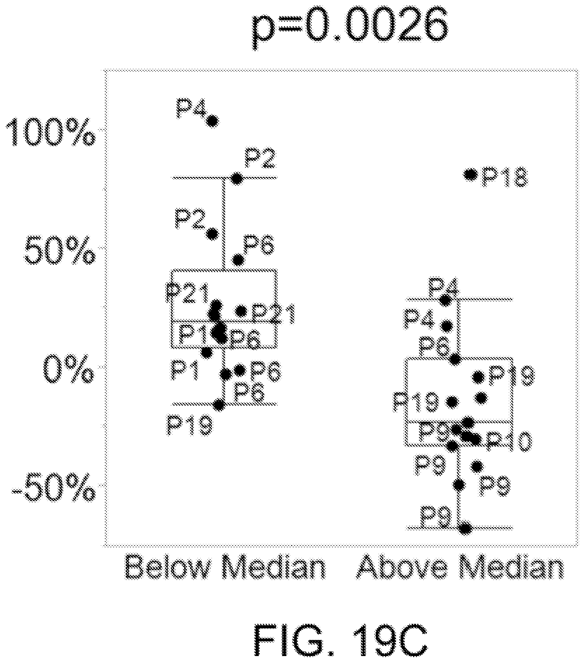

Figure 19 shows that FMX MRI may be a predictive tool for tumor response to MM-398. (A) MM-398 and FMX have similar properties, including 1) extended PK, 2) the ability to deposit in tumor tissues through the EPR effect (i.e. leaky vasculature), and 3) uptake by macrophages. Therefore, visualization of FMX on MRI may be able to predict MM-398 deposition. Figure 19A is a schematic showing a use of ferumoxytol (FMX) as a predictive biomarker for cancer treatment with liposomal irinotecan (e.g., MM-398) (B) FMX concentration of individual patient lesions was calculated using a standard curve from MR images obtained 24h post-FMX injection. (C) graph showing FMX signal from lesions at 24h are grouped relative to the median value observed in the FMX MRI evaluable lesions and compared to the best change in lesion size based on CT scans (data available from 9 patients; total of 31 lesions).

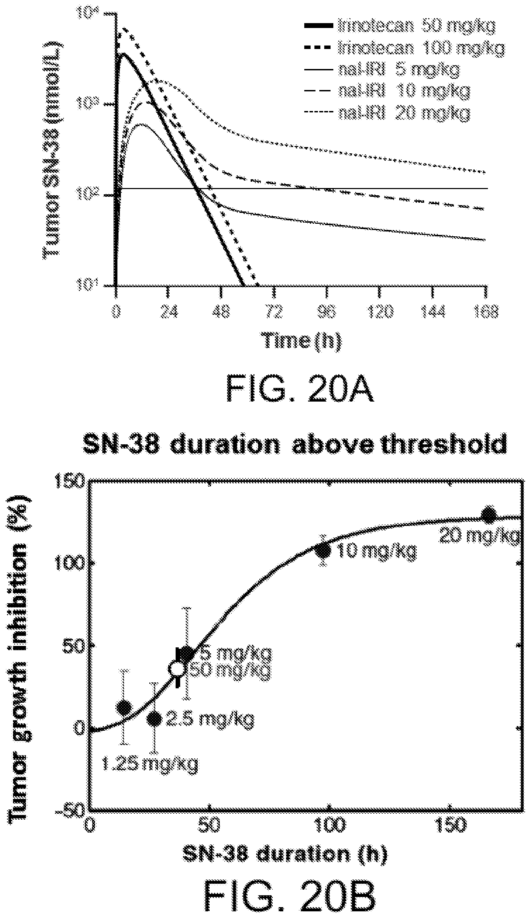

Figure 20A is a graph showing the tumor SN-38 (nmol/L) measured in tumors after administration of free (non-liposomal) irinotecan (CPT-11) at 50mg/kg or 100 mg/kg, compared to the administration of MM-398 (5 mg/kg, 10 mg/kg or 20 mg/kg).

Figure 20B is a graph showing levels of tumor growth inhibition as a function of time of SN-38 concentration required to yield tumor response.

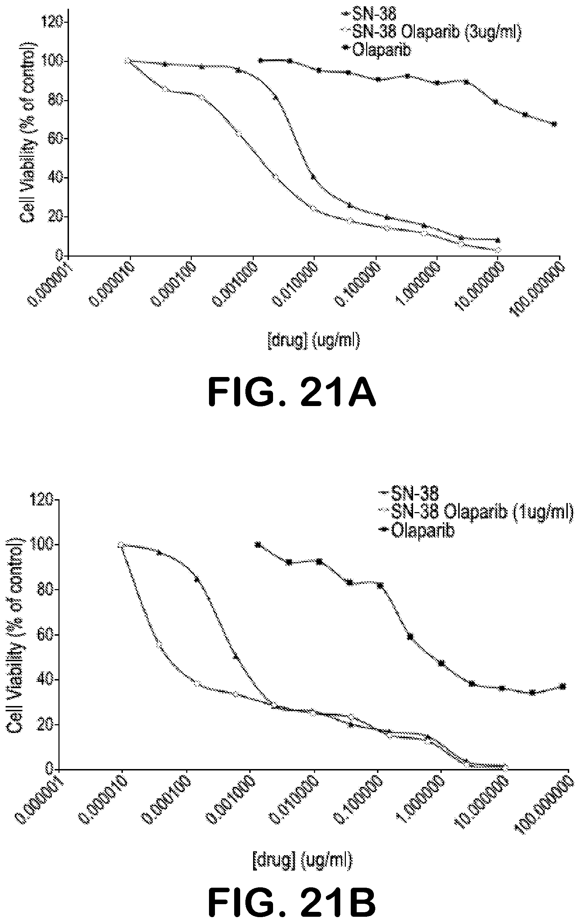

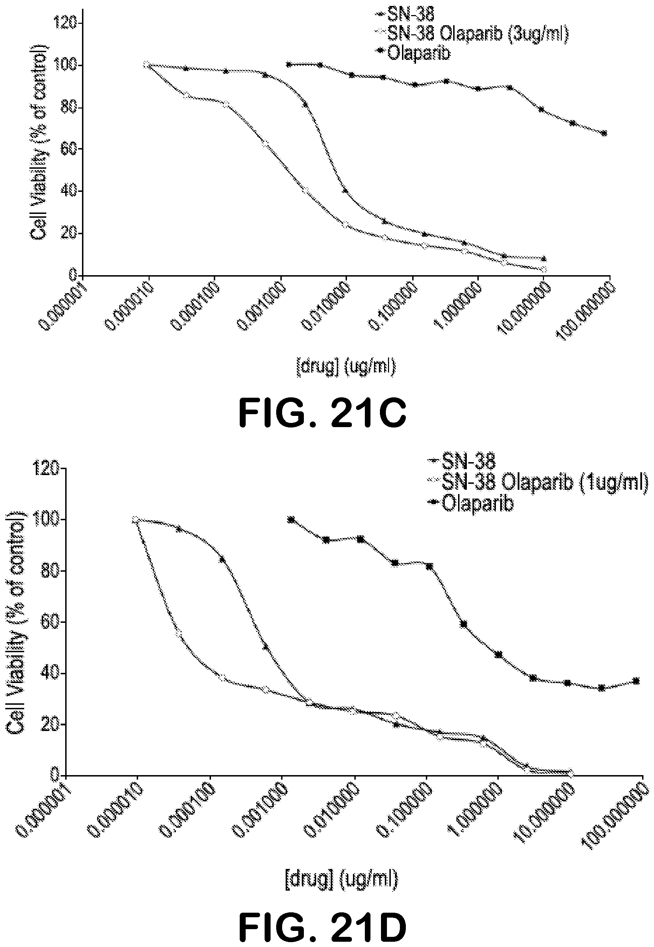

Figure 21 shows line graphs that depict cell viability following treatment with SN-38 or olaparib as single agents or in combination. C-33A (cervical carcinoma, ATCC®HTB-31™; A) or OVCAR-8 (ovarian carcinoma, from NCI-60 panel; B) SK-OV-3(ovarian carcinoma, ATCC® HTB-77™; C) or OVCAR-3 (ovarian adenocarcinoma, ATCC® HTB-161™, D) cells were plated at 1000cells/well in a 348-well plate and treated with SN-38 and olaparib, each alone or in combination, for 24h, washed, and then incubated for an additional 72h with fresh media, following which cell viability was assessed. Treatment of the cells with a combination of SN-38 and olaparib decreased the IC50 as compared to treatment with single agents in all cell lines tested.

## Detailed Description

The present disclosure provides a method of treating a patient with cancer and having a tumor, the method comprising a treatment regimen that may be repeated at weekly or longer intervals (e.g., Q2W, Q3W, or Q4W), each

instance of the treatment comprising:

i. intravenously administering to the patient an effective amount of an irinotecan liposomal formulation of a Top1 inhibitor such as irinotecan, topotecan, lurtotecan, indotecan, and indimitecan; and
ii. administering to the patient and effective amount of a PARP inhibitor wherein the PARP inhibitor is administered after an interval following completion of the administration of the Top1 inhibitor, e.g., an effective irinotecan plasma clearing interval.

In a further embodiment, the method comprises:

i. intravenously administering to the patient an effective amount of an irinotecan liposomal formulation having a terminal elimination half-life of about 26.8 hours and a maximal irinotecan plasma concentration of about 38.0 micrograms/ml; and
ii. administering to the patient and effective amount of a PARP inhibitor wherein the PARP inhibitor is administered after an interval of 24 hours or up to three days following completion of the administration of the irinotecan.

In some embodiments of the above two methods, the effective plasma clearing interval is from about 24 to about 240 hours such as, the effective plasma clearing interval is from about 48 to about 168 hours, for example, about 48 to about 90 hours. In some embodiments of the above two methods, above embodiment or as disclosed elsewhere herein, the effective plasma clearing interval is 2, 3, 4 or 5 days. In some embodiments of the above two methods, above embodiments or as disclosed elsewhere herein, the effective amount of MM-398 is from about 60 mg/m$^2$ to about 120 mg/m$^2$. In some embodiments of the above two methods, above embodiments or as disclosed elsewhere herein, the effective amount of MM-398 is about 80 mg/m$^2$. In some embodiments of the above two methods, above embodiments or as disclosed elsewhere herein, the PARP inhibitor is administered at a dose of from about 20 mg/day to about 800 mg/day. In some embodiments of the above two methods, above embodiments or as disclosed elsewhere herein, the PARP inhibitor is administered at a dose of from about 10 percent to 100 percent of its maximum tolerated dose. In some embodiments of the above two methods, above embodiments or as disclosed elsewhere herein, the PARP inhibitor is administered once or twice daily at a dose of from about 20 mg to about 400 mg. In some embodiments the PARP inhibitor is selected from the group consisting of talazoparib, niraparib, olaparib, veliparib, iniparib, rucaparib, CEP 9722, talazoparib and BGB-290 for example is veliparib. In some embodiments of the above two methods, above embodiments or as disclosed elsewhere herein, the cancer is cervical cancer, ovarian cancer, triple negative breast cancer, non-small cell lung cancer, small cell lung cancer, gastrointestinal stromal tumors gastric cancer, pancreatic cancer, colorectal cancer, or a neuroendocrine cancer.

In an embodiment, the invention also provides use of liposomal irinotecan in combination with a Poly(ADP-ribose) Polymerase (PARP) inhibitor in an antineoplastic therapy for the treatment of a solid tumor, wherein the liposomal irinotecan is repeatedly administered once every two weeks and the PARP inhibitor is administered daily for 3 to 10 days between consecutive administrations of the liposomal irinotecan, without administering the PARP inhibitor within 3 days of the liposomal irinotecan. The PARP inhibitor can be administered on each of consecutive days 3 to 10 between the days when the liposomal irinotecan is administered.

The invention also provides liposomal irinotecan for use in an antineoplastic therapy for the treatment of a solid tumor in a patient, wherein the liposomal irinotecan is administered in combination with a Poly(ADP-ribose) Polymerase (PARP) inhibitor, the treatment comprising a 28-day antineoplastic therapy treatment cycle consisting of: administering the liposomal irinotecan on days 1 and 15 of the treatment cycle, and administering the PARP inhibitor on one or more days starting at least 3 days after the liposomal irinotecan and ending at least 1 day prior to administration of additional liposomal irinotecan. In some embodiments, the PARP inhibitor is not administered for at least 3 days after the administration of liposomal irinotecan, such as wherein the PARP inhibitor is not administered for at least 3 days prior to the next administration of liposomal irinotecan.

In some embodiments of any of the uses or methods set out herein, the PARP inhibitor is administered on one or more of days 5-12 of the antineoplastic therapy treatment cycle. In some embodiments of any of the uses or methods set out above, the PARP inhibitor is administered on one or more of days 19-25 of the antineoplastic therapy treatment cycle. In some embodiments of any of the uses or methods set out above, the PARP inhibitor is administered on one or more of days 3-12 of the antineoplastic therapy treatment cycle. In some embodiments of any of the uses or methods set out above, the PARP inhibitor is administered on one or more of days 17-25 of the antineoplastic therapy treatment cycle.

As already noted, in some embodiments, such as of the methods or uses described within this section, the liposomal irinotecan has an irinotecan terminal elimination half-life of 26.8 hours and a maximal irinotecan plasma concentration of 38.0 micrograms/ml. In some embodiments of any of the uses or methods set out above, wherein the PARP inhibitor is not administered within 3 days before or after the administration of the liposomal irinotecan.

In some embodiments of any of the uses or methods set out herein, liposomal irinotecan is administered at a dose of 80 mg/m$^2$ (salt) or 70 mg/m$^2$ (free base). In some embodiments of any of the uses or methods set out herein, each administration of the PARP inhibitor is administered at a dose of from about 20 mg/day to about 800 mg/day. In some embodiments of any of the uses or methods set out herein, each administration of the PARP inhibitor is administered once or twice daily at a dose of from about 20 mg/day to about 400 mg/day.

In some embodiments of any of the uses or methods set out herein, liposomal irinotecan is administered at a dose of 80 mg/m$^2$ (salt) or 70 mg/m$^2$ (free base), and the PARP inhibitor is administered at a dose of from about 20 mg/day to about 800 mg/day.

In some embodiments of any of the uses or methods set out herein, liposomal irinotecan is administered at a dose of 80 mg/m$^2$ (salt) or 70 mg/m$^2$ (free base), and the PARP inhibitor is administered once or twice daily at a dose of from about 20 mg/day to about 400 mg/day.

In some embodiments of any of the uses or methods set out herein, wherein the PARP inhibitor is selected from the group consisting of niraparib, olaparib, veliparib, rucaparib and talazoparib. In some embodiments of any of the uses or methods set out herein, the cancer is cervical cancer, ovarian cancer, triple negative breast cancer, non-small cell lung cancer, small cell lung cancer, gastrointestinal stromal tumors gastric cancer, pancreatic cancer, colorectal cancer, or a neuroendocrine cancer.

In some embodiments of any of the uses or methods set out herein, the cancer is cervical cancer and the PARP inhibitor is veliparib. In some embodiments of any of the uses or methods set out herein, the cancer is cervical cancer and the PARP inhibitor is olaparib.

In some embodiments of any of the uses or methods set out herein, the cancer is cervical cancer and the PARP inhibitor is veliparib, liposomal irinotecan is administered at a dose of 80 mg/m$^2$ (salt) or 70 mg/m$^2$ (free base), and the PARP inhibitor is administered at a dose of from about 20 mg/day to about 800 mg/day. In some embodiments of any of the uses or methods set out herein, the cancer is cervical cancer and the PARP inhibitor is olaparib, liposomal irinotecan is administered at a dose of 80 mg/m$^2$ (salt) or 70 mg/m$^2$ (free base), and the PARP inhibitor is administered at a dose of from about 20 mg/day to about 800 mg/day. In some embodiments of any of the uses or methods set out herein, the cancer is cervical cancer and the PARP inhibitor is veliparib, liposomal irinotecan is administered at a dose of 80 mg/m$^2$ (salt) or 70 mg/m$^2$ (free base), and the PARP inhibitor is administered once or twice daily at a dose of from about 20 mg/day to about 400 mg/day. In some embodiments of any of the uses or methods set out herein, the cancer is cervical cancer and the PARP inhibitor is olaparib, liposomal irinotecan is administered at a dose of 80 mg/m$^2$ (salt) or 70 mg/m$^2$ (free base), and the PARP inhibitor is administered once or twice daily at a dose of from about 20 mg/day to about 400 mg/day.

In some embodiments of any of the uses or methods set out herein, the cancer is cervical cancer and the PARP inhibitor is veliparib, liposomal irinotecan is administered at a dose of 80 mg/m$^2$ (salt) or 70 mg/m$^2$ (free base), and the PARP inhibitor is administered at a dose of from about 20 mg/day to about 800 mg/day, wherein the liposomal irinotecan is repeatedly administered once every two weeks and the PARP inhibitor is administered daily for 3 to 10 days between consecutive administrations of the liposomal irinotecan, without administering the PARP inhibitor within 3 days of the liposomal irinotecan. In some embodiments of any of the uses or methods set out herein, the cancer is cervical cancer and the PARP inhibitor is olaparib, liposomal irinotecan is administered at a dose of 80 mg/m$^2$ (salt) or 70 mg/m$^2$ (free base), and the PARP inhibitor is administered at a dose of from about 20 mg/day to about 800 mg/day, wherein the liposomal irinotecan is repeatedly administered once every two weeks and the PARP inhibitor is administered daily for 3 to 10 days between consecutive administrations of the liposomal irinotecan, without administering the PARP inhibitor within 3 days of the liposomal irinotecan. In some embodiments of any of the uses or methods set out herein, the cancer is cervical cancer and the PARP inhibitor is veliparib, liposomal irinotecan is administered at a dose of 80 mg/m$^2$ (salt) or 70 mg/m$^2$ (free base), and the PARP inhibitor is administered once or twice daily at a dose of from about 20 mg/day to about 400 mg/day, wherein the liposomal irinotecan is repeatedly administered once every two weeks and the PARP inhibitor is administered daily for 3 to 10 days between consecutive administrations of the liposomal irinotecan, without administering the PARP inhibitor within 3 days of the liposomal irinotecan. In some embodiments of any of the uses or methods set out herein, the cancer is cervical cancer and the PARP inhibitor is olaparib, liposomal irinotecan is administered at a dose of 80 mg/m$^2$ (salt) or 70 mg/m$^2$ (free base), and the PARP inhibitor is administered once or twice daily at a dose of from about 20 mg/day to about 400 mg/day, wherein the liposomal irinotecan is repeatedly administered once every two weeks and the PARP inhibitor is administered daily for 3 to 10 days between consecutive administrations of the liposomal irinotecan, without administering the PARP inhibitor within 3 days of the liposomal irinotecan.

In one embodiment, the cancer is a breast cancer, e.g., metastatic breast cancer, comprising a mutation in one of the breast cancer associated genes, BRCA1 or BRCA2. In another embodiment, the cancer is ovarian cancer comprising a mutation in BRCA1 and BRCA2. Mutations in the tumor suppressor genes BRCA1 and BRCA2 are associated with increased risk of breast cancer (about 5X the risk of a person without a BRCA mutation) and/or increased risk of ovarian cancer (about 10-30 times the risk of a person without a BRCA mutation). Statistics for BRCA-related ovarian cancer typically encompass not only cancer of the ovaries themselves, but also peritoneal cancer and cancer of the

Fallopian tubes; women with a BRCA mutation have more than 100 times the normal rate of Fallopian tube cancer. BRCA mutations are also with increased risk of prostate cancer risk in men. Cancer cells lacking normal BRCA1 or BRCA2 (or other DNA-repair enzymes such as ATM) depend instead on PARP-regulated DNA repair, and thus are hypersensitive to PARP inhibition.

In some embodiments of any of the uses or methods set out herein, the use of method further comprising the use of ferumoxytol as an imaging agent to select patients to receive the liposomal irinotecan and PARP inhibitor, for example wherein the method further comprises administering ferumoxytol and then obtaining a MRI image of the patient 24 hours after ferumoxytol administration.

Irinotecan is a Top1 inhibitor. The chemical name of irinotecan is (*S*)-4,11-diethyl-3,4,12,14-tetrahydro-4-hydroxy-3,14-dioxo1*H*-pyrano[3',4':6,7]-indolizino[1,2-b]quinolin-9-yl-[1,4'bipiperidine]-1'-carboxylate. Irinotecan is also referred to by the name CPT-11 and by the trade name CAMPTOSAR. Irinotecan acts as a prodrug, and is converted by esterase enzymes into the more active metabolite, SN-38.

The present disclosure also provides for methods of administering a combination of a topoisomerase-1 (Top1) inhibitor (e.g., irinotecan and/or its metabolite SN-38) and a PARP inhibitor to a tumor with reduced peripheral toxicity. TheTop1 inhibitor can be administered in a liposome formulation resulting in the prolonged accumulation of the Top1 inhibitor in a solid tumor compared to peripheral plasma and/or healthy organs. Subsequently, a PARP inhibitor can be administered after a period of time permitting a reduction in the amount of the Top1 inhibitor outside the tumor relative to the amount of Top1 inhibitor within the tumor. The Top1 inhibitor used in the invention is liposomal irinotecan that provides SN-38 to a solid tumor.

Methods of treating a cancer are provided, as well as therapeutic uses of PARP inhibitor compounds in combination with liposomal irinotecan formulations for the treatment of cancer, particularly cancer comprising solid tumors.

The uses and methods disclosed herein are based in part on experiments evaluating the combination of a topoisomerase 1 inhibitor (e.g., liposomal irinotecan or SN-38) and a PARP inhibitor in both pre-clinical and human clinical studies. The topoisomerase 1 inhibitor was administered in certain *in vitro* animal models using a formulation delivering a more prolonged exposure of the topoisomerase 1 inhibitor (e.g., irinotecan and/or the irinotecan active metabolite designated SN-38) within solid tumors than in peripheral tissue and plasma outside the tumor. Combinations of the topopisomerase 1 inhibitor SN38 and/or irinotecan and PARP inhibitor compounds were tested in various *in vitro* experiments. As detailed in Example 2, the *in vitro* testing of multiple combinations of a topoisomerase 1 inhibitor (SN38) and various PARP inhibitors in more than 20 different cancer cell lines (including cervical, breast, ovarian, colorectal, pancreatic, and small cell lung cancer cell lines) all demonstrated decreased cancer cell line viability (Figures 1A, 1B, 1C, 1D, 1E, 2A, 2B, 2C, 2D, 2E, and 17A). The liposomal irinotecan (MM398) demonstrated greater tumor volume reduction than non-liposomal (free) irinotecan (CPT11) in mouse xenograft studies across multiple types of cancer cell lines (including breast, ovarian, colorectal and pancreatic cancer cell lines).

As detailed in Example 3, the tolerability of a topoisomerase 1 inhibitor (liposomal irinotecan) administered in combination with various PARP inhibitors was evaluated by measuring the change in animal (mouse) body weight in multiple murine models by comparing various dosing schedules. In some experiments, both liposomal irinotecan and a PARP inhibitor were administered together on the same day (day 1). In other experiments, the PARP inhibitor was first administered daily starting 2, 3 or 4 days after each administration of the liposomal irinotecan. The PARP inhibitor was administered for multiple consecutive days (e.g., 3 consecutive days), and not administered on the same day as the topoisomerase 1 inhibitor. As detailed in multiple experiments herein, administration of the PARP inhibitor at least one day after the liposomal irinotecan resulted in improved tolerability of comparable combined doses of the PARP inhibitor and liposomal irinotecan (MM-398) as measured by change in percent bodyweight in the animal (e.g., Figures 10A-10D, 8A, 8B, 8C, 12A, and 12B). Delaying the administration of the PARP inhibitor 2, 3 or 4 days after administration of the liposomal irinotecan led to greater overall tolerability of a combined administration of the liposomal irinotecan and the PARP inhibitor, compared to the administration of the liposomal irinotecan and the PARP inhibitor on the same day. For example, administration of veliparib on days 2, 3 and 4 after administration of liposomal irinotecan on day 1 resulted in successively increased tolerability (measured as higher percent mouse bodyweight) of the combination of these two drugs (observed at 15 mg/kg liposomal irinotecan dose on day 1 followed by veliparib dosing on days 2, 3 and 4; at 28 mg/kg liposomal irinotecan dosage on day 1 followed by veliparib dosing on days 3, 4, and 5 in Figure 12B, or followed by veliparib dosing on days 2, 3 and 4 in Figure 12B; and at 50 mg/kg liposomal irinotecan dose on day 1 followed by veliparib dosing on days 4, 5 and 6, or followed by veliparib dosing on days 2, 3 and 4 or followed by veliparib dosing on days 3, 4, and 5 in Figure 12A). Similarly, administering olaparib starting on days 2 or 3 after MM398 resulted in comparable or improved tolerability compared to administration of both agents on day 1. For example, administering a 200 mg/kg dose of olaparib to mice on days 2, 3, 4 and 5 after administration of 10 mg/kg MM398 liposomal irinotecan on day 1 resulted in a lower reduction in bodyweight than administering the same doses of both MM398 and olaparib on days 1, 2, 3 and 4.

Combinations of a topopisomerase 1 inhibitor (SN38 and/or irinotecan) and PARP inhibitor compounds were tested in various preclinical *in vivo* experiments to evaluate the effectiveness of the administration of various PARP

inhibitors starting 3 or 4 days after administration of the liposomal topoisomerase 1 inhibitor MM398. As detailed in Example 4, the administration of liposomal irinotecan (MM398) on day 1 followed by the PARP inhibitor veliparib on either days 3, 4 and 5 or days 4, 5, and 6, resulted in decreased tumor volume and extended percent survival in mouse xenograft models of cervical cancer using two different cell lines (MS751 and C33A) (Figures 11A, 11B, 13A, 13B, 14 and 15).

Based in part on these experiments, methods of treating human cancer include the administration of a PARP inhibitor one or more days (preferably 2, 3, 4, 5 or 6 days) after the administration of liposomal topoisomerase inhibitor such as liposomal irinotecan. Preferably, the PARP inhibitor and the liposomal irinotecan are not administered on the same day. Example 6 provides preferred embodiments for the use of liposomal irinotecan and one or more PARP inhibitors for the treatment of human cancer, such as cervical cancer, while other embodiments (e.g., Table 3) are also provided.

*Topoisomerase Inhibitors, Including Liposomal Irinotecan and Camptothecin Conjugates*

The topoisomerase inhibitor can be administered in any form that provides for the prolonged retention of a topoisomerase-1 inhibitor activity within a tumor compared to outside the tumor, after administration of the topoisomerase inhibitor. For example, the topoisomerase inhibitor can be a formulation that delivers SN-38 to a tumor cell *in vivo,* administered in an amount and manner providing a higher concentration of the SN-38 within the tumor than outside the tumor for a period of time after administration of the topoisomerase inhibitor. Suitable formulations of topoisomerase inhibitors include conjugate molecules of a topoisomerase inhibitor (e.g., camptothecin conjugated to a polymer or antibody), liposomes containing a topoisomerase inhibitor or other targeted release formulation technologies. The Top1 inhibitor is preferably formulated to provide prolonged accumulation in a tumor site, compared to accumulation in healthy (non-cancer) tissue outside the tumor site (e.g., in the plasma and/or healthy organs such as colon, duodenum, kidney, liver, lung and spleen). Various Top1 inhibitor liposomal formulations are described in U.S. Patent No. 8,147,867 and U.S. Patent Application Publication No. 2015/0005354.

In one embodiment, the topoisomerase inhibitor is SN-38, camptothecin or a compound that is converted to SN-38 within the body, such as irinotecan. Irinotecan and SN-38 are examples of Top1 inhibitors. Irinotecan is converted by esterase enzymes into the more active metabolite, SN-3

The topoisomerase inhibitor can be camptothecin conjugated to a biocompatible polymer such as a cyclodextrin or cyclodextrin analog (e.g., sulfonated cyclodextrins). For example, the topoisomerase inhibitor can be a cyclodextrin-containing polymer chemically bound to a camptothecin, irinotecan, SN-38 or other topoisomerase 1 inhibitor compound. A cyclodextrin-camptothecin conjugated topoisomerase 1 inhibitor can be administered at a pharmaceutically acceptable dose including 6, 12, or 18 mg/m$^2$ weekly administration, or 12, 15 or 18 mg/m$^2$ biweeekly administration. Examples of camptothecin-cyclodextrin conjugate topoisomerase 1 inhibitors (e.g., the cyclodextrin-containing polymer conjugate with camptothecin designated "CRLX101"), and related intermediates for preparing the same, are disclosed, for example, in Greenwald et al., Bioorg. Med. Chem., 1998, 6, 551-562, as well as United States Patent Application 2010/0247668, United States Patent Application 2011/0160159 and United States Patent Application 2011/0189092

The topoisomerase inhibitor can also be a liposomal formulation of a topoisomerase inhibitor such as irinotecan, camptothecin or topotecan. The topoisomerase inhibitor used in the invention is liposomal irinotecan. Liposomal irinotecan (e.g., MM-398, also called "nal-IRI") is a highly stabilized liposomal formulation of irinotecan that provides for sustained exposure of irinotecan, and the active metabolite SN-38 in the tumor to a higher proportion of cells during the more sensitive S-phase of the cell cycle. MM-398 is a liposomal irinotecan that has shown promising preclinical and clinical activity in a range of cancer types, and was recently approved in the United States in combination with 5-FU/LV for patients with metastatic adenocarcinoma of the pancreas after disease progression following gemcitabine-based therapy. Compared with free irinotecan, nal-IRI has an extended PK profile with prolonged local tumor exposure of MM-398 and SN-38. Since SN-38 is cleared more quickly from normal tissues than from tumor, it is hypothesized that delayed dosing of veliparib relative to MM-398 will allow for the expected window of maximum irinotecan-induced toxicity to pass in the absence of concurrent veliparib toxicity. However, the tumor levels of SN-38 are predicted to be sustained upon subsequent veliparib dosing, therefore maintaining the ability of both drugs to act on tumor tissue simultaneously and maintain synergy.

One suitable liposomal Top1 inhibitor formulation is liposomal irinotecan available under the brand name ONIVYDE® (irinotecan liposome injection) (Merrimack Pharmaceuticals, Inc, Cambridge, MA), previously designated "MM-398" prior to FDA approval, and liposomal irinotecan products that are bioequivalent to ONIVYDE.

As used herein, the term "MM-398" refers to a nanoliposomal irinotecan composition. The dose of MM-398 refers to the dose of irinotecan based on the molecular weight of irinotecan hydrochloride trihydrate unless clearly indicated otherwise.

As used herein, unless otherwise indicated, the dose of irinotecan in ONIVYDE/MM-398 refers to the dose of irinotecan based on the molecular weight of irinotecan hydrochloride trihydrate (i.e., "(salt)" dose), unless clearly indicated

otherwise. The dose may also be expressed as the irinotecan free base (i.e., "(base)" dose). Converting a dose based on irinotecan hydrochloride trihydrate to a dose based on irinotecan free base is accomplished by multiplying the dose based on irinotecan hydrochloride trihydrate with the ratio of the molecular weight of irinotecan free base (586.68 g/mol) and the molecular weight of irinotecan hydrochloride trihydrate (677.19 g/mol). This ratio is 0.87 which can be used as a conversion factor. For example, the 80 mg/m$^2$ dose based on irinotecan hydrochloride trihydrate is equivalent to a 69.60 mg/m$^2$ dose based on irinotecan free base (80 x 0.87). In the clinic this is rounded to 70 mg/m$^2$ to minimize any potential dosing errors. Similarly, a 120 mg/m$^2$ dose of irinotecan hydrochloride trihydrate is equivalent to 100 mg/ m$^2$ of irinotecan free base.

The MM-398 irinotecan liposome injection) includes an irinotecan sucrosofate salt, encapsulated in liposomes for intravenous use. The drug product liposome is a small unilamellar lipid bilayer vesicle, approximately 110 nm in diameter, which encapsulates an aqueous space which contains irinotecan in a gelated or precipitated state, as the sucrosofate salt. The liposome carriers are composed of 1,2-distearoyl-sn-glycero-3-phosphocholine (DSPC), 6.81 mg/mL; cholesterol, 2.22 mg/mL; and methoxy-terminated polyethylene glycol (MW 2000)-distearoylphosphatidylethanolamine (MPEG-2000-DSPE), 0.12 mg/mL. Each mL also contains 2-[4-(2-hydroxyethyl)piperazin-1-yl]ethanesulfonic acid (HEPES) as a buffer, 4.05 mg/mL; sodium chloride as isotonicity reagent, 8.42 mg/mL. The solution is buffered at pH 7.25.

ONIVYDE/MM-398 has been shown to improve the pharmacokinetic and safety profile of the free irinotecan, through high retention of the irinotecan molecules within the liposome, by extending the half-life of irinotecan in the plasma, and increased exposure of tumor cells to irinotecan compared with other organs. Table 1 below provides a summary of median (%IQR)* total irinotecan and SN-38 pharmacokinetic parameters observed in patients with solid tumors after administration of ONIVYDE/MM-398 at a dose of 80 mg/m$^2$ irinotecan (salt) dose administered once every 2 weeks.

Summary of Median (%IQR)* Total Irinotecan and SN-38 Pharmacokinetic Parameters in Patients with Solid Tumors.

| **Dose (mg/m$^2$)** | **Total Irinotecan** | | | | | SN-38 | | |
|---|---|---|---|---|---|---|---|---|
| | **$C_{max}$** [μg/ml] | **$t_{1/2}$** [h]† | **$AUC_{0-\infty}$** [h·μg/ml]† | **$V_d$** [L/m$^2$]† | **CL** [L h/m$^2$]† | **$C_{max}$** [ng/ml] | **$t_{1/2}$** [h]† | **$AUC_{0-\infty}$** [h·ng/ml]† |
| 80 (n=25) | 38.0 (36%) | 26.8 (110%) | 1030 (169%) | 2.2 (55%) | 0.077 (143%) | 4.7 (89%) | 49.3 (103%) | 587 (69%) |

* %IQR: % Interquartile Ratio $\text{Interquartile Ratio} = \frac{\text{Interquartile range}}{\text{Median}} \cdot 100\%$

† $t_{1/2}$, $AUC_{0-\infty}$ and $V_d$ were only calculated for a subset of patients with sufficient number of samples in the terminal phase: n=23 for total irinotecan; n=13 for SN-38.

$C_{max}$: Maximum plasma concentration

$t_{1/2}$: Terminal elimination half-life

$AUC_{0-\infty}$: Area under the plasma concentration curve extrapolated to time infinity

$V_d$: Volume of distribution

For MM-398, over the dose range of 60 to 180 mg/m$^2$, the maximum concentrations of both total irinotecan and SN-38 increase linearly with dose. The AUCs of total irinotecan increase linearly with dose; the AUCs of SN-38 increase less than proportionally with dose. The half-lives of both total irinotecan and SN-38 do not change with dose. In a pooled analysis from 353 patients, higher plasma SN-38 $C_{max}$ was associated with increased likelihood of experiencing neutropenia, and higher plasma total irinotecan $C_{max}$ was associated with increased likelihood of experiencing diarrhea. Direct measurement of liposomal irinotecan shows that 95% of irinotecan remains liposome-encapsulated during circulation. The volume of distribution of MM-398 80 mg/m$^2$ is 2.2 L/m$^2$. The volume of distribution of Irinotecan HCl is between 110 L/m$^2$ (dose=125mg/m$^2$) and 234 L/m$^2$ (dose=340 mg/m$^2$). The plasma protein binding of MM-398 is <0.44% of the total irinotecan in MM-398. The plasma protein binding of irinotecan HCl is 30% to 68% and approximately 95% of SN-38 is bound to human plasma proteins. The plasma clearance of total irinotecan from MM-398 80 mg/m$^2$ is 0.077 L/h/m$^2$ with a terminal half live of 26.8 h. Following administration of irinotecan HCl 125 mg/m$^2$, the plasma clearance of irinotecan is 13.3 L/h/m$^2$ with a terminal half live of 10.4 h. MM398 liposomal irinotecan can provide irinotecan and its active metabolite, SN-38, inside a patient, which are metabolically cleared by the human cytochrome P450 3A4 isoenzyme (CYP3A4) and uridine diphosphate-glucuronosyl transferase 1A1 (UGT1A1), respectively. The metabolic conversion of irinotecan to the active metabolite SN-38 is mediated by carboxylesterase enzymes. *In vitro* studies indicate

that irinotecan, SN-38 and another metabolite, aminopentane carboxylic acid (APC), do not inhibit cytochrome P-450 isozymes. SN-38 is subsequently conjugated predominantly by the enzyme UGT1A1 to form a glucuronide metabolite. UGT1A1 activity is reduced in individuals with genetic polymorphisms that lead to reduced enzyme activity such as the UGT1A1*28 polymorphism. Approximately 10% of the North American population is homozygous for the UGT1A1*28 allele (also referred to as UGT1A1 7/7 genotype). Based on the results of the population pharmacokinetic analysis, patients homozygous and non-homozygous for the UGT1A1*28 allele (UGT1A1 7/7 genotype) have similar SN-38 exposure. The urinary excretion of irinotecan HCl is 11% to 20%; SN-38, <1%; and SN-38 glucuronide, 3%. The cumulative biliary and urinary excretion of irinotecan HCl and its metabolites (SN-38 and SN-38 glucuronide), over a period of 48 hours following administration of irinotecan HCl in two patients, ranged from approximately 25% (100 mg/m$^2$) to 50% (300 mg/m$^2$). A mass balance study in Sprague-Dawley rats, using liposomal encapsulated $^{14}$C-irinotecan, showed that once irinotecan was released from the liposomes, it followed the same elimination pathway as unencapsulated irinotecan. Fecal excretion was the major route of excretion in male and female rats, accounting for 78.3% and 83.4%, respectively, of the total radioactivity dose administered of liposomal encapsulated $^{14}$C-irinotecan over 168 hours.

Various irinotecan liposomal formulations are described in U.S. Patent No. 8,147,867 and U.S. Patent Application Publication No. 2015/0005354. MM-398 is believed to include about 80,000 molecules of irinotecan in a gelated or precipitated state as a sucrosofate salt encapsulated in a liposome of about 100 nm in diameter. MM-398 has been shown to improve the pharmacokinetic and safety profile of the free irinotecan, through high retention of the irinotecan molecules within the liposome, by extending the half-life of irinotecan in the plasma, and increased exposure of tumor cells to irinotecan compared with other organs.

In the methods of this disclosure, the effective amount of liposomal irinotecan is provided as MM-398 at from about 60 mg/m$^2$ to about 120 mg/m$^2$. In a further embodiment, the effective amount of MM-398 is about 80 mg/m$^2$, optionally administered in combination with 400 mg/m$^2$ of leucovorin over 30 minutes, followed by intravenous administration of 2400 mg/m$^2$ of 5-fluorouracil as an infusion over 46 hours. In some embodiments, the dose is 70, 80, 90, 100, 110 or 120 mg/m$^2$ (based on the weight of irinotecan hydrochloride trihydrate salt) and doses of 50, 60, 70, 80, 95, and 100 mg/m$^2$ (based on the weight of irinotecan free base), each given once every two (2) weeks (e.g., on days 1 and 15 of a 28 day antineoplastic treatment cycle). In some embodiments, the effective amount of MM-398 is about 90 mg/m$^2$ (free base).

Liposomal irinotecan MM-398 extends the tumor exposure of the topoisomerase 1 inhibitor SN-38. MM-398 liposomal irinotecan was found to be more active than irinotecan in multiple murine xenograph models. The duration of tumor exposure to the topoisomerase 1 inhibitor SN-38 above a threshold minimum concentration (e.g., 120 nM) correlated with antitumor activity of the liposomal irinotecan. In addition, MM-398 liposomal irinotecan can provide prolonged SN-38 tumor durations that exceed those provided by non-liposomal irinotecan. For example, Figure 17B depicts tumor content of SN-38 in multiple murine cervical cancer models. Nude mice bearing cervical tumors were injected with a single dose of MM-398 at 10 mg/kg and tumor content of CPT-11 and SN-38 were measured by LC-MS. Figure 20A is a graph showing the tumor SN-38 (nmol/L) measured in tumors after administration of free (non-liposomal) irinotecan (CPT-11) at 50mg/kg or 100 mg/kg, compared to the administration of MM-398 (5 mg/kg, 10 mg/kg or 20 mg/kg). The graph depicts the prolonged accumulation of SN-38 (concentration) measured in a tumor after liposomal irinotecan (MM-398) administration compared to other organs, obtained using a using HT-29 colorectal cancer (CRC) tumor xenograft-bearing mice. Figure 20B is a graph showing levels of tumor growth inhibition as a function of time of SN-38 concentration required to yield tumor response. Levels of SN-38 of 120 nM was identified as the SN-38 tumor concentration required to yield tumor response. The in vitro IC50 for SN-38 effect on cell line can be used as an in vivo threshold (GI50 for HT-29 was observed to be about 60 nM). MM-398 liposomal irinotecan was observed to prolong the duration of SN-38 exposure at doses of 10 mg/kg and 20 mg/kg.

*PARP inhibitors*

In the methods of this disclosure, the PARP inhibitor is selected from the group consisting of talazoparib, niraparib, olaparib, veliparib, iniparib, rucaparib, CEP 9722 or BGB-290. In a further embodiment, the PARP inhibitor is veliparib.

PARPs are a family of enzymes involved in DNA repair that act via two mechanisms: catalytic inhibition and trapping of PARP-DNA complexes, and inhibition of this repair pathway can result in cell death following DNA damage. In preferred embodiments, combining PARP inhibitors with Top1 inhibitors results in increased efficacy in the clinic compared to either agent alone. While it has been demonstrated that synergism between PARP inhibitors and Top1 inhibitors is due to PARP catalytic inhibition, and does not involve PARP trapping, this promising preclinical activity has given rise to unacceptable toxicity in the clinic for these combinations.

The PARP inhibitor can be selected from compounds that inhibit Poly(ADP-ribose) polymerase (PARP), a family of enzymes involved in DNA repair. Preferably, the PARP inhibitor is a compound that acts via two mechanisms: catalytic inhibition and trapping of PARP-DNA complexes. The PARP inhibitor can be one or more clinically available PARP

inhibitor compounds (e.g. talazoparib, niraparib, olaparib, and veliparib, among others), including compounds that can act via both mechanisms, although to different degrees. For example, niraparib is much more potent at PARP trapping than veliparib, whereas they both exhibit similar PARP catalytic activity.

In a further embodiment, the PARP inhibitor is veliparib, olaparib, rucaparib or niraparib. In another embodiment, the PARP inhibitor is veliparib, or olaparib. The PARP inhibitor can be veliparib administered after liposomal irinotecan. The PARP inhibitor can be olaparib administered after liposomal irinotecan

Olaparib is indicated as monotherapy in patients with deleterious or suspected deleterious germline BRCA mutated (as detected by an FDA-approved test) advanced ovarian cancer who have been treated with three or more prior lines of chemotherapy. The recommended dose of olaparib for this indication is 400 mg (eight 50 mg capsules) taken twice daily, for a total daily dose of 800 mg. Patients taking olaparib are instructed to avoid concomitant use of strong and moderate CYP3A inhibitors and consider alternative agents with less CYP3A inhibition. If the inhibitor cannot be avoided, reduce the Lynparza dose to 150 mg (three 50 mg capsules) taken twice daily for a strong CYP3A inhibitor or 200 mg (four 50 mg capsules) taken twice daily for a moderate CYP3A inhibitor.

The PARP inhibitor can inhibit PARP 1 and/or PARP 2. For example, the PARP inhibitor can be a PARP ½ inhibitor with IC50 of SnM/1nM in cell-free assays and 300-times less effective against tankyrase-1 (e.g., olaparib). The PARP inhibitor can be an inhibitor of PARP 1 and PARP2 with Ki of 5.2 nM and 2.9 nM respectively in cell-free assays, and inactive to SIRT2 (e.g., veliparib). The PARP inhibitor can be an inhibitor of PARP1 with a Ki of 1.4 nM in a cell-free assay, and can also show binding affinity for other PARP domains (e.g., rucaparib). The PARP inhibitor can be effective against triple negative breast cancer (TNBC) alone or in combination with other agents. The PARP inhibitor can be a PARP1 inhibitor with an IC50 of 0.58 nM in a cell free assay that does not inhibit PARG and is sensitive to a PTEN mutation (e.g., talazoparib). The PARP inhibitor can be a potent and selective tankyrase inhibitor with an IC50 of 46 nM and 25 nM for TNKS 1/2, respectively (e.g., G007-LK). The PARP inhibitor can be a potent inhibitor of PARP 1 with a Ki of less than about 5nM in a cell free assay (e.g., AG-14361). The PARP inhibitor can be a selective inhibitor of PARP 2 with an IC50 of 0.3 micromolar, and can be about 27-fold selective against PARP 1 (e.g., UPF-1069). The PARP inhibitor can be a potent and selective inhibitor with an IC50 for PARP 3 of about 0.89 micromolar, and about 7-fold selectivity over PARP 1 (e.g., ME0328). The PARP inhibitor can be an inhibitor of PARP 1 and PARP2 with Ki values of 1 nM and 1.5 nM, respectively.

Preferred examples of PARP inhibitors are provided in the table 2A below, as well as pharmaceutically acceptable prodrugs, salts (e.g., tosylates) and esters thereof.

**Table 2A: Examples of PARP inhibitors**

| | |
|---|---|
| Olaparib (AZD-2281) | |
| Veliparib (ABT-888) | |
| Niraparib (MK04827) | |
| Rucaparib (AG 014699) | |

(continued)

| | |
|---|---|
| Talazoparib (BMN-673) | |
| Iniparib (BSI-201 | |

The dose of the PARP inhibitor and the frequency of dosing can be selected based on various characteristics of the PARP inhibitor, including the pharmacokinetic properties of the compound (e.g., half-life), prior dosing regimens and patient characteristics. Parameters that can be used in selecting the PARP inhibitor dose include those listed in Table 2B below.

In addition, patients can be selected to receive treatment combining a topoisomerase inhibitor and a PARP inhibitor. For example, patients can be selected based on their status in BRCA (e.g. BRCA1, BRCA2), Homologous Recombination Deficiency (HRD), BROCA-HR or other genetic risk panel analysis of a patient.

**Table 2B Characteristics of Some PARP inhibitors**

| Characteristic | Veliparib | Olaparib | Rucaparib | Niraparib | Talazoparib |
|---|---|---|---|---|---|
| Molecular Weight | 244.3 | 434.5 | 323.4 | 320.4 | 380.4 |
| PARP1 IC50 | 4.73-5.2 | 1.94-5 | 1.4-1.98 | 2.1-3.8 | 0.57-1.2 |
| PAR EC50 | 5.9 | 3.6 | 4.7 | | 2.5 |
| Monotherapy dosing | 200-400 mg BID | 300 mg BID | 240-600 mg BID | 300 mg QD | 1 mg QD |
| CDx | BRCA | BRCA, HRD | HRD | BRCA, HR | HRD, HR |

In the methods of this disclosure, the PARP inhibitor is administered at a therapeutically effective dose (e.g., a dose selected for the PARP inhibitor monotherapy, such as from about 200 mg/day to about 800 mg/day for veliparib). In a further embodiment, the PARP inhibitor is administered twice daily at a dose of from about 100 to about 400 mg. In some embodiments veliparib, rucaparib or olaparib is administered twice daily at a dose of from about 100 to about 400 mg. In some embodiments, 200 mg BID dose of veliparib is administered to patients after (e.g., 3-5 days after) each administration of liposomal irinotecan

In the methods of this disclosure, the PARP inhibitor is administered after an "effective irinotecan plasma clearing interval." "Effective irinotecan plasma clearing interval" is that interval between the administration of the liposomal irinotecan and the PARP inhibitor that allows sufficient clearance of irinotecan and SN-38 from the blood plasma and allows an effective quantity of irinotecan and/or SN38 to remain in one or more tumors within the patient for the subsequent administration of the PARP inhibitor to have a desired therapeutic effect. The effective plasma clearing interval in the methods of this disclosure is from about 24 to about 168 hours, including 48 hours to about 168 hours. In a further embodiment, the effective plasma clearing interval is from about 48 to about 96 hours. In a further embodiment, the effective plasma clearing interval is 24 hours or 2, 3, 4 or 5 days.

In the methods of this disclosure, the cancer is cervical cancer, ovarian cancer, triple negative breast cancer (TNBC), non-small cell lung cancer (NSCLC), small cell lung cancer (SCLC), gastric cancer, pancreatic cancer, colorectal cancer, or neuroendocrine tumor.

In one embodiment, a method of treating a patient according to the present invention results in a pathologic complete response (pCR), complete response (CR), partial response (PR) or stable disease (SD).

The methods of this disclosure can further comprise administering to the patient one or more additional agents

including, but not limited, to anti-emetics such as a 5-HT3 antagonist; agents for treating of diarrhea, such as loperamide; dexamethasone; or other chemotherapeutic agents.

In one embodiment, the methods of the present disclosure result in a pathologic complete response (pCR), complete response (CR), partial response (PR) or stable disease (SD). In another embodiment the combination therapy with MM-398 and a PARP inhibitor, e.g., veliparib, results in therapeutic synergy.

In certain embodiments, the MM-398 and the PARP inhibitor are administered in at least one cycle. A cycle comprises the administration of a first agent (e.g., a first prophylactic or therapeutic agents) for a period of time, followed by the administration of a second agent (e.g., a second prophylactic or therapeutic agents) for a period of time, optionally followed by the administration of a third agent (e.g., a third prophylactic or therapeutic agents) for a period of time and so forth, and repeating this sequential administration, i.e., the cycle. In one embodiment, the combination of MM-398 and a PARP inhibitor is administered for at least one cycle. In one embodiment the cycle is a 2 week cycle. In another embodiment, the cycle is a 3 week cycle. In another embodiment, the cycle is a 4 week cycle. In one embodiment MM-398 is administered at the beginning of the cycle and administration of a PARP inhibitor (e.g., veliparib) is delayed until at least about 12, 24, 48, 72, 96, or 120 hours, after the administration of MM-398.In one embodiment MM-398 is administered at the beginning of the cycle and administration of a PARP inhibitor (e.g., veliparib) is delayed until at least about 24, 48, 72, 96, or 120 hours, after the administration of MM-398. In one embodiment, MM-398 is administered as part of a 28 day cycle on days 1 and 15 and the PARP inhibitor is administered on days 3-12 and on days 17-25. In another embodiment, MM-398 is administered as part of a 28 day cycle on days 1 and 15 and the PARP inhibitor is administered on days 5-12 and days 19-25.

In some examples, including the protocols in Table 3, the PARP inhibitor is not administered within 3 days of the administration of liposomal topoisomerase 1 inhibitor such as MM-398 liposomal irinotecan (i.e., the PARP inhibitor is only administered on days that are both at least 2, 3, 4 or 5 days after the administration of the liposomal topoisomerase 1 inhibitor, and 2, 3, 4 or 5 days prior to the next administration of the liposomal topoisomerase 1 inhibitor). Table 3 shows dose timing protocols for administering a therapeutically effective amount of a PARP inhibitor and liposomal irinotecan on certain days of a 28-day antineoplastic treatment cycle.

**Table 3: Examples of 28-day Treatment Cycles**

| Protocol | PARP inhibitor given on days | Liposomal Irinotecan given on days |
|---|---|---|
| 1 | 3-12; 17-25 | 1, 15 |
| 2 | 4-12; 17-25 | 1, 15 |
| 3 | 5-12; 17-25 | 1, 15 |
| 4 | 6-12; 17-25 | 1, 15 |
| 5 | 3-12; 18-25 | 1, 15 |
| 6 | 4-12; 18-25 | 1, 15 |
| 7 | 5-12; 18-25 | 1, 15 |
| 8 | 6-12; 18-25 | 1, 15 |
| 9 | 3-12; 19-25 | 1, 15 |
| 10 | 4-12; 19-25 | 1, 15 |
| 11 | 5-12; 19-25 | 1, 15 |
| 12 | 6-12; 19-25 | 1, 15 |

In some examples, the PARP inhibitor is administered on one or more of days of a 28-day antineoplastic treatment cycle. For example, the PARP inhibitor can be administered on one or more of days 3, 4, 5, 6, 7, 8, 9, 10, 11 and 12 and 19, 20, 21, 22, 23, 24 and 25 of the 28-day antineoplastic treatment cycle when the liposomal irinotecan (e.g., MM-398) is administered once every two weeks, or on days 1 and 15 of the 28-day antineoplastic treatment cycle.

Methods of treatment and therapeutic uses of PARP inhibitors and topoisomerase inhibitors.

## Examples

The following non-limiting examples illustrate the methods of the present disclosure.

**Example 1:**

**Phase I Study of a Combination of MM-398 and Veliparib in Solid Tumors Background--PARP inhibitors and Top1 inhibitors**

Poly(ADP-ribose) polymerase (PARP) inhibitors are a new class of chemotherapeutic agents currently in development for the treatment of various cancer types. PARPs are a family of enzymes involved in DNA repair, and inhibition of this repair pathway results in cell death. PARP inhibitors, therefore, have been investigated in tumor types with other known DNA repair pathway deficiencies, such as breast and ovarian tumors with BRCA1 or BRCA2 mutations, which results in synthetic lethality. PARP inhibitors act via two mechanisms: catalytic inhibition and trapping of PARP-DNA complexes. Clinically available PARP inhibitors (e.g. talazoparib, niraparib, olaparib, and veliparib, among others) act via both mechanisms, although to different degrees. For example, niraparib is much more potent at PARP trapping than veliparib, whereas they both exhibit similar PARP catalytic activity. It is hypothesized that this translates into the decreased toxicity observed with veliparib compared with either niraparib or olaparib. Decreased toxicity does not preclude efficacy, however, as veliparib has demonstrated clinical activity in a Phase I study of 88 patients (60 BRCA+ and 28 BRCA-wt) receiving veliparib monotherapy, where the overall response rate (ORR; CR+PR) was 23% with a clinical benefit rate (CBR; CR + PR + stable disease) of 58% in BRCA+ patients, and an ORR of 40% with a CBR of 68% at the MTD and RP2D. The ORR in BRCA-wt patients evaluable for response was 4% with a CBR of 38% [5].

In BRCA-wt patients, where monotherapy treatment does not demonstrate synthetic lethality, the ability to improve tumor response may be achieved with combination therapy. As such, pre-treatment of tumor cells with other anticancer agents that damage DNA is thought to sensitize tumor cells to PARP inhibitors. Top1 inhibitors are a class of chemotherapeutic agents aimed at inhibiting DNA replication and are known to induce DNA strand breaks that involve PARP for their repair. It is hypothesized that combining PARP inhibitors with Top1 inhibitors will result in increased efficacy in the clinic compared to either agent alone. While PARP inhibitors are still being developed, Top1 inhibitors have already demonstrated successful clinical activity in various tumor types, as noted above. Recently, it has been demonstrated that synergism between PARP inhibitors and Top1 inhibitors is due to PARP catalytic inhibition, and does not involve PARP trapping. Therefore, when combining the two classes of drugs, using veliparib, a less potent PARP trapper, in combination with a Top1 inhibitor is predicted to allow for optimal synergy while minimizing dose-limiting toxicity, and was selected for this study (Figure 1). When combining the two classes of drugs using olaparib, a more potent PARP trapper as compared to veliparib, in combination with a Top1 inhibitor is predicted to allow for optimal synergy (Figure 21). Toxicity related to olaparib dosing was decreased by staggering the administration of olaparib and Top1 inhibitor. Additionally, Top1-induced DNA damage can also be repaired through alternate endonuclease repair pathways, such as the XPF-ERCC1 pathway. Therefore, tumors deficient in endonuclease repair pathways are also predicted to be more sensitive to cell death with irinotecan (CPT-11). Pre-clinically, the cytotoxicity of veliparib plus CPT-11 was further enhanced in XPF-deficient cells. In the clinic, irinotecan plus veliparib is expected to result in increased efficacy when targeting tumors with deficiencies in various DNA damage response pathways.

**Clinical experience of veliparib in combination with Top1 inhibitors**

PARP inhibitors and Top1 inhibitor combinations have been tested in phase I clinical trials. However, the development of these chemotherapy combination regimens has been limited by the increased toxicities that are observed, resulting in dose reductions that may limit efficacy. In particular, significant myelosuppression was seen in a dose-escalation study of veliparib and topotecan, where the maximum tolerated dose was exceeded at the first planned dose level. The result was decreased doses of topotecan, and no escalation of veliparib, with a final veliparib dosage of 10 mg BID, a 40-fold decrease compared to the established monotherapy dose of 400 mg BID. In a Phase I trial of veliparib in combination with irinotecan, dose-limiting toxicities (DLTs) included febrile neutropenia (grade 3), leukopenia and neutropenia (grade 4), and resulted in a 10-fold lower dose of veliparib compared with veliparib monotherapy. Another phase 1 dose-escalation study combined veliparib with bimonthly FOLFIRI in patients with advanced solid tumors. Importantly, three out of four DLTs on this study were neutropenia events, and the grade 3/4 neutropenia rate was 47%. Yet, in these Phase 1 trials, some efficacy in individual patients has been observed. For example, 5 PR were observed among the 32 treated patients in the veliparib plus irinotecan trial, while 12 PR and 1 CR were observed in a study enrolling 96 total patients receiving veliparib in combination with FOLFIRI. Table 1 below provides a summary of available clinical data for trials that combined veliparib with a Top1 inhibitor, illustrating the dose reductions of one or more drugs with combination treatment, as well as considerable toxicity. The challenge, therefore, is to determine how to safely combine these two classes of drugs, as the potential efficacy of the combination remains promising.

**Summary of clinical trials combining veliparib with a Top1 inhibitor.**

| Trial | Top1 inhibitor | MTD/ RP2D | DLTs | Most common G3/4 AEs | Most common AEs | Reference |
|---|---|---|---|---|---|---|
| Phase 1 dose-escalation study of veliparib (V) with bimonthly FOLFIRI in patients with advanced solid tumors | Irinotecan (as part of a FOLFIRI regimen with 5-FU) | V: 200 mg bid, Days 1-5 and 15-19 every 28 days Irinotecan: 150 (reduced) or 180 (standard) mg/m$^2$ bi-weekly | neutropenia (n=3; PI, 160 mg and 270 mg BID V; P2, 100 mg BID V); and gastritis and vomiting (PI, 270 mg BID V) | >30 patients each: neutropenia (47%), nausea (38%), and diarrhea (34%) | diarrhea (61%), nausea (60%), neutropenia (59%), vomiting (48%), fatigue (47%), anemia and alopecia (each, 41%) | J Clin Oncol 32,2014 (suppl 15S; abstr 2574) |
| Phase I study of the safety, pharmacokinetics (PK), and pharmacodynamics (PD) of the poly(ADP-ribose) polymerase (PARP) inhibitor veliparib (ABT-888; V) in combination with irinotecan (CPT-11; Ir) in patients (pts) with advanced solid tumors. | Irinotecan | V: 40 mg BID 15 days on/6 days off (21 day cycle) Irinotecan: 100 mg/m2 on Days 1 and 8 of a 21-day cycle | fatigue, diarrhea, febrile neutropenia (gr 3), leukopenia and, neutropenia (gr 4) | Not provided | diarrhea (59%), nausea (56%), leucopenia (50%), fatigue (47%), neutropenia (47%), anemia (34%), and vomiting (31%) | J Clin Oncol 29:2011 (suppl; abstr 3000) |
| Phase I study of PARP inhibitor ABT-888 (veliparib) in combination with topotecan in adults with refractory solid tumors and lymphomas | Topotecan | V: 10 mg BID on days 1-5 of 21-day cycles Topotecan: 0.6 (reduced) mg/m2/day on days 1-5 of a 21-day cycle | grade 4 neutropenia and thrombocytope nia (1 pt), grade 4 neutropenia lasting longer than 5 days (2 pts), febrile neutropenia (2 pts), grade 4 thrombocytope nia (2 pts) | Not provided | Not provided | CancerRes 2011;71: 562 6-5634. |

**MM-398 Mechanism of Action**

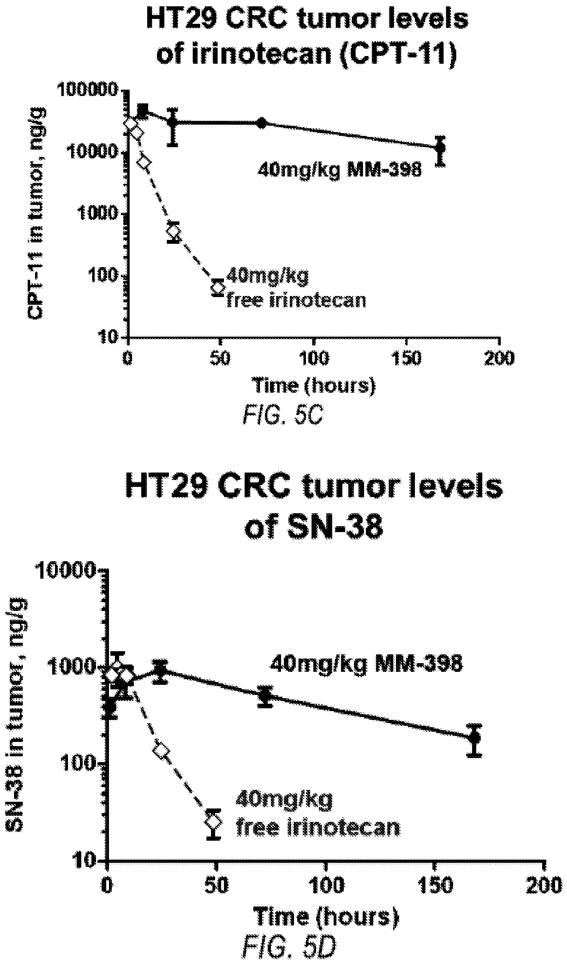

MM-398 is a nanoliposomal formulation of irinotecan (nal-IRI), consisting of approximately 80,000 molecules of irinotecan encapsulated in a liposome of -100 nm in diameter. This stable formulation is designed to improve the pharmacokinetic and safety profile of the free drug, by extending exposure and protecting the irinotecan molecules within the liposome. Liposomes are also known to preferentially deposit in tumor tissue through the enhanced permeability and retention (EPR) effect, which results from abnormal tumor vasculature permitting extravasation of macromolecules, as well as impaired lymphatic drainage that promotes the retention of these molecules within the tumor microenvironment. The EPR effect allows for prolonged tumor tissue exposure to MM-398 which in turn allows MM-398 exposure to a higher proportion of cells during the more sensitive S-phase of the cell cycle. In a murine biodistribution study, the active metabolite of irinotecan, SN-38, was measured in various tissues following MM-398 dosing and it was determined that SN-38 persisted in the tumor tissue longer than normal tissues, including kidney and liver (Figure 4). Additional pre-clinical pharmacokinetic (PK) studies show both extended plasma PK, as well as extended tumor PK, following dosing with MM-398 relative to dosing with free irinotecan (Figure 5). Both irinotecan and SN-38 are cleared very rapidly (within 8 hours) from the plasma following free irinotecan administration. However, MM-398 clearance is considerably slower with a half-life of approximately 48 hours as shown in Figure 5A; as >90% of irinotecan is encapsulated throughout in the plasma, irinotecan levels are reflective of MM-398 concentration. SN-38 plasma exposure is also greater, though $C_{max}$ levels are reduced, following MM-398 administration, suggesting the advantage of the liposomal formulation in prolonging exposure and half-life (Figure 5B). In tumor tissue, CPT-11 and SN-38 are cleared in approximately 2 days following dosing with free irinotecan, however both CPT-11 and SN-38 persist in the tumor tissue for at least 1 week following an equivalent dose of MM-398.

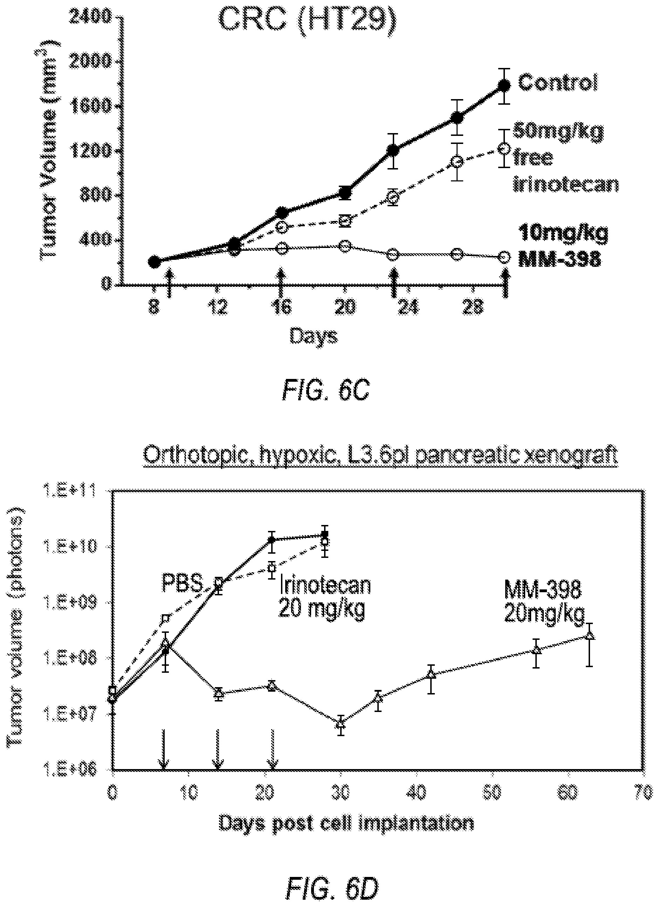

Tumor permeability as well as tumor tissue carboxylesterase (CES) activity, which is responsible for the enzymatic conversion of CPT-11 to SN-38, are predicted to be critical factors for local tumor exposure of SN-38 following MM-398 dosing. *In vivo* tumor xenograft studies have demonstrated that efficacy of MM-398 is related to high CES activity and/or high tumor levels of CPT-11 following dosing with MM-398. Additionally, MM-398 has demonstrated superior activity compared to equivalent dosing of free irinotecan in several pre-clinical models including breast, colon, ovarian, and pancreatic tumor xenograft models (Figure 6).

**Clinical Experience with MM-398 and Ferumoxytol MRI**

Clinically, MM-398 has also demonstrated prolonged exposure of SN-38. PK results from a Phase II study of gastric cancer patients demonstrated extended plasma PK of both CPT-11 and SN-38 upon treatment with MM-398 compared to treatment with free irinotecan (Figure 7A/B). Further, a Phase I study (protocol #MM-398-01-01-02) investigated tumor levels of both CPT-11 and SN-38 following treatment with MM-398 using post-treatment biopsies. Based on model predictions, SN-38 levels in tumor were expected to be higher than in plasma, suggesting local conversion of CPT-11 to SN-38 in the tumor microenvironment with MM-398 (Figure 7C). Predictions were confirmed by measuring levels of CPT-11 and SN-38 in tumor biopsy samples collected from patients 72 hours post-dose, demonstrating 5-fold higher levels of SN-38 in the tumor than the plasma (Figure 7D-E).

Collectively, the evidence suggests that the prolonged exposure to SN-38 will lead to prolonged DNA damage. SN-38 binds reversibly to the topoisomerase 1 cleavage complex ("Toplcc"). Therefore, the cleavage complex- "trapped" SN-38 is in equilibrium with free SN-38. The binding affinity is relatively low (but compensated by total selectivity) due to the $IC_{50}$ being in the high nanomolar range. In short, intracellularly, free SN-38 is a reliable reflection of Toplcc-bound SN-38. SN-38 metabolism relies on glucuronidation by UGT1A1 and excretion from the liver via ABCC2. UGT1A1 is found at much higher levels in normal liver than in other tissues and tumors (except for hepatocellular cancers). SN-38 in the tumor tissue will therefore not be metabolized to any significant extent. Figure 7 shows clinical data suggesting sustained circulating levels and more striking, sustained tumor levels. Thus, it has now been discovered that continuing DNA damage is occurring with MM-398 (as opposed rapidly cleared free irinotecan, which would cause initial DNA damage which was then rapidly repaired).

The phase I study of MM-398 also examined the feasibility of magnetic resonance (MR) imaging to predict tumor-associated macrophage (TAM) content and MM-398 deposition. TAMs appear to play a key role in the deposition, retention and activation of MM-398 within the tumor microenvironment. In this clinical study, ferumoxytol (FMX) a microparticulate preparation of a superparamagnetic iron oxide coated with polyglucose sorbitol carboxymethylether) was used as an imaging contrast agent and MR images were obtained at 1h, 24h, and 72h following FMX injection. FMX is an approved therapy that is indicated for the treatment of iron deficiency anemia in adult patients with chronic kidney disease; however a growing number of cancer patients without iron deficiency are being administered FMX as an imaging agent to visualize macrophage content and vasculature. Like MM-398, FMX is also a nanoparticle with a diameter of approximately 17-31 nm. As tumor permeability was predicted to be an important factor in MM-398 efficacy, FMX was also investigated for use as a surrogate for liposome deposition (Figure 19A). A benefit of FMX is that this agent helps

to identify patients that are less likely to respond to MM-398 because of poor drug uptake. Ferumoxytol as a diagnostic test enables the detection of a patient population that would significantly benefit from MM-398 that would otherwise be uncategorized.

With respect to risks associated with FMX infusion, as per the Feraheme® package insert, the following warnings and precautions are indicated for FMX: hypersensitivity reactions, hypotension, iron overload and ability to affect the diagnostic capability of MRI. Across three randomized clinical trials that enrolled 605 patients treated with FMX, the following adverse events were reported by ≥1% of patients treated with ferumoxytol: nausea, dizziness, hypotension, peripheral edema, headache, edema, vomiting, abdominal pain, chest pain, cough, pruritus, pyrexia, back pain, muscle spasms, dyspnea and rash. All IV iron products carry a risk of potentially life-threatening allergic reactions. In the initial clinical trials of Feraheme®, conducted predominantly in patients with chronic kidney disease, serious hypersensitivity reactions were reported in 0.2 percent (3/1,726) of patients receiving Feraheme®. Other adverse reactions potentially associated with hypersensitivity (e.g., pruritus, rash, urticaria or wheezing) were reported in 3.7 percent (63/1,726) of these patients. In other trials that did not include patients with chronic kidney disease, moderate to severe hypersensitivity reactions, including anaphylaxis, were reported in 2.6 percent (26/1,014) of patients treated with Feraheme®. Since the approval of Feraheme® on June 30, 2009, cases of serious hypersensitivity, including death, have occurred. In study MM-398-01-01-02, a total of 15 patients have received ferumoxytol to-date and 13/15 patients continued on study to receive MM-398. No hypersensitivity reactions or adverse events related to ferumoxytol were reported for these 15 patients. Patients with advanced incurable cancers being treated with investigational agents such as MM-398 have end stage cancer with very limited treatment options and a very high risk of dying from their underlying disease. The incremental risk of treating patients with FMX followed by MM-398 appears to be small relative to overall risks associated with MM-398 treatment of metastatic cancer patients. Precautions are taken to ensure FMX is administered according to the label instructions, including careful monitoring of patients during and for 30 minutes following ferumoxytol infusion, when administered as part of an MM-398 clinical trial. Additional precautions are taken not to administer FMX to patients with any of the following conditions: evidence of iron overload, a known hypersensitivity to ferumoxytol or any other IV iron product, a documented history of multiple drug allergies, or those for whom MRI is otherwise contraindicated.

The MRI results from study MM-398-01-01-02 demonstrated that the amount of FMX depositing in tumor lesions was able to be quantified (Figure 19B), and it was subsequently shown that a correlation existed between tumor lesion ferumoxytol uptake by MRI and response to MM-398 (Figure 19C). This correlation is now being studied further in an expansion of the Phase 1 study, and is included as a correlative imaging study for a trial of MM-398 + veliparib.

Ultimately, the development of FMX as a companion diagnostic agent has the potential to both spare non-responder patients unnecessary exposure to treatment-related toxicity while also serving as an enrichment tool to increase the proportion of treated patients that may respond.

**Treatment Plan**

MM-398 will be administered by intravenous (IV) infusion over 90 minutes at a dose of 80 mg/m$^2$ every two weeks. The starting dose of MM-398 at 80 mg/m$^2$ was chosen as a dose that was successfully used in the NAPOLI trial in pancreatic cancer in combination with 5-FU and leucovorin, therefore the MM-398 dose will be held constant and will not be escalated. Veliparib will be administered orally twice daily by the patient at home; a diary will be kept to document the dosage and time of day the drug was dosed. The number of dosing days of veliparib will be explored through the dose escalation scheme in the table below. The starting dose of veliparib at 100 mg bid is one-half the dose administered in combination with irinotecan as part of FOLFIRI and recently reported at the ASCO annual meeting (J Clin Oncol 32, 2014 (suppl 15S; abstr 2574)). Safety at the starting dose level is ensured by initiation of veliparib beginning day 5 after MM-398 dosing. Data obtained to date suggest SN-38 will be cleared from plasma by this point, but still accumulated in tumor tissue (see Figure 7C). Dose level 2, 200 mg BID of veliparib, was the MTD of veliparib used in the FOLFIRI regimen; therefore if Dose Level 2 is deemed safe following the evaluation period, increasing the number of dosing days of veliparib is the planned dose escalation step for the next dose level (Dose Level 3). If the dose and schedule of veliparib at Dose Level 3 is deemed safe, then escalation of the veliparib dose will proceed. If Dose Level 3 is not deemed safe, an alternate dosing schedule may be explored, where the number of dose days of veliparib is de-escalated.

MM-398 is administered by intravenous (IV) infusion over 90 minutes at a dose of 80 mg/m$^2$ every two weeks. Veliparib is co-administered orally twice daily by the patient at home according to the following schedule:

| Dose Level[1] | Veliparib Dose (mg BID) | Veliparib Dose Days | MM-398 Dose (mg/m$^2$ q2w) |
|---|---|---|---|
| 1 | 100 | Day 2, 3, 4, or 5-12; 16, 17, 18, or 19-25 | 80, Day 1, 15 |
| 2 | 200 | Day 2, 3, 4, or 5-12; 16, 17, 18, or 19-25 | 80, Day 1, 15 |
| 3 | 200 | Day 2, 3, 4, or 5-12; 16 or 17-25 | 80, Day 1, 15 |

(continued)

| Dose Level[1] | Veliparib Dose (mg BID) | Veliparib Dose Days | MM-398 Dose (mg/m$^2$ q2w) |
|---|---|---|---|
| 4 | 300 | Day 2, 3, 4, or 5-12; 16, 17, 18, or 19-25 | 80, Day 1, 15 |
| 5 | 400 | Day 2, 3, 4, or 5-12; 16, 17, 18, or 19-25 | 80, Day 1, 15 |
| [1] Additional dose levels and alternate dosing schedules may be explored upon agreement of Sponsor, Medical Monitor and Investigators. ** After the MTD is reached, and for the first cycle only, we plan to enroll approximately 18 patients obtain tumor biopsies according to the schema outlined in the correlates section below. | | | |

**Example 2: In Vitro Studies**

*In vitro* studies were performed testing combinations of various PARP inhibitors and topoisomerase inhibitors liposomal irinotecan and SN-38.

Figures 1A-1D show line graphs that depict cervical cancer cell viability following treatment with SN-38 and/or various PARP inhibitors. Unless otherwise indicated, the data in each of these figures was obtained by measuring cell viability of 5 different cervical cancer cells (ME-180 in Figure 1A, MS-751 in Figure 1B, C-33A in Figure 1C, SW756 in Figure 1D and SiHa in Figure 1E) with 1000 cells/well in a 384 well plate treated with SN-38 (topoisomerase 1 inhibitor) and/or one of 3 different PARP inhibitors (veliparib, niraparib, or olaparib) at 0.33 micrograms/mL) for 24 hours, followed by washing and incubation for an additional 72 hours with fresh media.

The combination of the topoisomerase 1 inhibitor SN-38 and various PARP inhibitors (veliparib, olaparib and rucaparib) were tested in vitro with various small cell lung cancer (SCLC), pancreatic cancer and breast cancer cell lines. At 2nM SN-38 concentration, an additive/synergistic growth inhibition of the cancer cells was observed in combination with olaparib, veliparib and rucaparib (with veliparib observed to be slightly less potent in the combination with SN-38 than olaparib and rucaparib). At all concentrations tested, the static growth of the cancer cell population was achieved. Figures 2A-2E are graphs showing the results of in vitro experiments evaluating combinations of the topoisomerase 1 inhibitor SN38 with various PARP inhibitors, formatted according to the tables 4-5 below (plates of 5,000 cells/well, 100 microliters per well; drugs added with 20x at 10 microliters per drug, top up to 100 microliters total with DMEM; then initiate scan every 4 hours up to 68 hours).

**Table 4**

| | Small Cell Lung Cancer | | Pancreatic Cancer | | TNBC |
|---|---|---|---|---|---|
| Treatment | DMS-114 | NCI-H1048 | CFPAC-1 | BxPC-3 | MDA-MB-231 |
| SN-38 & Olaparib | Plate 1 | Plate 2 | Plate 3 | Plate 4 | Plate 5 |
| SN-38 & Rucaparib | Plate 1 | Plate 2 | Plate 3 | Plate 4 | Plate 5 |
| SN-38 & Veliparib | Plate 1 | Plate 2 | Plate 3 | Plate 4 | Plate 5 |

**Table 5**

| Target Concentrations | | | | |
|---|---|---|---|---|
| Drug | Active Range based on XTC008 | Estimated tumor range (nM) | Dose Level | Conc' (nM) |
| SN-38 | 1-50 nM | 3-163 nM (398); IRI < 200nM | S1 | 2 |
| | | | S2 | 5 |
| | | | S3 | 10 |
| | | | S4 | 20 |
| | | | S5 | 50 |
| Olaparib | 1000-10000 nM | 8000nM | O1 | 2000 |
| | | | O2 | 4000 |
| | | | O3 | 8000 |
| Veliparib | 1000-10000 nM | > 2000 nM | V1 | 2000 |
| | | | V2 | 4000 |
| | | | V3 | 8000 |
| Rucaparib | 1-100 nM (Panc) | < 6000 nM | R1 | 2000 |
| | | | R2 | 4000 |
| | | | R3 | 8000 |

Additive/synergistic effects were observed between SN-38 at 2nM combined with the tested PARP inhibitors olaparib, veliparib and rucaparib with DMS-114 SCLC cells. Figure 2A is a graph showing the results of *in vitro* measurement of % cell number over time for DMS-114 small cell lung cancer cells treated with the topoisomerase inhibitor SN-38 and the PARP inhibitor rucaparib.

The NCI-H1048 SCLC cells were slow-growing and very sensitive to combinations of olaparib and rucaparib with SN-38 at 2nM. Figure 2B is a graph showing the results of *in vitro* measurement of % cell number over time for NCI-H1048 small cell lung cancer cells treated with the topoisomerase inhibitor SN-38 and the PARP inhibitor rucaparib.

Additive/synergistic effects were observed between SN-38 at 2nM combined with the tested PARP inhibitors olaparib, veliparib and rucaparib with CFPAC-1 pancreatic cancer cells. Figure 2C is a graph showing the results of *in vitro* measurement of % cell number over time for CFPAC-1 pancreatic cancer cells treated with the topoisomerase inhibitor SN-38 and the PARP inhibitor rucaparib.

Figure 2D is a graph showing the results of *in vitro* measurement of % cell number over time for BxPC-3 pancreatic cancer cells treated with the topoisomerase inhibitor SN-38 and the PARP inhibitor rucaparib. Figure 2E is a graph showing the results of *in vitro* measurement of % cell number over time for MDA-MB-231 triple negative breast cancer (TNBC) cancer cells treated with the topoisomerase inhibitor SN-38 and the PARP inhibitor rucaparib.

Figure 17A depicts the *in vitro* activity of SN-38 in cervical models. Cervical cells lines were treated with veliparib and SN-38 at either the same time or with scheduling with Veliparib being added 24 h after SN-38, and cell viability was measured using CTG assay.

**Example 3: Pre-Clinical Dose Tolerability Studies**

Various pre-clinical *in vivo* experiments were conducted to evaluate delayed dosing of veliparib relative to liposomal irinotecan can alleviate systemic toxicity, including a pre-clinical dose tolerability study. The combination of veliparib and irinotecan has been plagued by dose-limiting toxicities that have prevented this combination from being dosed at high (effective) doses of each drug, thereby limiting its clinical utility. To address this problem, pre-clinical studies evaluated administering a liposomal preparation of a topoisomerase 1 inhibitor, followed by the administration of a PARP inhibitor at least 1 day (preferably 2-3 days) after the day on which the liposomal topoisomerase 1 inhibitor was administered.

The advantage of dosing with MM-398 compared to free irinotecan is the extended PK profile and prolonged local tumor exposure of MM-398. Since SN-38 is cleared more quickly from normal tissues than from tumor, delayed dosing of veliparib (e.g. starting veliparib dosing a few days after MM-398 administration) allows for the window of maximum irinotecan-induced toxicity to pass in the absence of concurrent veliparib toxicity. However, the tumor levels of SN-38 are sustained longer than in healthy tissue, such that upon PARP inhibitor dosing subsequent to liposomal Top1 inhibitor (e.g., MM-398) administration, both drugs will act on tumor tissue simultaneously.

To demonstrate that delayed dosing of veliparib relative to nal-IRI can alleviate systemic toxicity, a pre-clinical

dose tolerability study was performed. Mice were dosed chronically with nal-IRI once weekly at various doses on Day 1, while veliparib was dosed once daily at a fixed dose for 3 consecutive days each week (either on Days 2-4, Days 3-5, or Days 4-6), and body weight was followed as a gross measure of toxicity. All mice were dosed chronically once weekly on day 1, with veliparib subsequently dosed for 3 consecutive days either on days 2-4 (8A), days 3-5 (8B), or days 4-6 (8C). Mice were weighed daily and % bodyweight gain is indicated on the Y-axis. Weight loss is indicative of intolerability of the combination. Notably, the highest (50 mg/kg) dose of MM-398 liposomal irinotecan was best tolerated (i.e., lowest measured reduction in % bodyweight observed over the experiment) when the veliparib was administered on days 4, 5 and 6 (Figure 8C). Similarly, the combination of veliparib and MM-398 was best tolerated at lower MM-398 liposomal irinotecan doses when the veliparib was only administered on days 4, 5, and 6 after MM-398 administration. Toxicity of the combination was seen at the highest doses of MM-398 when given in close proximity to the veliparib doses (Figure 8A). However, this toxicity could be alleviated either by dose reducing MM-398 or delaying the start of veliparib dosing, whereby the highest dose of MM-398 could be successfully dosed with veliparib if given on Days 4-6 following Day 1 dosing of MM-398 (Figures 8A-8C). The Day 4-6 veliparib dosing schedule (following day 1 dosing of MM398) was followed in subsequent efficacy studies which demonstrated synergy of the combination in two cervical cancer tumor xenograft models, in which veliparib alone was not efficacious (Figure 11A) and a second model in which neither MM-398 or veliparib were efficacious as single agents (Figure 11B), however the combination demonstrated tumor growth inhibition (Figure 11B).

To exemplify an embodiment demonstrating that delayed dosing of olaparib relative to MM-398 can alleviate systemic toxicity, a pre-clinical dose tolerability study was performed. Figure 9 depicts a graphical representation of a murine tolerability study design comparing MM-398 and olaparib as a monotherapy or in combination using a fixed dose of MM-398 and varying doses of olaparib, with various dosing schedules for different groups: Group1: MM-398 alone IV (10mg/kg); Group 2: olaparib alone oral (200mg/kg); Group 3: MM-398 (d1) + olaparib (200mg/kg, d1-5); Group 4: MM-398 (d1) + olaparib (150mg/kg, d1-5; Group 5: MM-398 (d1) + olaparib (200mg/kg, d1-4); Group 6: MM-398 (d1) + olaparib (200mg/kg, d2-5); Group 7: MM-398 (d1) + olaparib (200mg/kg, d3-5); group 8: DMSO alone oral. Figures 10A-10D are line graphs demonstrating the toxicities associated with MM-398 and olaparib given as monotherapy or combined therapy at varying doses of olaparib and varying schedule of PARP inhibitor administration following MM-398 in a murine model. Mice that received monotherapy of MM-398, olaparib were dosed 5x weekly. Mice that received a combination of a constant concentration of MM-398 (10mg/kg) and varying concentration of olaparib were dosed in varying schedules: Group 3: MM-398 (d1) + olaparib (200mg/kg, d1-5); Group 4: MM-398 (d1) + olaparib (150mg/kg, d1-5; Group 5: MM-398 (d1) + olaparib (200mg/kg, d1-4); Group 6: MM-398 (d1) + olaparib (200mg/kg, d2-5); Group 7: MM-398 (d1) + olaparib (200mg/kg, d3-5). Mice were monitored for treatment dependent toxicities by charting (A) body weight and (B) percent survival. Addition of olaparib seemed to be more toxic as compared to monotherapy, however delaying start of olaparib administration to d3 seemed to decrease olaparib specific toxicity as compared to concurrent therapy. Mice were dosed chronically with MM-398 once weekly at various doses on Day 1, while olaparib was dosed once daily at a weekly fixed dose for 5, 4 or 3 consecutive days each week (either on Days 1-5, Days 1-4, Days 2-5 or Days 3-5), and body weight and percent survival were followed as a gross measure of toxicity. Toxicity of the combination was seen at the highest doses of MM-398 when given in close proximity to the olaparib doses (Figure 8). However, this toxicity could be alleviated either by delaying the start of olaparib dosing, whereby the highest dose of MM-398 could be successfully dosed with olaparib if given on Days 3-5 following Day 1 dosing of MM-398 (Figures 10A-10D).

Mice were dosed chronically with MM-398 once weekly at various doses on Day 1, while veliparib was dosed once daily at a fixed dose for 3 consecutive days each week (either on Days 2-4, Days 3-5, or Days 4-6) and body weight was followed as a gross measure of toxicity. Toxicity of the combination was seen at the highest doses of nal-IRI when given in close proximity to the veliparib doses. However, this toxicity could be alleviated either by dose reducing nal-IRI or delaying the start of veliparib dosing. This dosing schedule was followed in subsequent mouse efficacy studies which demonstrated synergy of the combination in two cervical cancer tumor xenograft models, in which veliparib alone was not efficacious, and a second model in which neither nal-IRI or veliparib were efficacious as single agents, however the combination demonstrated tumor growth inhibition.

The tolerability of the combination of MM398 in a mouse model on day 1 was evaluated in combination with the administration of veliparib on days 1-3, days 2-4 and days 3-5. The tolerability of the combined regimen in mice (measured by change in percent bodyweight over 20 days) increased as the first administration of the veliparib occurred on day 2 and day 3, with day 3 initial veliparib dosing providing the most tolerated dosing schedule. Figure 12A is a graph that further depicts the in vivo tolerability of the 50 milligrams/kilogram (mpk) dose of MM-398 on day 1 in combination with 50 mg/kg veliparib given on days 1, 2 3 or 2, 3, 4 or 3, 4, 5 after administration of the MM-398, as reflected in percent change in body weight with an adjusted lower limit. Figure 12B is a graph that further depicts the *in vivo* tolerability of the 28 mpk dose of MM-398 on day 1 in combination with 50 mg/kg veliparib given on days 1, 2 3 or 2, 3, 4 or 3, 4,5 after administration of the MM-398, as reflected in percent change in body weight with an adjusted lower limit.

Figure 16 is a graph showing that treatment of mice with the combination of MM-398 with veliparib in C33A xenograft model described in Example 5 also lead to decreases in body weight as compared to administration of either

drug alone.

These studies demonstrated that this toxicity could be alleviated by delaying the start of PARP inhibitor dosing, preferably by 2-3 days after the day on which liposomal irinotecan was administered. A dosing schedule where the PARP inhibitor was only administered on days subsequent to administration of liposomal irinotecan was followed in mouse efficacy studies (Example 4) demonstrating therapeutic synergy of the combination of a PARP inhibitor and liposomal irinotecan in two cervical cancer tumor xenograft models (in which veliparib alone was not efficacious, and a second model in which neither MM-398 or veliparib were efficacious as single agents, however the combination demonstrated tumor growth inhibition).

**Example 4: Pre-Clinical Efficacy of Liposomal Irinotecan**

*In vivo* tumor xenograft studies demonstrated that the efficacy of liposomal irinotecan is greater than free irinotecan. In addition, *in vivo* tumor xenograft studies demonstrated MM-398 is related to high CES activity and/or high tumor levels of CPT-11 following dosing with MM-398. Additionally, MM-398 has demonstrated superior activity compared to equivalent dosing of free irinotecan in several pre-clinical models including breast, colon, ovarian, and pancreatic tumor xenograft models.

Liposomal irinotecan (MM-398) has greater efficacy in various cancer models, compared to non-liposomal irinotecan. Cancer cells were implanted subcutaneously in mice; when tumors were well established and had reached mean volumes of 200 mm3, IV treatment with free irinotecan, MM-398 or control was initiated. The doses of free and nanoliposomal irinotecan used in each study are indicated above, with dose time points indicated by arrows. Tumor permeability as well as tumor tissue carboxylesterase (CES) activity, which is responsible for the enzymatic conversion of CPT-11 to SN-38, are predicted to be critical factors for local tumor exposure of SN-38 following MM-398 dosing. In vivo tumor xenograft studies have demonstrated that efficacy of MM-398 is related to high CES activity and/or high tumor levels of CPT-11 following dosing with MM-398. Additionally, MM-398 has demonstrated superior activity compared to equivalent dosing of free irinotecan in several pre-clinical models including breast, colon, ovarian, and pancreatic tumor xenograft models.

**Example 5: Pre-Clinical Activity of Liposomal Irinotecan and PARP inhibitors**

Referring to Figure 11A and Figure 11B, the antitumor activity of MM-398 was studied in combinations with veliparib (PARPi) in multiple cervical xenograft models. In this study, MS-751 and C33A xenograft models of cervical cancer were employed to probe the effect of administering suboptimal doses of MM-398 in combination with the PARP inhibitor veliparib. Differential tissue levels of MM-398 at 24 and 72 hours indicated that MM-398 and the active metabolite SN-38 cleared faster from the liver, spleen, colon, and plasma, than from tumors. The combination of veliparib and MM-398 gave improvements in key PD biomarkers (cleaved caspase and yH2AX) when compared to veliparib or MM-398 alone. Figures 11A and 11B show that the combination of MM-398 + veliparib is synergistic. Two different cervical cancer xenograft models were utilized to study the efficacy of MM-398 dosed once weekly on Day 1 (arrows), veliparib dosed at 50 mg/kg orally once daily for 3 consecutive days on Days 4-6 of each week, or the combination dosed on the same schedule as the single agent treatments combined. (A) MS751 cervical cancer xenograft model using MM-398 dosed at 5 mg/kg and (B) C33A cervical cancer xenograft model using MM-398 dosed at 2 mg/kg. In the study, control mice were the same strain, and were harvested prior to tested mice (slightly younger). Data is not presented for mice removed from study for weight loss or for mice removed unintentionally before end date.

*Cervical MS-751 Xenograft Model*

The MS-751 Xenograft Model details are summarized in Table 6.

**Table 6**

| Mouse strain: | Nude (Tacoma) | | | |
|---|---|---|---|---|
| Tumor | Cervical MS-751, C33A | | | |
| Inoculation: | 5*10^6 (s.c.) in 30% MG | | | |
| Drug: | MM-398 (iv) + Veliparib (oral) | | | |
| Groups: | | Animal per group: | Dose (mpk) | |
| 1 | Saline | 10 | | |

(continued)

| Groups: | | Animal per group: | Dose (mpk) | |
|---|---|---|---|---|
| 2 | MM-398 | 10 | 5 | |
| 3 | veliparib/oral | 10 | 50 | 3/4/5th day |
| 4 | MM-398 +veliparib | 10 | 5+50 | 3/4/5th day |

Figure 13A shows that tumor volume decreased when MM-398 (5 mpk dose) was administered in combinations with veliparib in the MS751 xenograft model (p = 0.03) as compared to administration of either drug alone. Figure 13B shows that percent survival was better for mice treated with MM-398 (5 mpk dose) in combinations with veliparib in MS751 xenograft model as compared to treatment with either drug alone either drug administered alone. Figure 13C shows that treatment with the combination of MM-398 with veliparib in MS751 xenograft model lead to decreases in body weight as compared to administration of either drug alone.

*C33A Cervical Xenograft Model*

The C33A Xenograft Model details are summarized in Table 7.

**Table 7**

| Mice: | Female, Ncr Nudes (Taconic), 5-6 weeks. | |
|---|---|---|
| Cell Lines: | C33 A | |
| Tumor Inoculation: $5 \times 10^6$ in 100 μl Matrigel (30 vol%) sc | | |
| 15 mice per a cell line | | |
| Groups: | Dose, mpk: | |
| MM-398 alone | 2 | |
| Veliparib alone | 50 | |
| MM-398 + Veliparib (3-4-5 d) | 2+60 | |
| End-life Collection: 72 h after first injection | | |
| Frozen (Tumor, Liver, Spleen, Plasma) | | |
| FFPA (Tumor) | | |
| Analysis: | | |
| gamma H2AX and cleaved caspase/Tunnel in FFPE (Lia) | | |
| CPT-11 and SN-38 in all tissues for MM-398 flash frozen only (Roswell) | | |

Figure 14 shows that the combination of MM-398 with veliparib in the C33A xenograft model leads to decreases in tumor volume as compared to either drug alone administered alone. Figure 15 shows that percent survival was better for mice MM-398 (5 mpk dose) in combinations with veliparib in C33A xenograft model as compared to either drug administered alone.

**Example 6: Clinical Use of Liposomal Irinotecan and PARP inhibitors**

*Clinical Use of Liposomal Irinotecan and Veliparib*

This is a Phase 1 human dose escalation study to characterize the safety, tolerability, MTD and PK of MM-398 in combination with veliparib in order to determine an optimal combination dose and schedule that will be identified as the recommended Phase 2 dose. The following schematic outlines two different schedules of veliparib dosing that will be explored in combination with MM-398 bi-weekly dosing:

28-day cycle:
MM-398
MM-398
Day:
1 2 3 4 5 12 15 19 25 28
Schedule 1:
Schedule 2:
Veliparib
Veliparib

MM-398 will be administered by intravenous (IV) infusion over 90 minutes at a dose of 80 mg/m$^2$ every two weeks. MM-398 is administered by intravenous (IV) infusion over 90 minutes at a dose of 80 mg/m$^2$ (salt) irinotecan once every two weeks (days 1 and 15 of each 28-day treatment cycle). Veliparib is co-administered orally twice daily by the patient at home according to the following schedule:

**Table 8**

| Dose Level[1] | Veliparib Dose (mg BID) | Veliparib Dose Days | MM-398 Dose (salt) (mg/m$^2$ q2w) |
|---|---|---|---|
| 1 | 100 | Day 5-12; 19-25 | 80, Day 1, 15 |
| 2 | 200 | Day 5-12; 19-25 | 80, Day 1, 15 |
| 3 | 200 | Day 5-12; 17-25 | 80, Day 1, 15 |
| 4 | 300 | Day 5-12; 19-25 | 80, Day 1, 15 |
| 5 | 400 | Day 5-12; 19-25 | 80, Day 1, 15 |
| [1]Additional dose levels and alternate dosing schedules may be explored upon agreement of Sponsor, Medical Monitor and Investigators.<br>** After the MTD is reached, and for the first cycle only, we plan to enroll approximately 18 patients obtain tumor biopsies according to the schema outlined in the correlates section below. | | | |

The study will enroll 3 patients per dose cohort following a traditional 3 + 3 dose escalation design. Dose limiting toxicities (DLTs) will be evaluated during the first cycle of treatment (28 days) in order to determine the MTD. If there are no DLTs within the safety evaluation period, then the next cohort can be initiated following agreement between the Investigators and Medical Monitor. If a DLT occurs, then the cohort will be expanded to 6 patients. If 2 or more patients have DLTs within a given dose level, then the dose will not be escalated further; however, lower doses may be explored. Additional dosing schedules may also be explored depending on the safety, tolerability, and PK observed.

Given that these individual therapies have been studied in previous clinical trials, it is important that the safety assessment takes into account the expected safety profile of the standard dose regimens. For all treatment regimens, any toxicity that is related to disease progression will not be considered a DLT. The following events, occurring during cycle 1 of the study combination, will be considered DLTs if deemed drug-related:

grade 3 or 4 neutropenia complicated by fever ≥ 38.5 °C (i.e. febrile neutropenia) and/or documented infection;

grade 4 neutropenia that does not resolve within 7 days despite optimal therapy (withholding study drug and GCSF administration);

grade 4 thrombocytopenia that does not resolve within 7 days or any grade 3-4 thrombocytopenia complicated with hemorrhage;

grade 4 anemia that does not resolve within 7 days despite optimal therapy (withholding study drug and red blood cell transfusions);

inability to begin subsequent treatment course within 14 days of the scheduled date, due to study drug toxicity;

any grade 3-4 non-hematologic toxicity (except fatigue/asthenia < 2 weeks in duration; vomiting or diarrhea lasting less than 72 hours whether treated with an optimal antiemetic or anti-diarrheal regimen or not; or alkaline phosphatase

changes).

≥ grade 2 seizure

Patients will be treated until disease progression as determined by RECIST v1.1 criteria evaluated by CT scan every 8 weeks from first dose of study drug. The inclusion and exclusion criteria for the clinical trial are summarized in the table 9 below.

**Table 9**

| Inclusion Criteria | Exclusion Criteria |
|---|---|
| • Patients must have histologic or cytologic confirmation of cancer for which there is no known standard therapy capable of extending life expectancy.<br>• ECOG Performance Status 0 or 1<br>• Tumor lesion(s) amenable to multiple pass percutaneous biopsies and patient willing to undergo required pre- and post-treatment biopsies<br>• Must have adequate:<br>• Bone marrow function<br>◦ ANC > 1,500 cells/μl without the use of hematopoietic growth factors<br>◦ Platelet count > 100,000 cells/μl<br>◦ Hemoglobin > 9 g/dL<br>• Hepatic function<br>◦ Normal serum total bilirubin<br>◦ AST and ALT ≤ 2.5 x ULN (≤ 5 x ULN is acceptable if liver metastases are present)<br>• Renal function<br>◦ Serum creatinine ≤ 1.5 x ULN<br>• Normal ECG | • Active CNS metastasis<br>• Clinically significant GI disorders, including history of small bowel obstruction unless the obstruction was a surgically treated remote episode<br>• Prior irinotecan therapy; or topotecan therapy or bevacizumab therapy within 6 months of first dose of study treatment<br>• Prior chemotherapy or biological therapy within 3 weeks, or within a time interval less than 5 half-lives of the agent, prior to first dose of study treatment<br>• Prior radiotherapy within 4 weeks of first dose of study treatment<br>• Patients who have had radiation to the pelvis or other bone marrow-bearing sites will be considered on a case by case basis and may be excluded if the bone marrow reserve is not considered adequate (i.e. radiation to >25% of |
| • ≥18 years of age<br>• Able to understand and sign informed consent<br>• Prior PARP inhibitor therapy is allowed<br>• Willing to undergo pre-treatment ferumoxytol MRI (patients will be excluded from undergoing ferumoxytol MRI if they have evidence of iron overload , a known hypersensitivity to ferumoxytol or any other IV iron product, a documented history of multiple drug allergies, or those for whom MRI is otherwise contraindicated, including claustrophobia or anxiety related to undergoing MRI) | bone marrow)<br>• Known hypersensitivity to MM-398<br>• Active infection<br>• Pregnant or breast feeding |

The dose escalation portion of the trial may require up to 30 patients if 6 patients are required at each of 5 dose levels. An additional 18 patients may be used to explore the effect of veliparib on the biologic correlates. Thus, the accrual ceiling will be set at 48 patients.

The study is proposed to include all solid tumor types, however, particular indications that are of high interest for this study includes the following: cervical cancer, ovarian cancer, triple negative breast cancer (TNBC), non-small cell lung cancer (NSCLC), small cell lung cancer (SCLC), gastric cancer, pancreatic cancer, and neuroendocrine tumors.

The methods and uses herein can also be applied to other tumor suitable types including those noted for increased frequency of DNA damage response (DDR) pathway deficiencies (or 'BRCAness') found in sporadic tumors, which are predicted to be sensitive to PARP inhibitors. As mentioned previously, BRCA1 or BRCA2 deficiencies, found particularly in triple negative breast cancer and high-grade serous ovarian cancer, sensitize cells to PARP-inhibitors. Likewise, loss of function of other genes and proteins involved in DDR pathways, including the endonuclease XPF-ERCC1, the homologous recombination repair proteins meiotic recombination protein 11 (MRE11) and Fanconi anemia

pathway (FANC) proteins, also sensitize cells to PARP inhibitors. Fanconi anemia pathway deficiencies have been demonstrated in lung, cervical, and breast and ovarian cancers. These and other DDR pathway deficiencies may be predictive biomarkers for PARP inhibitor therapy, and will be explored retrospectively in this study. Veliparib, specifically, has also demonstrated clinical activity in a number of indications, including BRCA-positive and BRCA wild-type breast and ovarian cancer, as well as gastric cancer in combination with FOLFIRI. For the proposed study, indications were chosen not only for their high unmet medical need, but for potential sensitivity to irinotecan and/or veliparib based on the afore-mentioned pre-clinical and/or clinical experience. While the PARP inhibitor olaparib has recently been FDA approved as a monotherapy in BRCA+ ovarian cancer, this study will not limit treatment in the ovarian patient population to BRCA+ patients, as this is a phase I study of a combination therapy and may retrospectively identify patients with other DDR pathway deficiencies in addition to BRCA.

*Use of Liposomal Irinotecan and Olaparib*

MM-398 is administered by intravenous (IV) infusion over 90 minutes at a dose of 80 mg/m$^2$ (based on the corresponding amount of irinotecan hydrochloride trihydrate, equivalent to 70 mg/m$^2$ irinotecan free base) every two weeks. Olaparib is co-administered orally twice daily by the patient at home according to the following schedule (Table 10).

**Table 10**

| Dose Level[1] | Olaparib Dose (mg BID) | Olaparib Dose Days | MM-398 Dose (mg/m$^2$ q2w)* |
|---|---|---|---|
| 1 | 100 | Day 5-12; 19-25 | 80, Day 1, 15 |
| 2 | 200 | Day 5-12; 19-25 | 80, Day 1, 15 |
| 3 | 200 | Day 5-12; 17-25 | 80, Day 1, 15 |
| 4 | 300 | Day 5-12; 19 -25 | 80, Day 1, 15 |
| 5 | 400 | Day 5-12; 19-25 | 80, Day 1, 15 |
| *= The 80 mg/m$^2$ MM-398 dose is based on the corresponding amount of irinotecan hydrochloride trihydrate (equivalent to 70 mg/m$^2$ based on irinotecan free base). | | | |

**Example 7: Measuring phosphorylated H2AX in Tumor Biopsies**

Phosphorylated H2AX (γ-H2AX) plays an important role in the recruitment and/or retention of DNA repair and checkpoint proteins such as BRCA1, MRE11/RAD50/NBS1 complex, MDC1 and 53BP1. DNA damage has been shown to increase H2AX phosphorylation in cancer cells following exposure to camptothecins. If the PARP inhibitor compound(s) is/are able to increase the degree of DNA damage due to irinotecan from MM-398, it may be detectable by measurement of H2AX phosphorylation. An immunofluorescence assay was used in previous clinical studies. Patient peripheral blood mononuclear cells (PBMCs), hair follicles, and/or tumor biopsy samples will be collected if there is readily accessible disease. The association between the pharmacodynamic response measured by γ-H2AX level can be assessed by Fisher's test or the Wilcoxon rank sum test, as appropriate; this evaluation will be done at the MTD +/- a maximum of 2 dose levels (Figure 18).

**Table 11. Schedule for biopsies and surrogate samples**

| Dose Level | | PARPi Dose (mg BID) | PARPi Dose Days | MM-398 Dose (mg/m$^2$ q2w) | Biopsy in am for PD marker |
|---|---|---|---|---|---|
| 1 | | 100 | Day 5-12; 19-25 | 80, Day 1, 15 | -- |
| 2 | | 200 | Day 5-12; 19-25 | 80, Day 1, 15 | -- |
| 3 | | 200 | Day 3-12; 17-25 | 80, Day 1, 15 | Days 1, 5, 19 |
| 4 | | 300 | Day 3-12; 17-25 | 80, Day 1, 15 | Days 1, 5, 19 |
| 5 | | 400 | Day 3-12; 17-25 | 80, Day 1, 15 | Days 1, 5, 19 |
| Confirm | A | MTD | Day 3-12; 19-25 | 80, Day 1, 15 | Days 1, 5, 19 |
| | B | MTD | Day 5-12; 17-25 | 80, Day 1, 15 | Days 1, 5, 19 |

**Example 8: Administering and Detecting Ferumoxytol to Predict Deposition of Topoisomerase Inhibitor from Liposomal Irinotecan**

Figures 19A-19C show that FMX MRI may be a predictive tool for tumor response to MM-398. Figure 19A is a schematic showing that MM-398 and FMX have similar properties, including 1) extended PK, 2) the ability to deposit in tumor tissues through the EPR effect (i.e. leaky vasculature), and 3) uptake by macrophages. Therefore, visualization of FMX on MRI may be able to predict MM-398 deposition. (B) FMX concentration of individual patient lesions was calculated using a standard curve from MR images obtained 24h post-FMX injection. (C) FMX signal from lesions at 24h are grouped relative to the median value observed in the FMX MRI evaluable lesions and compared to the best change in lesion size based on CT scans (data available from 9 patients; total of 31 lesions).

The phase I study of MM-398 also examined the feasibility of magnetic resonance (MR) imaging to predict tumor-associated macrophage (TAM) content and MM-398 deposition. TAMs appear to play a key role in the deposition, retention and activation of MM-398 within the tumor microenvironment. In this clinical study, ferumoxytol (FMX) a microparticulate preparation of a superparamagnetic iron oxide coated with polyglucose sorbitol carboxymethylether) was used as an imaging contrast agent and MR images were obtained at 1h, 24h, and 72h following FMX injection. FMX is an approved therapy that is indicated for the treatment of iron deficiency anemia in adult patients with chronic kidney disease; however a growing number of cancer patients without iron deficiency are being administered FMX as an imaging agent to visualize macrophage content and vasculature. Like MM-398, FMX is also a nanoparticle with a diameter of approximately 17-31 nm. As tumor permeability was predicted to be an important factor in MM-398 efficacy, FMX was also investigated for use as a surrogate for liposome deposition (Figure 19A). A benefit of FMX is that this agent helps to identify patients that are less likely to respond to MM-398 because of poor drug uptake. Ferumoxytol as a diagnostic test enables the detection of a patient population that would significantly benefit from MM-398 that would otherwise be uncategorized.

The MRI results from a human clinical trial study demonstrated that the amount of FMX depositing in tumor lesions was able to be quantified (Figure 19B), and it was subsequently shown that a correlation existed between tumor lesion ferumoxytol uptake by MRI and response to MM-398 (Figure 19C). This correlation is now being studied further in an expansion of the Phase 1 study, and is included as a correlative imaging study for a trial of MM-398 + veliparib.

FMX is an iron replacement product indicated for the treatment of iron deficiency anemia in adult patients with chronic kidney disease. Although not approved as an indication, ferumoxytol has also been used as an imaging agent in cancer patients and will be utilized as such in this study. At least 2 days prior to Cycle 1 Day 1 (maximum of 8 days prior) a single dose of 5 mg/kg FMX will be administered by intravenous injection. The total single dose will not exceed 510 mg, the maximum approved single dose of FMX. This dosing schedule is less intense than the approved label, which recommends two doses of 510 mg 3 to 8 days apart; however since FMX is being used as imaging agent in this study as opposed to a replacement product for iron deficiency, a lower dose is more appropriate. Three MRIs will be performed for each patient over 2 days. All patients will have a baseline image acquired prior to the FMX infusion, and a second image acquired 1-4 h after the end of FMX administration. All patients will return the following day for a 24 h FMX-MRI using the same protocol and sequences as previously. Each patient will be required to complete their FMX-MRIs on the same scanner to reduce inter-scan variability. The body area to be scanned will be determined by the location of the patient's disease. Each MRI study will be evaluated for image quality and signal characteristics of tumors and reference tissue on T1-, T2- and T2*- weighted sequences. Once a completed set of images from each patient has been received, a qualitative review will be performed and sent to a quantitative lab for analysis. The data will be analyzed in a similar fashion as described above.

**Imaging Correlates Table 12**

| Correlative Objective | Imaging Technique | Organ(s) Scanned and Timing of Scans |
|---|---|---|
| Ferumoxytol (FMX) uptake | MRI | Sites of disease; 3 scans completed approximately 2-6 days prior to Cycle 1 Day 1. Scan time points:<br>-baseline (immediately prior to FMX infusion)<br>-lh (post-FMX infusion)<br>-24h (post-FMX infusion) |

(continued)

| Correlative Objective | Imaging Technique | Organ(s) Scanned and Timing of Scans |
|---|---|---|
| Histone gamma-H2AX (Pommier, DTB-CCR; Doroshow, Leidos) | Immunofluorescence microscopy ELISA (in development) | - Tumor biopsy before treatment, and during treatment.<br>- Hair follicles during treatment. PBMC before treatment and during treatment |

Imaging Correlate Study

Patients will be eligible to participate in the FMX imaging study if they do not meet any of the following criteria:

- ◦ Evidence of iron overload as determined by:
  - Fasting transferrin saturation of >45% and/or
  - Serum ferritin levels >1000 ng/ml
- ◦ A history of allergic reactions to any of the following:
  - compounds similar to ferumoxytol or any of its components as described in full prescribing information for ferumoxytol injection
  - any IV iron replacement product (e.g. parenteral iron, dextran, iron-dextran, or parenteral iron polysaccharide preparations)
  - multiple drugs
- ◦ Unable to undergo MRI or for whom MRI is otherwise contraindicated (e.g. presence of errant metal, cardiac pacemakers, pain pumps or other MRI incompatible devices; or history claustrophobia or anxiety related to undergoing MRI)

If a patient consents to FMX-MRI, the patient will receive ferumoxytol infusion and undergo the required FMX-MRI scans approximately 2-6 days prior to beginning MM-398 treatment (the FMX period). FMX will be administered at a dose of 5 mg/kg up to a maximum of 510 mg. All other aspects of administration will be consistent with the latest ferumoxytol prescribing information. A detailed FMX-MRI protocol will be included in the study imaging manual. Briefly, each patient will be required to complete their FMX-MRIs on the same scanner to reduce inter-scan variability. Each MRI study will be evaluated for image quality and signal characteristics of tumors and reference tissue on T1-, T2- and T2*- weighted sequences. Once a completed set of images from each patient has been received, the images will be loaded onto the viewing workstation for qualitative review and then sent to a quantitative lab (handled by central imaging CRO) for analysis.

Multiple MR images will be collected on Day 1-Day 2 of the FMX period at various time points: a baseline image acquired prior to the FMX infusion, a second image occurring 1-4 h after the end of FMX administration, and a third image at approximately 24 h post-FMX, using the same protocol and sequences as on Day 1. The body areas to be scanned will be determined by the location of the patient's disease; detailed instructions will be described in the study imaging manual.

**Example 9: Clinical Use of Liposomal Irinotecan in Combination with 5-fluorouracil and leucovorin**

Clinical efficacy of MM-398 has also been demonstrated in gemcitabine-refractory metastatic pancreatic cancer patients: in a randomized, Phase 3, international study (NAPOLI-1), MM-398 was given in combination with 5-fluorouracil/leucovorin (5-FU/LV) and significantly prolonged overall survival (OS) compared to 5-FU/LV treatment alone. The median OS for the MM-398-containing arm was 6.1 months compared to 4.2 months for the control arm (HR=0.67, p=0.0122). Because the active pharmaceutical ingredient in MM-398 is irinotecan, the safety profile was, as anticipated, qualitatively similar to irinotecan, where the most common adverse events (≥30%) are nausea, vomiting, abdominal pain, diarrhea, constipation, anorexia, neutropenia, leukopenia (including lymphocytopenia), anemia, asthenia, fever, body weight decreasing, and alopecia (irinotecan package insert). Table 14 provides a summary of Grade 3 or higher safety data of patients treated with MM-398 plus 5-FU/LV from the NAPOLI-1 study. Table 13 provides toxicities observed in the Phase I monotherapy study, for comparison.

**Table 13. Summary of the most common (>10%) grade 3 or greater adverse events from the 13 patients treated with MM-398 monotherapy at a dose of 80 mg/m$^2$ every 2 weeks during the phase I study.**

| Adverse Events ≥ Grade 3 in Study MM-398-01-01-02 | |
|---|---|
| | n (%) |
| Diarrhea | 4 (30.8) |
| Hypokalemia | 3 (23.1) |
| Abdominal pain | 2 (15.4) |
| Anemia | 2 (15.4) |
| Nausea | 2 (15.4) |
| Neutropenia | 2 (15.4) |

**Table 14. Summary of Grade 3 or higher AEs from the NAPOLI-1 phase III study.**

| | MM-398 + 5-FU/LV[1] (N=117) | 5-FU/LV[2] (N=134) |
|---|---|---|
| GRADE ≥3 NON-HEMATOLOGIC AEs IN >5% PATIENTS, %[3] | % | % |
| Fatigue | 14 | 4 |
| Diarrhea | 13 | 5 |
| Vomiting | 11 | 3 |
| Nausea | 8 | 3 |
| Asthenia | 8 | 7 |
| Abdominal pain | 7 | 6 |
| Decreased appetite | 4 | 2 |
| Hypokalemia | 3 | 2 |
| Hypernatremia | 3 | 2 |
| GRADE ≥3 HEMATOLOGIC AES BASED ON LABORATORY VALUES, %[3,4] | | |
| Neutrophil count decreased | 20 | 2 |
| Hemoglobin decreased | 6 | 5 |
| Platelet count decreased | 2 | 0 |

[1] Dose: 80 mg/m$^2$ MM-398 + 2400 mg/m$^2$ over 46 h/400 mg/m$^2$ 5-FU/LV q2w
[2] Dose: 2000 mg/m$^2$ over 24 h/200 mg/m$^2$ 5-FU/LV weekly x 4, q6w
[3] Per CTCAE Version 4
[4] Includes only patients who had at least one post-baseline assessment

**Example 10: Cell survival for various TNBC cell lines following SN-38 and PARP inhibitor combination treatment.**

Tables 15 and 16 provide the results of *in vitro* measurements of cell survival for various triple negative breast cancer (TNBC) cancer cell lines to determine the cell viability following treatment with SN-38 and/or a PARP inhibitor. Table 15 provides IC50 data and Table 16 provides Maximum Kill data.

The experiments that generated these data were performed in 384 well format. Cells were plated at 1000 cells/well and then incubated for 24 hours. Then SN-38 and/or one of four different PARP inhibitors (talazoparib niraparib, olaparib or rucaparib) was added and incubated for an additional 24 hours then the wells were washed with PBS to remove the drug and fresh media was added back into the wells. The plates were then allowed to incubate for 72 hours period. After the 72 hour incubation period the media was removed and cell viability was determined using the CellTiter-Glo® cell viability assay (Promega, Madison WI) according to the product instructions. Figures 3A and 3B are line graphs that depict cell viability in BT20 and HCC38 breast cancer cell lines, respectively, following treatment with SN-38 and/or talazoparib.

**Table 15 IC50 log10(uM)**

| Exp. 1 | Treatment | Cell Line | | | | | |
|---|---|---|---|---|---|---|---|
| | | BT20 | SUM159PT | HCC38 | HCC1187 | HCC1806 | BT549 |
| | SN38 | -0.18 | -2.35 | -2.80 | -0.68 | -2.08 | -0.10 |
| | Niraparib | 2.14 | 0.35 | 1.23 | 2.11 | 1.27 | 2.03 |
| | SN38 & Niraparib (3ug/ml) | -0.67 | -3.99 | -0.12 | -1.58 | -2.80 | -0.39 |
| | SN38 & Niraparib (1ug/ml) | -0.70 | -3.42 | -4.09 | -1.45 | -2.62 | -0.64 |
| | SN38 & Niraparib (0.3ug/ml) | -0.71 | -2.85 | -4.23 | -1.61 | -2.55 | -0.74 |
| | SN38 & Niraparib (0.1ug/ml) | -0.61 | -2.87 | -4.05 | -1.41 | -2.52 | -0.55 |
| Exp. 2 | Treatment | Cell Line | | | | | |
| | | BT20 | SUM149PT | SUM159PT | HCC70 | HCC1187 | BT549 |
| | SN38 | -0.69 | 0.24 | -2.39 | -0.07 | -0.64 | -0.04 |
| | Olaparib | 1.24 | 2.40 | 0.18 | $-4.2 \times 10^{7}$ | 2.41 | 2.04 |
| | SN38 & Olaparib (3ug/ml) | -1.48 | -0.19 | -3.70 | -0.58 | -1.77 | -0.55 |
| | SN38 & Olaparib (1ug/ml) | -1.49 | -0.34 | -3.31 | -0.49 | -1.67 | -0.48 |
| | SN38 & Olaparib (0.3ug/ml) | -1.44 | -0.18 | -2.92 | -0.50 | -1.35 | -0.35 |
| | SN38 & Olaparib (0.1ug/ml) | -1.29 | -0.11 | -2.92 | -0.48 | -1.56 | -0.04 |
| Exp. 3 | Treatment | Cell Line | | | | | |
| | | BT20 | SUM149PT | SUM159PT | HCC38 | HCC1954 | BT549 |
| | SN38 | -0.37 | 0.27 | -2.66 | -2.89 | -0.97 | -0.05 |
| | Rucaparib | 1.27 | 1.68 | -0.07 | -0.07 | 1.60 | 1.75 |
| | SN38 & Rucaparib (3ug/ml) | -1.33 | -0.16 | -3.64 | 4.93 | -1.22 | -0.48 |
| | SN38 & Rucaparib (1ug/ml) | -1.47 | -0.23 | -3.28 | -3.88 | -1.33 | -0.57 |
| | SN38 & Rucaparib (0.3ug/ml) | -1.48 | -0.49 | -3.23 | -4.01 | -1.51 | -0.49 |
| | SN38 & Rucaparib (0.1ug/ml) | -1.24 | -0.10 | -3.11 | -3.29 | -1.57 | -0.52 |
| Exp. 4 | Treatment | Cell Line | | | | | |
| | | BT20 | SUM159PT | HCC38 | HCC1187 | HCC1954 | SKBR3 |
| | SN38 | -0.24 | -2.33 | -2.75 | -0.98 | -0.65 | -1.38 |
| | Talazoparib | 1.45 | -1.03 | -1.23 | 2.28 | 3.64 | $-2.8 \times 10^{4}$ |
| | SN38 & Talazoparib (3ug/ml) | -1.88 | -4.01 | -3.41 | -1.79 | -1.64 | -2.05 |
| Exp. 1 | Treatment | Cell Line | | | | | |
| | SN38 & Talazoparib (1ug/ml) | -1.70 | -4.01 | -4.01 | -1.79 | -1.51 | -2.65 |
| | SN38 & Talazoparib (0.3ug/ml) | -1.10 | -4.01 | -5.46 | -1.94 | -1.45 | -2.23 |
| | SN38 & Talazoparib (0.1 ug/ml) | -1.36 | -4.01 | -2.87 | -1.92 | -1.29 | -2.41 |

**Table 16 Maximum Kill**

| Exp. 1 | Treatment | Cell Line | | | | | |
|---|---|---|---|---|---|---|---|
| | | BT20 | SUM159PT | HCC38 | HCC1187 | HCC1806 | BT549 |
| | SN38 | 100 | 97 | 96 | 90 | 93 | 95 |
| | Niraparib | 100 | 97 | 100 | 98 | 100 | 100 |
| | SN38 & Niraparib (3ug/ml) | 100 | 100 | | 89 | 91 | 94 |
| | SN38 & Niraparib (1ug/ml) | 100 | 100 | 93 | 93 | 92 | 92 |
| | SN38 & Niraparib (0.3ug/ml) | 100 | 99 | 100 | 89 | 92 | 92 |
| | SN38 & Niraparib (0.1ug/ml) | 100 | 100 | 100 | 89 | 93 | 94 |
| Exp. 2 | Treatment | | Cell Line | | | | |
| | | BT20 | SUM149PT | SUM159PT | HCC70 | HCC1187 | BT549 |
| | SN38 | 100 | 96 | 97 | 97 | 100 | 93 |
| | Olaparib | 98 | 100 | 94 | 50 | 87 | 100 |
| | SN38 & Olaparib (3ug/ml) | 98 | 97 | 100 | 98 | 100 | |
| | SN38 & Olaparib (lug/ml) | 99 | 96 | 97 | 100 | 91 | 96 |
| | SN38 & Olaparib (0.3ug/ml) | 100 | 98 | 99 | 100 | 99 | 94 |
| | SN38 & Olaparib (0.1ug/ml) | 100 | 96 | 99 | 100 | 99 | 96 |
| Exp. 3 | Treatment | | | Cell Line | | | |
| | | BT20 | SUM149PT | SUM159PT | HCC38 | HCC1954 | BT549 |
| | SN38 | 100 | 95 | 99 | 92 | 94 | 94 |
| | Rucaparib | 100 | 99 | 97 | 87 | 100 | 100 |
| | SN38 & Rucaparib (3ug/ml) | 92 | 97 | 99 | | 96 | 93 |
| | SN38 & Rucaparib (lug/ml) | 100 | 97 | 99 | 98 | 94 | 92 |
| | SN38 & Rucaparib (0.3ug/ml) | 94 | 95 | 100 | 98 | 95 | 93 |
| | SN38 & Rucaparib (0.1ug/ml) | 96 | 100 | 97 | 97 | 93 | 94 |
| Exp. 4 | Treatment | | Cell Line | | | | |
| | | BT20 | SUM159PT | HCC38 | HCC1187 | HCC1954 | SKBR3 |
| | SN38 | 100 | 96 | 92 | 88 | 100 | 88 |
| | Talazoparib | 100 | 94 | 92 | | 100 | |
| | SN38 & Talazoparib (3ug/ml) | 100 | | | 89 | 93 | 90 |
| | SN38 & Talazoparib (1ug/ml) | 90 | | | 89 | 94 | 89 |
| | SN38 & Talazoparib (0.3ug/ml) | 93 | | | 89 | 94 | 100 |
| | SN38 & Talazoparib (0.1ug/ml) | 93 | | | 100 | 96 | 87 |

## Claims

1. Liposomal irinotecan for use in an antineoplastic therapy for the treatment of a solid tumor in a patient, wherein the liposomal irinotecan is administered in combination with a Poly(ADP-ribose) Polymerase (PARP) inhibitor , wherein the liposomal irinotecan is repeatedly administered, and wherein the treatment comprises:

i) administering to the patient liposomal irinotecan once every two weeks; and
ii) administering the PARP inhibitor daily for 3 to 10 days between consecutive administrations of the liposomal irinotecan, wherein the PARP inhibitor is administered at least 2, 3, 4 or 5 days after the administration of the liposomal irinotecan and at least 2, 3, 4 or 5 days prior to the next administration of the liposomal irinotecan.

**2.** Liposomal irinotecan for use in an antineoplastic therapy for the treatment of a solid tumor in a patient, wherein the liposomal irinotecan is administered in combination with a Poly(ADP-ribose) Polymerase (PARP) inhibitor, the treatment comprising a 28-day antineoplastic therapy treatment cycle consisting of:

i) administering the liposomal irinotecan on days 1 and 15 of the treatment cycle, and
ii) administering the PARP inhibitor on one or more days starting at least 3 days after the liposomal irinotecan and ending at least 1 day prior to administration of additional liposomal irinotecan.

**3.** The liposomal irinotecan for use of claim 2, wherein the PARP inhibitor is not administered for at least 3 days prior to the next administration of liposomal irinotecan.

**4.** The liposomal irinotecan for use of any one of claims 2-3, wherein the PARP inhibitor is administered on one or more of days 5 to 12 or on one or more of days 3 to 12 of the antineoplastic therapy treatment cycle.

**5.** The liposomal irinotecan for use of any one of claims 2-4, wherein the PARP inhibitor is administered on one or more of days 19 to 25 or on one or more of days 17 to 25 of the antineoplastic therapy treatment cycle.

**6.** The liposomal irinotecan for use of claim 1, wherein the PARP inhibitor is administered on each of consecutive days 3 to 10 between the days when the liposomal irinotecan is administered.

**7.** The liposomal irinotecan for use of any one of claims 1-6, wherein the liposomal irinotecan has an irinotecan terminal elimination half-life of 26.8 hours and a maximal irinotecan plasma concentration of 38.0 micrograms/ml.

**8.** The liposomal irinotecan for use of any one of claims 1-7, wherein each administration of liposomal irinotecan is administered at a dose of 80 mg/m$^2$ (salt) or 70 mg/m$^2$ (free base).

**9.** The liposomal irinotecan for use of any one of claims 1-8, wherein each administration of the PARP inhibitor is administered at a dose of from about 20 mg/day to about 800 mg/day and/or wherein the PARP inhibitor is administered once or twice daily at a dose of from about 20 mg/day to about 400 mg/day.

**10.** The liposomal irinotecan for use of any one of claims 1-9, wherein the PARP inhibitor is selected from the group consisting of niraparib, olaparib, veliparib, rucaparib and talazoparib.

**11.** The liposomal irinotecan for use of any one of claims 1-10, wherein the patient with a solid tumor has a cancer selected from cervical cancer, ovarian cancer, triple negative breast cancer, non-small cell lung cancer, small cell lung cancer, gastrointestinal stromal tumors gastric cancer, pancreatic cancer, colorectal cancer, or a neuroendocrine cancer.

**12.** The liposomal irinotecan for use of any one of claims 1-11, wherein the patient with a solid tumor has cervical cancer and the PARP inhibitor is veliparib.

**13.** The liposomal irinotecan for use of any one of claims 1-11, wherein the patient with a solid tumor has cervical cancer and the PARP inhibitor is olaparib.

**14.** The liposomal irinotecan of any one of claims 1-13, wherein the liposomal irinotecan includes irinotecan sucrosofate salt encapsulated in liposomes for intravenous use.

## Patentansprüche

**1.** Liposomales Irinotecan zur Verwendung bei einer antineoplastischen Therapie für die Behandlung eines soliden Tumors bei einem Patienten, wobei das liposomale Irinotecan in Kombination mit einem PARP(Poly(ADP-Ribose) Polymerase)-Inhibitor verabreicht wird, wobei das liposomale Irinotecan wiederholt verabreicht wird und wobei die

Behandlung Folgendes umfasst:

i) Verabreichen von liposomalem Irinotecan einmal alle zwei Wochen an den Patienten; und
ii) tägliches Verabreichen des PARP-Inhibitors 3 bis 10 Tage lang zwischen aufeinander folgenden Verabreichungen des liposomalen Irinotecans, wobei der PARP-Inhibitor wenigstens 2, 3, 4 oder 5 Tage nach der Verabreichung des liposomalen Irinotecans und wenigstens 2, 3, 4 oder 5 Tage vor der nächsten Verabreichung des liposomalen Irinotecans verabreicht wird.

**2.** Liposomales Irinotecan zur Verwendung bei einer antineoplastischen Therapie für die Behandlung eines soliden Tumors bei einem Patienten, wobei das liposomale Irinotecan in Kombination mit einem PARP(Poly(ADP-Ribose) Polymerase)-Inhibitor verabreicht wird, wobei das liposomale Irinotecan wiederholt verabreicht wird, wobei die Behandlung einen 28-tägigen Antineoplastische-Therapie-Behandlungszyklus umfasst, bestehend aus:

i) Verabreichen des liposomalen Irinotecans an Tag 1 und 15 des Behandlungszyklus und
ii) Verabreichen des PARP-Inhibitors an einem oder mehreren Tagen, beginnend wenigstens 3 Tage nach dem liposomalen Irinotecan und endend wenigstens 1 Tag vor Verabreichung von weiterem liposomalem Irinotecan.

**3.** Liposomales Irinotecan zur Verwendung nach Anspruch 2, wobei der PARP-Inhibitor vor der nächsten Verabreichung von liposomalem Irinotecan wenigstens 3 Tage lang nicht verabreicht wird.

**4.** Liposomales Irinotecan zur Verwendung nach einem der Ansprüche 2-3, wobei der PARP-Inhibitor an einem oder mehreren der Tage 5 bis 12 oder an einem oder mehreren der Tage 3 bis 12 des Antineoplastische-Therapie-Behandlungszyklus verabreicht wird.

**5.** Liposomales Irinotecan zur Verwendung nach einem der Ansprüche 2-4, wobei der PARP-Inhibitor an einem oder mehreren der Tage 19 bis 25 oder an einem oder mehreren der Tage 17 bis 25 des Antineoplastische-Therapie-Behandlungszyklus verabreicht wird.

**6.** Liposomales Irinotecan zur Verwendung nach Anspruch 1, wobei der PARP-Inhibitor jeweils an aufeinander folgenden Tagen 3 bis 10 zwischen den Tagen, an denen das liposomale Irinotecan verabreicht wird, verabreicht wird.

**7.** Liposomales Irinotecan zur Verwendung nach einem der Ansprüche 1-6, wobei das liposomale Irinotecan eine Terminale-Irinotecan-Eliminierung-Halbwertszeit von 26,8 Stunden und eine maximale Irinotecan-Plasmakonzentration von 38,0 Mikrogramm pro ml aufweist.

**8.** Liposomales Irinotecan zur Verwendung nach einem der Ansprüche 1-7, wobei die Verabreichung von liposomalem Irinotecan jeweils bei einer Dosis von 80 mg/m$^2$ (Salz) oder 70 mg/m$^2$ (freie Base) verabreicht wird.

**9.** Liposomales Irinotecan zur Verwendung nach einem der Ansprüche 1-8, wobei die Verabreichung des PARP-Inhibitors jeweils bei einer Dosis von etwa 20 mg/Tag bis etwa 800 mg/Tag verabreicht wird und/oder wobei der PARP-Inhibitor ein- oder zweimal täglich in einer Dosis von etwa 20 mg/Tag bis etwa 400 mg/Tag verabreicht wird.

**10.** Liposomales Irinotecan zur Verwendung nach einem der Ansprüche 1-9, wobei der PARP-Inhibitor ausgewählt ist aus der Gruppe bestehend aus Niraparib, Olaparib, Veliparib, Rucaparib und Talazoparib.

**11.** Liposomales Irinotecan zur Verwendung nach einem der Ansprüche 1-10, wobei bei dem Patienten mit einem soliden Tumor ein Krebs ausgewählt aus Zervixkarzinom, Ovarialkarzinom, dreifach negativem Brustkrebs, nichtkleinzelligem Lungenkrebs, kleinzelligem Lungenkrebs, Stromatumoren des Magen-Darm-Trakts Magenkrebs, Bauchspeicheldrüsenkrebs, Kolorektalkarzinom oder einem neuroendokrinen Krebs vorliegt.

**12.** Liposomales Irinotecan zur Verwendung nach einem der Ansprüche 1-11, wobei bei dem Patienten mit einem soliden Tumor ein Zervixkarzinom vorliegt und es sich bei dem PARP-Inhibitor um Veliparib handelt.

**13.** Liposomales Irinotecan zur Verwendung nach einem der Ansprüche 1-11, wobei bei dem Patienten mit einem soliden Tumor ein Zervixkarzinom vorliegt und es sich bei dem PARP-Inhibitor um Olaparib handelt.

**14.** Liposomales Irinotecan zur Verwendung nach einem der Ansprüche 1-13, wobei das liposomale Irinotecan Irinotecan-Saccharoseoctasulfatsalz eingeschlossen in Liposomen zur intravenösen Anwendung umfasst.

## Revendications

1. Irinotécan liposomal pour une utilisation dans une thérapie antinéoplasique pour le traitement d'une tumeur solide chez un patient, dans lequel l'irinotécan liposomal est administré en association avec un inhibiteur de poly(ADP-ribose) polymérase (PARP), dans lequel l'irinotécan liposomal est administré d'une manière répétée, et dans lequel le traitement comprend :

   i) l'administration au patient d'irinotécan liposomal une fois toutes les deux semaines ; et
   ii) l'administration de l'inhibiteur de PARP chaque jour pendant 3 à 10 jours entre des administrations consécutives de l'irinotécan liposomal, l'inhibiteur de PARP étant administré au moins 2, 3, 4 ou 5 jours après l'administration de l'irinotécan liposomal et au moins 2, 3, 4 ou 5 jours avant l'administration suivante de l'irinotécan liposomal.

2. Irinotécan liposomal pour une utilisation dans une thérapie antinéoplasique pour le traitement d'une tumeur solide chez un patient, dans lequel l'irinotécan liposomal est administré en association avec un inhibiteur de poly(ADP-ribose) polymérase (PARP), le traitement comprenant un cycle de traitement de thérapie antinéoplasique de 28 jours consistant en :

   i) l'administration de l'irinotécan liposomal les jours 1 et 15 du cycle de traitement, et
   ii) l'administration de l'inhibiteur de PARP un ou plusieurs jours, en partant au moins 3 jours après l'irinotécan liposomal, et se terminant au moins un jour avant administration d'irinotécan liposomal additionnel.

3. Irinotécan liposomal pour une utilisation selon la revendication 2, dans lequel l'inhibiteur de PARP n'est pas administré pendant au moins 3 jours avant l'administration suivante d'irinotécan liposomal.

4. Irinotécan liposomal pour une utilisation selon l'une quelconque des revendications 2-3, dans lequel l'inhibiteur de PARP est administré un ou plusieurs des jours 5 à 12, ou un ou plusieurs des jours 3 à 12 du cycle de traitement de thérapie antinéoplasique.

5. Irinotécan liposomal pour une utilisation selon l'une quelconque des revendications 2 à 4, dans lequel l'inhibiteur de PARP est administré un ou plusieurs des jours 19 à 25 ou un ou plusieurs des jours 17 à 25 du cycle de traitement de thérapie antinéoplasique.

6. Irinotécan liposomal pour une utilisation selon la revendication 1, dans lequel l'inhibiteur de PARP est administré chacun des jours consécutifs 3 à 10 entre les jours au cours desquels l'irinotécan liposomal est administré.

7. Irinotécan liposomal pour une utilisation selon l'une quelconque des revendications 1 à 6, dans lequel l'irinotécan liposomal a une demi-vie d'élimination terminale d'irinotécan de 26,8 heures et une concentration plasmatique maximale d'irinotécan de 38,0 microgrammes/ml.

8. Irinotécan liposomal pour une utilisation selon l'une quelconque des revendications 1 à 7, dans lequel chaque administration d'irinotécan liposomal est effectuée à une dose de 80 mg/m$^2$ (sel) ou de 70 mg/m$^2$ (base libre).

9. Irinotécan liposomal pour une utilisation selon l'une quelconque des revendications 1 à 8, dans lequel chaque administration de l'inhibiteur de PARP est effectuée à une dose d'environ 20 mg/jour à environ 800 mg/jour et/ou dans lequel l'inhibiteur de PARP est administré une ou deux fois par jour à une dose d'environ 20 mg/jour à environ 400 mg/jour.

10. Irinotécan liposomal pour une utilisation selon l'une quelconque des revendications 1 à 9, dans lequel l'inhibiteur de PARP est choisi dans le groupe consistant en le niraparib, l'olaparib, le véliparib, le rucaparib et le talazoparib.

11. Irinotécan liposomal pour une utilisation selon l'une quelconque des revendications 1 à 10, dans lequel le patient ayant une tumeur solide présente un cancer choisi parmi le cancer du col de l'utérus, le cancer des ovaires, le cancer du sein triple négatif, le cancer du poumon non à petites cellules, le cancer du poumon à petites cellules, les tumeurs stromales gastro-intestinales, le cancer gastrique, le cancer du pancréas, le cancer colorectal, ou un cancer neuroendocrinien.

12. Irinotécan liposomal pour une utilisation selon l'une quelconque des revendications 1 à 11, dans lequel le patient

ayant une tumeur solide présente un cancer du col de l'utérus, et l'inhibiteur de PARP est le véliparib.

**13.** Irinotécan liposomal pour une utilisation selon l'une quelconque des revendications 1 à 11, dans lequel le patient ayant une tumeur solide présente un cancer du col de l'utérus, et l'inhibiteur de PARP est l'olaparib.

**14.** Irinotécan liposomal selon l'une quelconque des revendications 1 à 13, dans lequel l'irinotécan liposomal comprend un sel sucrosofate d'irinotécan encapsulé dans des liposomes pour une utilisation intraveineuse.

ME-180 cells viability (1000cells/well in 384-well plate) treated with SN-38 and with different PARP inhibitors (0.33 ug/ml) for 24hrs, washed and incubated for additional 72hrs with fresh media
% of control
120
100
80
60
40
20
0
0.000001 0.000010 0.000100 0.001000 0.010000 0.100000 1.000000 10.000000 100.000000
ug/ml
Veliparib
SN-38
SN-38 Niraparib (0.3ug/ml)
SN-38 Olaparib (0.3ug/ml)
SN-38 Veliparib (0.3ug/ml)
Olaparib
Niraparib

FIG. 1A

NS-751 cells viability (1000cells/well in 384-well plate) treated with SN-38 and with different PARP inhibitors (0.33 ug/ml) for 24hrs, washed and incubated for additional 72hrs with fresh media
% of control
120
100
80
60
40
20
0
0.000001
0.000010
0.000100
0.001000
0.010000
0.100000
1.000000
10.000000
100.000000
ug/ml
Veliparib
SN-38
SN-38 Niraparib (0.3ug/ml)
SN-38 Olaparib (0.3ug/ml)
SN-38 Veliparib (0.3ug/ml)
Olaparib
Niraparib

FIG. 1B

C-33A cells viability (1000cells/well in 384-well plate) treated with SN-38 and with different PARP inhibitors (0.33 ug/ml) for 24hrs, washed and incubated for additional 72hrs with fresh media
% of control
120
100
80
60
40
20
0
0.000001
0.000010
0.000100
0.001000
0.010000
0.100000
1.000000
10.000000
100.000000
ug/ml
Veliparib
SN-38
SN-38 Niraparib (0.3ug/ml)
SN-38 Olaparib (0.3ug/ml)
SN-38 Veliparib (0.3ug/ml)
Olaparib
Niraparib

FIG. 1C

SW756 cells viability (1000cells/well in 384-well plate) treated with
SN-38 and with different PARP inhibitors (0.33 ug/ml) for 24hrs, washed and
incubated for additional 72hrs with fresh media
% of control
120
100
80
60
40
20
0
0.000001
0.000010
0.000100
0.001000
0.010000
0.100000
1.000000
10.000000
100.000000
ug/ml
Veliparib
SN-38
SN-38 Niraparib (0.3ug/ml)
SN-38 Olaparib (0.3ug/ml)
SN-38 Veliparib (0.3ug/ml)
Olaparib
Niraparib

FIG. 1D

SiHa cells viability (1000cells/well in 384-well plate) treated with SN-38 and with different PARP inhibitors (0.33 ug/ml) for 24hrs, washed and incubated for additional 72hrs with fresh media
% of control
120
100
80
60
40
20
0
0.000001
0.000010
0.000100
0.001000
0.010000
0.100000
1.000000
10.000000
100.000000
ug/ml
Veliparib
SN-38
SN-38 Niraparib (0.3ug/ml)
SN-38 Olaparib (0.3ug/ml)
SN-38 Veliparib (0.3ug/ml)
Olaparib
Niraparib

FIG. 1E

DMS-114_Rucaparib: SN-38 (2nM)
Cell Number (%)
400
300
200
100
0
0
20
40
60
80
Time (Hours)
Control
Rucaparib (2000nM)
Rucaparib (4000nM)
Rucaparib (8000nM)
SN-38 (2nM) + Rucaparib (0nM)
SN-38 (2nM) + Rucaparib (2000nM)
SN-38 (2nM) + Rucaparib (4000nM)
SN-38 (2nM) + Rucaparib (8000nM)

FIG. 2A

NCI-H1048_Rucaparib: SN-38 (2nM)
Cell Number (%)
200
150
100
50
0
0
20
40
60
80
Time (Hours)
Control
Rucaparib (2000nM)
Rucaparib (4000nM)
Rucaparib (8000nM)
SN-38 (2nM) + Rucaparib (0nM)
SN-38 (2nM) + Rucaparib (2000nM)
SN-38 (2nM) + Rucaparib (4000nM)
SN-38 (2nM) + Rucaparib (8000nM)

FIG. 2B

CFPAC-1_Rucaparib: SN-38 (2nM)
Cell Number (%)
500
400
300
200
100
0
0
20
40
60
80
Time (Hours)
Control
Rucaparib (2000nM)
Rucaparib (4000nM)
Rucaparib (8000nM)
SN-38 (2nM) + Rucaparib (0nM)
SN-38 (2nM) + Rucaparib (2000nM)
SN-38 (2nM) + Rucaparib (4000nM)
SN-38 (2nM) + Rucaparib (8000nM)

FIG. 2C

BxPC-3_Rucaparib: SN-38 (2nM)
Cell Number (%)
300
200
100
0
Time (Hours)
0
20
40
60
80
Control
Rucaparib (2000nM)
Rucaparib (4000nM)
Rucaparib (8000nM)
SN-38 (2nM) + Rucaparib (0nM)
SN-38 (2nM) + Rucaparib (2000nM)
SN-38 (2nM) + Rucaparib (4000nM)
SN-38 (2nM) + Rucaparib (8000nM)

FIG. 2D

MDA-MB-231_Rucaparib: SN-38 (2nM)
Cell Number (%)
0
100
200
300
400
500
Time (Hours)
0
20
40
60
80
Control
Rucaparib (2000nM)
Rucaparib (4000nM)
Rucaparib (8000nM)
SN-38 (2nM) + Rucaparib (0nM)
SN-38 (2nM) + Rucaparib (2000nM)
SN-38 (2nM) + Rucaparib (4000nM)
SN-38 (2nM) + Rucaparib (8000nM)

FIG. 2E

**BT20 cell survival treated with SN-38 and Talazoparib**

FIG. 3A

SN-38
Talazoparib
SN-38 & Talazoparib (3ug/ml)
SN-38 & Talazoparib (1ug/ml)
SN-38 & Talazoparib (0.3ug/ml)
SN-38 & Talazoparib (0.1ug/ml)

Cell Line BT20, Drug SN-38 x Time SameTime

Cell Survival

1.2
1
0.8
0.6
0.4
0.2
0
-0.2

-5 -4 -3 -2 -1 0 1 2 3

SN-38, log10(uM)

HCC38 cell survival treated with
SN-38 and Talazoparib

FIG. 3B

SN-38
Talazoparib
SN-38 & Talazoparib (3ug/ml)
SN-38 & Talazoparib (1ug/ml)
SN-38 & Talazoparib (0.3ug/ml)
SN-38 & Talazoparib (0.1ug/ml)

Cell Line HCC38, Drug SN-38 x Time SameTime

Cell Survival

1.2
1
0.8
0.6
0.4
0.2
0
-0.2

-5 -4 -3 -2 -1 0 1 2 3

SN-38, log10(uM)

Concentration (ng/g)
10⁴
10³
10²
10¹
10⁰
0
24
48
72
96
120
144
168
Time(hr)
Liver
Spleen
Kidney
Lungs
Colon
Duodenum
Tumor


*FIG. 4A*

SN38 duration over 120nM (hours)
150
100
50
0
Colon
Duodenum
Kidney
Liver
Lung
Spleen
Tumor


*FIG. 4B*

Plasma CPT-11, ng/ml
Time, hr
MM-398 5mg/kg
MM-398 10mg/kg
MM-398 20mg/kg
IRI 10mg/kg
IRI 40mg/kg
Simulations


FIG. 5A

FIG. 5B

HT29 CRC tumor levels
of irinotecan (CPT-11)
CPT-11 in tumor, ng/g
100000
10000
1000
100
10
0
50
100
150
200
Time (hours)
40mg/kg MM-398
40mg/kg
free irinotecan

*FIG. 5C*

HT29 CRC tumor levels
of SN-38
SN-38 in tumor, ng/g
10000
1000
100
10
0
50
100
150
200
Time (hours)
40mg/kg MM-398
40mg/kg
free irinotecan

*FIG. 5D*

Breast (BT474)
tumor volume (mm3)
4000
3000
2000
1000
0
Control
50mg/kg
free irinotecan
50mg/kg MM=398
5
15
25
35
45
55
65
time post tumor implantation (d)

*FIG. 6A*

Ovarian (OVCAR8)
Tumor Volume mm3
350
300
250
200
150
100
50
0
Control
20mg/kg MM-398
45
50
55
60
65
70
75
Days

*FIG. 6B*

CRC (HT29)
Tumor Volume (mm$^3$)
2400
2000
1600
1200
800
400
0
8
12
16
20
24
28
Days
Control
50mg/kg
free
irinotecan
10mg/kg
MM-398

*FIG. 6C*

Orthotopic, hypoxic, L3.6pl pancreatic xenograft
Tumor volume (photons)
1.E+11
1.E+10
1.E+09
1.E+08
1.E+07
1.E+06
0
10
20
30
40
50
60
70
Days post cell implantation
PBS
Irinotecan
20 mg/kg
MM-398
20mg/kg

*FIG. 6D*

Irinotecan (CPT-11)

120mg/m2 MM-398
300mg/m2
free irinotecan
CPT-11 Conc., ng/ml
Time, hr

*FIG. 7A*

SN-38

MM-398
free irinotecan
SN-38 Conc., ng/ml
Time, hr

*FIG. 7B*

CPT-11 tumor
CPT-11 plasma
SN-38 tumor
SN-38 plasma
Concentration, ng/ml
Time, days

FIG. 7C

CPT-11
20000
15000
10000
5000
0
metabolites (ng/g)
metabolites (ng/g)
Tumor
Plasma

FIG. 7D

SN-38
80
60
40
20
0
Tumor
Plasma

FIG. 7E

Days 2,3,4
Days 3,4,5
Days 4,5,6
% bodyweight gain
Day after first treatment

FIG. 8A

FIG. 8B

FIG. 8C

Group 1
1 2 3 4 5 6 7
MM-398
10mpk,
Group 2
1 2 3 4 5 6 7
Olaparib
200mpk, po
Group 3
1 2 3 4 5 6 7
MM-398
10mpk,
Olaparib
200mpk, po
Group 4
1 2 3 4 5 6 7
MM-398
10mpk,
Olaparib
150mpk, po
Group 5
1 2 3 4 5 6
MM-398
10mpk, iv
Olaparib
200mpk, po
Group 6
1 2 3 4 5 6 7
MM-398
10mpk, iv
Olaparib
200mpk, po
Group 7
1 2 3 4 5 6 7
MM-398
10mpk,
Olaparib
150mpk, po
Group 8
1 2 3 4 5 6
DMSO (vehicle)
Identicle to G2, po
Total Weekly Olaparib Dosing:
G2: 1000 mg/kg
G3: 1000 mg/kg
G4: 750 mg/kg
G5: 800 mg/kg
G6: 800 mg/kg
G7: 800 mg/kg

FIG. 9

Weight (% of d0)
20
10
0
-10
-20
Body weight (% d0)
0
10
20
30
Days post-injection
Vehicle (DMSO, 1-5)
MM-398 (10mg/kg) (+PBS)
Olaparib (200mg/kg/day)
MM398 (10) + Olaparib (200, 1-4)
MM398 (10) + Olaparib (200, 2-5)

FIG. 10A

Weight (% of d0)
Body weight (% d0)
20
15
10
5
0
-5
0
10
20
30
Days post-injection
Vehicle (DMSO, 1-5)
MM-398 (10mg/kg) (+PBS)
Olaparib (200mg/kg/day)

FIG. 10B

Weight (% of d0)
Body weight (% d0)
20
10
0
-10
-20
0
10
20
30
Days post-injection
MM-398 (10mg/kg) (+PBS)
MM398 (10) + Olaparib (200, 1-4)
MM398 (10) + Olaparib (200, 2-5)

FIG. 10C

Weight (% of d0)
Body weight (% d0)
20
10
0
-10
-20
0
10
20
30
Days post-injection
Vehicle (DMSO, 1-5)
MM-398 (10mg/kg) (+PBS)
MM398 (10) + Olaparib (200, d1-5)
MM398 (10) + Olaparib (150, 1-5)
MM398 (10) + Olaparib (265, 3-5)

FIG. 10D

Tumor volume, $mm^3$
2000
1600
1200
800
400
0
14
21
28
35
42
49
56
63
70
Time post tumor inoculation, days
Control
MM-398
Veliparib
MM-398 + Veliparib


FIG. 11A

Tumor volume, $mm^3$
3500
3000
2500
2000
1500
1000
500
0
21
28
35
42
49
56
63
70
Time post tumor inoculation, days
Control
MM-398 (2 mpk)
Veliparib
MM-398 + Veliparib


FIG. 11B

MM-398 in combination with PARPi (ATB-888)
Percent change in body weight
10
0
-10
-20
1
2
3
4
5
6
7
8
9
10
11
12
13
14
15
16
17
18
19
20
Day
MM-398/50 mpk + PARPi/345
MM-398/50 mpk + PARPi/234
MM-398/50 mpk + PARPi/123

FIG. 12A

MM-398 in combination with PARPI (ATB-888)
Percent change in body weight
20
10
0
-10
-20
1
2
3
4
5
6
7
8
9
10
11
12
13
14
15
16
17
18
19
20
Day
MM-398/28 mpk + PARPi/345
MM-398/28 mpk + PARPi/234
MM-398/28 mpk + PARPi/123

FIG. 12B

Tumor volume, mm$^3$
2000
1600
1200
800
400
0
14
21
28
35
42
49
56
63
70
Time post tumor inoculation, days
Control
MM-398 (5 mpk)
Veliparib
MM-398 + Veliparib
p = 0.03


FIG. 13A

Percent survival
100
80
60
40
20
0
30
40
50
60
70
80
90
100
Time
Control
MM-398
Veliparib
MM-398+Veliparib


FIG. 13B

15.0
12.5
10.0
7.5
5.0
2.5
0.0
-2.5
-5.0
Body weight, %
18
25
32
39
46
53
60
Time post tumor inoculation, days
Control
MM-398 (5 mpk)
Veliparib
MM-398 + Veliparib

FIG. 13C

3500
3000
2500
2000
1500
1000
500
0
Tumor volume, mm3
21
28
35
42
49
56
63
70
Time post tumor inoculation, days
Control
MM-398 (2 mpk)
Veliparib
MM-398 + Veliparib

FIG. 14

Percent survival
100
80
60
40
20
0
30
44
58
72
86
100
114
128
Time
Control
MM-398
Veliparib
MM-398+Veliparib

FIG. 15

Body weight, %
15.0
12.5
10.0
7.5
5.0
2.5
0.0
-2.5
-5.0
23
30
37
44
51
58
Time post tumor inoculation, days
Control
MM-398
Veliparib
MM-398 + Veliparib

FIG. 16

Schedule
Same Time
24h later
IC50 Log(uM)
uM 0
-1
-2
nM -3
-4
C-4 II
HeLa
SiHa
C-4 I
CaSki
MES-SA
HT-3
MS-751
SK-UT-1
GH 329
DoTC2 4510
GH 354
SK-LMS
SW 756
ME 180
C-33A
Cervical Cell Lines

FIG. 17A

CPT-11
SN-38
SN-38, ng/g tissue
100
10
1
0.1
SiHa
ME180
C33A
SW756
MS751
HT-3
10000
1000
100
10
CPT-11, ng/g tissue

FIG. 17B

TRIAL DESIGN
Ferumoxytol MRI
28-day cycle: nal-IRI
nal-IRI
Day: -7 -2 -1 1 2 3 4 5 12 15 17 19 25 28
Schedule 1:
Schedule 2:
Veliparib
Veliparib

| Dose Escalation Cohorts | | | |
|---|---|---|---|
| Dose Level | Veliparib Dose (mg BID) | Veliparib Start Day | nal-IRI Dose (salt) (mg/m² q2w) |
| 1 | 100 | Day 5 | 80 |
| 2 | 200 | Day 5 | 80 |
| 3 | 200 | Day 3 | 80 |
| 4 | 300 | Day 3 | 80 |
| 5 | 400 | Day 3 | 80 |

FIG. 18

CPT-11
MM-398
Ferumoxytol (MRI)
1 - Extend PK/Circulation
2 - Deposition in tumor through leaky vasculature
3 - Uptake by phagocytic cells
3 - Conversion of CPT-11 to SN-38 (MM-398 only)
Tumor Cells

FIG. 19A

Lesion-specific FMX levels at 24h in all patients
Tumor FMX, 24 h (µg/ml)
150
100
50
0
-50
median
Patient
Breast
Cervical
Head & neck
Ovarian
Pancreatic
Other (mixed)

FIG. 19B

p=0.0026
100%
50%
0%
-50%
P4
P2
P2
P6
P21
P21
P1
P6
P1
P6
P6
P19
P18
P4
P4
P6
P19
P19
P9
P10
P9
P9
P9
Below Median
Above Median

FIG. 19C

Irinotecan 50 mg/kg
Irinotecan 100 mg/kg
nal-IRI 5 mg/kg
nal-IRI 10 mg/kg
nal-IRI 20 mg/kg
Tumor SN-38 (nmol/L)
Time (h)

FIG. 20A

SN-38 duration above threshold
Tumor growth inhibition (%)
20 mg/kg
10 mg/kg
5 mg/kg
50 mg/kg
2.5 mg/kg
1.25 mg/kg
SN-38 duration (h)

FIG. 20B

SN-38
SN-38 Olaparib (3ug/ml)
Olaparib
Cell Viability (% of control)
0
20
40
60
80
100
120
0.000001
0.000010
0.000100
0.001000
0.010000
0.100000
1.000000
10.000000
100.000000
[drug] (ug/ml)

FIG. 21A

SN-38
SN-38 Olaparib (1ug/ml)
Olaparib
Cell Viability (% of control)
0
20
40
60
80
100
120
0.000001
0.000010
0.000100
0.001000
0.010000
0.100000
1.000000
10.000000
100.000000
[drug] (ug/ml)

FIG. 21B

SN-38
SN-38 Olaparib (3ug/ml)
Olaparib
Cell Viability (% of control)
120
100
80
60
40
20
0
0.000001
0.000010
0.000100
0.001000
0.010000
0.100000
1.000000
10.000000
100.000000
[drug] (ug/ml)

FIG. 21C

SN-38
SN-38 Olaparib (1ug/ml)
Olaparib
Cell Viability (% of control)
120
100
80
60
40
20
0
0.000001
0.000010
0.000100
0.001000
0.010000
0.100000
1.000000
10.000000
100.000000
[drug] (ug/ml)

FIG. 21D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8147867 B
- US 20150005354
- US 20100247668 A
- US 20110160159 A
- US 20110189092 A
- US 20150005354 A


### Non-patent literature cited in the description


- **MESSERER et al.** *Clin Cancer Res.,* 01 October 2004, vol. 10 (19), 6638-49
- **GENTHER WILLIAMS et al.** Liposomal irinotecan: formulation development and therapeutic assessment in murine xenograft models of colorectal cancer. *Cancer Cell International,* 2015, vol. 15, 14
- **DAVIDSON et al.** Treatment with the PARP inhibitor, niraparib, sensitizes colorectal cancer cell lines to irinotecan regardless of MSI/MSS status. *Invest New Drugs,* April 2013, vol. 31 (2), 461-8
- **GREENWALD et al.** *Bioorg. Med. Chem.,* 1998, vol. 6, 551-562
- *J Clin Oncol,* 2014, vol. 32
- *J Clin Oncol,* 2011, vol. 29
- *Cancer Res,* 2011, vol. 71 (562), 6-5634